(19) Europäisches Patentamt
European Patent Office
Office européen des brevets

(11) EP 1 408 111 B1

(12) **EUROPEAN PATENT SPECIFICATION**

(45) Date of publication and mention of the grant of the patent:
**09.06.2010 Bulletin 2010/23**

(51) Int Cl.:
***C12N 15/11*** *(2006.01)* ***C12N 9/00*** *(2006.01)*
***C12N 5/10*** *(2006.01)*

(21) Application number: **04000536.5**

(22) Date of filing: **25.10.1996**

(54) **Method and reagent for the treatment of diseases or conditions related to levels of vascular endothelial growth factor receptor**

Verfahren und Reagenz zur Behandlung von Krankheiten die von der Konzentration des Rezeptors für den Wachstumsfaktor der vaskulären Endothelzellen verursacht werden

Procédé et réactif de traitement de maladies ou troubles liés aux niveaux du récepteur du facteur de croissance endothéliale vasculaire

(84) Designated Contracting States:
**CH DE FR GB IT LI**

(30) Priority: **26.10.1995 US 5974 P**
**11.01.1996 US 584040**

(43) Date of publication of application:
**14.04.2004 Bulletin 2004/16**

(62) Document number(s) of the earlier application(s) in accordance with Art. 76 EPC:
**96939515.1 / 0 859 837**

(73) Proprietors:
- **Sirna Therapeutics, Inc.**
  **Boulder, CO 80301 (US)**
- **Novartis Vaccines and Diagnostics, Inc.**
  **Emeryville, CA 94608 (US)**

(72) Inventors:
- **Pavco, Pamela**
  **Lafayette,**
  **Colorado 80026 (US)**
- **Stinchomb, Dan T.**
  **Fort Collins,**
  **Colorado 80524 (US)**
- **McSwiggen, James**
  **Boulder,**
  **Colorado 80301 (US)**
- **Escobedo, Jaime**
  **Alamo,**
  **Californa 94507 (US)**

(74) Representative: **Hussain, Deeba**
**Merck & Co., Inc.**
**Hertford Road**
**Hoddesdon**
**Hertfordshire EN11 9BU (GB)**

(56) References cited:
**WO-A-94/11499** **WO-A-94/21791**
**WO-A-95/13380**

- **SHIBUYA M ET AL: "NUCLEOTIDE SEQUENCE AND EXPRESSION OF A NOVEL HUMAN RECEPTOR-TYPE, TYROSINE KINASE GENE (FLT) CLOSELY RELATED TO THE FMS FAMILY" 1990, ONCOGENE, BASINGSTOKE, HANTS, GB, PAGE(S) 519-524 , XP000569874 ISSN: 0950-9232 * the whole document ***
- **USMAN N ET AL: "CHEMICAL MODIFICATION OF HAMMERHEAD RIBOZYMES: ACTIVITY AND NUCLEASE RESISTANCE" NUCLEIC ACIDS RESEARCH SYMPOSIUM SERIES, no. 31, 9 November 1994 (1994-11-09), pages 163-164, XP002002020**
- **PARRY T J ET AL: "Bioactivity of anti-angiogenic ribozymes targeting Flt-1 and KDR mRNA" May 1999 (1999-05), NUCLEIC ACIDS RESEARCH, OXFORD UNIVERSITY PRESS, SURREY, GB, PAGE(S) 2569-2577 , XP002971041 ISSN: 0305-1048 * the whole document ***

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 408 111 B1

## Description

Background Of The Invention

This invention relates to methods and reagents for the treatment of diseases or conditions relating to the levels of expression of vascular endothelial growth factor (VEGF) receptor(s).

VEGF, also referred to as vascular permeability factor (VPF) and vasculotropin, is a potent and highly specific mitogen of vascular endothelial cells (for a review see Ferrara, 1993 Trends Cardiovas. Med. 3, 244; Neufeld et al., 1994 Prog. Growth Factor Res. 5, 89). VEGF induced neovascularization is implicated in various pathological conditions such as tumor angiogenesis, proliferative diabetic retinopathy, hypoxia-induced angiogenesis, rheumatoid arthritis, psoriasis, wound healing and others.

VEGF, an endothelial cell-specific mitogen, is a 34-45 kDa glycoprotein with a wide range of activities that include promotion of angiogenesis, enhancement of vascular-permeability and others. VEGF belongs to the platelet-derived growth factor (PDGF) family of growth factors with approximately 18% homology with the A and B chain of PDGF at the amino acid level. Additionally, VEGF contains the eight conserved cysteine residues common to all growth factors belonging to the PDGF family (Neufeld *et al*., *supra*). VEGF protein is believed to exist predominantly as disulfide-linked homodimers; monomers of VEGF have been shown to be inactive (Plouet et al., 1989 EMBO J. 8, 3801).

VEGF exerts its influence on vascular endothelial cells by binding to specific high-affinity cell surface receptors. Covalent cross-linking experiments with $^{125}$I-labeled VEGF protein have led to the identification of three high molecular weight complexes of 225, 195 and 175 kDa presumed to be VEGF and VEGF receptor complexes (Vaisman et al., 1990 J. Biol. Chem. 265, 19461). Based on these studies VEGF-specific receptors of 180, 150 and 130 kDa molecular mass were predicted. In endothelial cells, receptors of 150 and the 130 kDa have been identified. The VEGF receptors belong to the superfamily of receptor tyrosine kinases (RTKs) characterized by a conserved cytoplasmic catalytic kinase domain and a hydrophylic kinase sequence. The extracellular domains of the VEGF receptors consist of seven immunoglobulin-like domains that are thought to be involved in VEGF binding functions.

The two most abundant and high-affinity receptors of VEGF are flt-1 (fms-like tyrosine kinase) cloned by Shibuya et al., 1990 Oncogene 5, 519 and KDR (kinase-insert-domain-containing receptor) cloned by Terman et al., 1991 Oncogene 6, 1677. The murine homolog of KDR, cloned by Mathews et al., 1991, Proc. Natl. Acad. Sci., USA, 88, 9026, shares 85% amino acid homology with KDR and is termed as flk-1 (fetal liver kinase-1). Recently it has been shown that the high-affinity binding of VEGF to its receptors is modulated by cell surface-associated heparin and heparin-like molecules (Gitay-Goren et al., 1992 J. Biol. Chem. 267, 6093).

VEGF expression has been associated with several pathological states such as tumor angiogenesis, several forms of blindness, rheumatoid arthritis, psoriasis and others. Following is a brief summary of evidence supporting the involvement of VEGF in various diseases:

1) Tumor angiogenesis: Increased levels of VEGF gene expression have been reported in vascularized and edema-associated brain tumors (Berkman et al., 1993 J. Clini. Invest. 91, 153). A more direct demostration of the role of VEGF in tumor angiogenesis was demonstrated by Jim Kim et al., 1993 Nature 362,841 wherein, monoclonal antibodies against VEGF were successfully used to inhibit the growth of rhabdomyosarcoma, glioblastoma multiforme cells in nude mice. Similarly, expression of a dominant negative mutated form of the flt-1 VEGF receptor inhibits vascularization induced by human glioblastoma cells in nude mice (Millauer et al., 1994, Nature 367, 576).
2) ocular diseses: Aiello et al., 1994 New Engl. J. Med. 331, 1480, showed that the ocular fluid, of a majority of patients suffering from diabetic retinopathy and other retinal disorders, contains a high concentration of VEGF. Miller et al., 1994 Am. J. Pathol. 145, 574, reported elevated levels of VEGF mRNA in patients suffering from retinal ischemia. These observations support a direct role for VEGF in ocular diseases.
3) Psoriasis: Detmar et al., 1994 J. Exp. Med. 180, 1141 reported that VEGF and its receptors were over-expressed in psoriatic skin and psoriatic dermal micro-vessels, suggesting that VEGF plays a significant role in psoriasis.
4) Rheumatoid arthritis: Immunohistochemistry and in situ hybridization studies on tissues from the joints of patients suffering from rheumatoid arthritis show an increased level of VEGF and its receptors (Fava et al., 1994 J. Exp. Med. 180, 341). Additionally, Koch et al., 1994 J. Immunol. 152, 4149, found that VEGF-specific antibodies were able to significantly reduce the mitogenic activity of synovial tissues from patients suffering from rheumatoid arthritis. These observations support a direct role for VEGF in rheumatoid arthritis.

In addition to the above data on pathological conditions involving excessive angiogenesis, a number of studies have demonstrated that VEGF is both necessary and sufficient for neovascularization. Takashita et al., 1995 J. Clin. Invest. 93, 662, demonstrated that a single injection of VEGF augmented collateral vessel development in a rabbit model of ischemia. VEGF also can induce neovascularization when injected into the cornea. Expression of the VEGF gene in CHO cells is sufficient to confer tumorigenic potential to the cells. Kim *et al*., *supra* and Millauer *et al*., *supra* used monoclonal antibodies against VEGF or a dominant negative form of flk-1 receptor to inhibit tumor-induced neovascularization.

During development, VEGF and its receptors are associated with regions of new vascular growth (Millauer et al., 1993 Cell 72, 835; Shalaby et al., 1993 J. Clin. Invest. 91, 2235). Furthermore, transgenic mice lacking either of the VEGF receptors are defective in blood vessel formation, infact these mouse do not survive; flk-1 appears to be required for differentiation of endothelial cells, while flt-1 appears to be required at later stages of vessel formation (Shalaby et al., 1995 Nature 376, 62; Fung et al., 1995 Nature 376, 66). Thus, these receptors must be present to properly signal endothelial cells or their precursors to respond to vascularization-promoting stimuli.

All of the conditions listed above, involve extensive vascularization. This hyper-stimulation of endothelial cells may be alleviated by VEGF antagonists. Thus most of the therapeutic efforts for the above conditions have concentrated on finding inhibitors of the VEGF protein.

Kim et al., 1993 Nature 362, 841 have been successful in inhibiting VEGF-induced tumor growth and angiogenesis in nude mice by treating the mice with VEGF-specific monoclonal antibody.

Koch et al., 1994 J. Immunol. 152, 4149 showed that the mitogenic activity of microvascular endothelial cells found in rheumatoid arthritis (RA) synovial tissue explants and the chemotactic property of endothelial cells from RA synovial fluid can be neutralized significantly by treatment with VEGF-specific antibodies.

Ullrich et al., International PCT Publication No. WO 94/11499 and Millauer et al., 1994 Nature 367, 576 used a soluble form of flk-1 receptor (dominant-negative mutant) to prevent VEGF-mediated tumor angiogenesis in immunodeficient mice.

Kendall and Thomas, International PCT Publication No. WO 94/21679 describe the use of naturally occuring or recombinantly-engineered soluble forms of VEGF receptors to inhibit VEGF activity.

Robinson, International PCT Publication No. WO 95/04142 describes the use of antisense oligonucleotides targeted against VEGF RNA to inhibit VEGF expression.

Jellinek et al., 1994 Biochemistry 33, 10450 describe the use of VEGF-specific high-affinity RNA aptamers to inhibit the binding of VEGF to its receptors.

Rockwell and Goldstein, International PCT Publication No. WO 95/21868, describe the use of anti-VEGF receptor monoclonal antibodies to neutralize the the effect of VEGF on endothelial cells.

## Summary Of The Invention

The invention features enzymatic nucleic acid molecules (ribozymes) that specifically inhibits the expression of the human flt-1 receptor and are targeted to nucleotide position 4229 of flt-1.

By "inhibit" it is meant that the activity of VEGF-R or level of mRNAs or equivalent RNAs encoding VEGF-R is reduced below that observed in the absence of the nucleic acid. In one embodiment, inhibition with ribozymes preferably is below that level observed in the presence of an enzymatically inactive RNA molecule that is able to bind to the same site on the mRNA, but is unable to cleave that RNA. In another embodiment, inhibition with antisense oligonucleotides is preferably below that level observed in the presence of for example, an oligonucleotide with scrambled sequence or with mismatches.

By "enzymatic nucleic acid molecule" it is meant an RNA molecule which has complementarity in a substrate binding region to a specified gene target, and also has an enzymatic activity which is active to specifically cleave target RNA. That is, the enzymatic RNA molecule is able to intermolecularly cleave RNA and thereby inactivate a target RNA molecule. This complementary regions allow sufficient hybridization of the enzymatic RNA molecule to the target RNA and thus permit cleavage. One hundred percent complementarity is preferred, but complementarity as low as 50-75% may also be useful in this invention. By "equivalent" RNA to VEGF-R is meant to include those naturally occurring RNA molecules in various animals, including human, mice, rats, rabbits, primates and pigs.

By "gene" it is meant a nucleic acid that encodes an RNA.

By "complementarity" it is meant a nucleic acid that can form hydrogen bond(s) with other RNA sequence by either traditional Watson-Crick or other non-traditional types (for example, Hoogsteen type) of base-paired interactions.

Six basic varieties of naturally-occurring enzymatic RNAs are known presently. Each can catalyze the hydrolysis of RNA phosphodiester bonds in trans (and thus can cleave other RNA molecules) under physiological conditions. Table I summarizes some of the characteristics of these ribozymes. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets. Thus, a single ribozyme molecule is able to cleave many molecules of target RNA. In addition, the ribozyme is a highly specific inhibitor of gene expression, with the specificity of inhibition depending not only on the base-pairing mechanism of binding to the target RNA, but also on the mechanism of target RNA cleavage. Single mismatches, or base-substitutions, near the site of cleavage can completely eliminate catalytic activity of a ribozyme.

Ribozymes that cleave the specified sites in VEGF-R mRNAs represent a novel therapeutic approach to treat tumor angiogenesis, ocular diseases, rhuematoid arthritis, psoriasis and others. Applicant indicates that ribozymes are able to inhibit the activity of VEGF-R (specifically flt-1 and flk-1/KDR) and that the catalytic activity of the ribozymes is required for their inhibitory effect. Those of ordinary skill in the art will find that it is clear from the examples described that other ribozymes that cleave VEGF-R mRNAs may be readily designed.

In preferred embodiments of this invention, the enzymatic nucleic acid molecule is formed in a hammerhead or hairpin motif, but may also be formed in the motif of a hepatitis delta virus, group I intron or RNaseP RNA (in association with an RNA guide sequence) or Neurospora VS RNA. Examples of such hammerhead motifs are described by Rossi et al., 1992, AIDS Research and Human Retroviruses 8, 183, of hairpin motifs by Hampel et al., EP0360257, Hampel and Tritz, 1989 Biochemistry 28, 4929, and Hampel et al., 1990 Nucleic Acids Res. 18, 299, and an example of the hepatitis delta virus motif is described by Perrotta and Been, 1992 Biochemistry 31, 16; of the RNaseP motif by Guerrier-Takada et al., 1983 Cell 35, 849, *Neurospora* VS RNA ribozyme motif is described by Collins (Saville and Collins, 1990 Cell 61, 685-696; Saville and Collins, 1991 Proc. Natl. Acad. Sci. USA 88, 8826-8830; Collins and Olive, 1993 Biochemistry 32, 2795-2799) and of the Group I intron by Cech et al., U.S. Patent 4,987,071. Those skilled in the art will recognize that all that is important in an enzymatic nucleic acid molecule of this invention is that it has a specific substrate binding site which is complementary to one or more of the target gene RNA regions, and that it have nucleotide sequences within or surrounding that substrate binding site which impart an RNA cleaving activity to the molecule.

Also disclosed is a method for producing a class of enzymatic cleaving agents which exhibit a high degree of specificity for the RNA of a desired target. The enzymatic nucleic acid molecule is preferably targeted to a highly conserved sequence region of target mRNAs encoding VEGF-R proteins (specifically flt-1 and flk-1/KDR) such that specific treatment of a disease or condition can be provided with either one or several enzymatic nucleic acids. Such enzymatic nucleic acid molecules can be delivered exogenously to specific tissue or cellular targets as required. Alternatively, the ribozymes can be expressed from DNA and/or RNA vectors that are delivered to specific cells.

Synthesis of nucleic acids greater than 100 nucleotides in length is difficult using automated methods, and the therapeutic cost of such molecules is prohibitive. In this invention, small nucleic acid motifs (*e.g.*, hammerhead or the hairpin ribozymes) are used for exogenous delivery. The simple structure of these molecules increases the ability of the nucleic acid to invade targeted regions of the mRNA structure. However, these nucleic acid molecules can also be expressed within cells from eukaryotic promoters (*e.g.*, Izant and Weintraub, 1985 Science 229, 345; McGarry and Lindquist, 1986 Proc. Natl. Acad. Sci. USA 83, 399; Sullenger-Scanlon et al., 1991, Proc. Natl. Acad. Sci. USA, 88, 10591-5; Kashani-Sabet et al., 1992 Antisense Res. Dev., 2, 3-15; Dropulic et al., 1992 J. Virol, 66, 1432-41; Weerasinghe et al., 1991 J. Virol, 65, 5531-4; Ojwang et al., 1992 Proc. Natl. Acad. Sci. USA 89, 10802-6; Chen et al., 1992 Nucleic Acids Res., 20, 4581-9; Sarver et al., 1990 Science 247, 1222-1225; Thompson et al., 1995 Nucleic Acids Res. 23, 2259). Those skilled in the art realize that any nucleic acid can be expressed in eukaryotic cells from the appropriate DNA/RNA vector. The activity of such nucleic acids can be augmented by their release from the primary transcript by a ribozyme (Draper et al., PCT WO93/23569, and Sullivan et al., PCT WO94/02595; Ohkawa et al., 1992 Nucleic Acids Symp. Ser., 27, 15-6; Taira et al., 1991, Nucleic Acids Res., 19, 5125-30; Ventura et al., 1993 Nucleic Acids Res., 21, 3249-55; Chowrira et al., 1994 J. Biol. Chem. 269, 25856).

Such nucleic acids are useful for the prevention of the diseases and conditions discussed above, and any other diseases or conditions that are related to the levels of VEGF-R (specifically flt-1) in a cell or tissue.

By "related" is meant that the reduction of VEGF-R (specifically flt-1) RNA levels and thus reduction in the level of the respective protein will relieve, to some extent, the symptoms of the disease or condition.

Ribozymes are added directly, or can be complexed with cationic lipids, packaged within liposomes, or otherwise delivered to target cells or tissues. The nucleic acid or nucleic acid complexes can be locally administered to relevant tissues ex vivo, or in vivo through injection, infusion pump or stent, with or without their incorporation in biopolymers. The ribozymes disclosed herein have binding arms which are complementary to the sequences in Tables II to III. Examples of such ribozymes also are shown in Tables II to III. Examples of such ribozymes consist essentially of sequences defined in these Tables. By "consists essentially of" is meant that the active ribozyme contains an enzymatic center equivalent to those in the examples, and binding arms able to bind mRNA such that cleavage at the target site occurs. Other sequences may be present which do not interfere with such cleavage.

In another aspect of the invention, ribozymes that cleave target RNA molecules and inhibit VEGF-R (specifically flt-1) activity are expressed from transcription units inserted into DNA or RNA vectors. The recombinant vectors are preferably DNA plasmids or viral vectors. Ribozyme expressing viral vectors could be constructed based on, but not limited to, adeno-associated virus, retrovirus, adenovirus, or alphavirus. Preferably, the recombinant vectors capable of expressing the ribozymes are delivered as described above, and persist in target cells. Alternatively, viral vectors may be used that provide for transient expression of ribozymes. Such vectors might be repeatedly administered as necessary. Once expressed, the ribozymes cleave the target mRNA. Delivery of ribozyme expressing vectors could be systemic, such as by intravenous or intramuscular administration, by administration to target cells ex-planted from the patient followed by reintroduction into the patient, or by any other means that would allow for introduction into the desired target cell.

By "vectors" is meant any nucleic acid- and/or viral-based technique used to deliver a desired nucleic acid.

Other features and advantages of the invention will be apparent from the following description of the preferred embodiments thereof, and from the claims.

Description Of The Preferred Embodiments

First the drawings will be described briefly.

Drawings

Figure 1 is a diagrammatic representation of the hammerhead ribozyme domain known in the art. Stem II can be ≥ 2 base-pair long.

Figure 2a is a diagrammatic representation of the hammerhead ribozyme domain known in the art; Figure 2b is a diagrammatic representation of the hammerhead ribozyme as divided by Uhlenbeck (1987, Nature, 327, 596-600) into a substrate and enzyme portion; Figure 2c is a similar diagram showing the hammerhead divided by Haseloff and Gerlach (1988, Nature, 334, 585-591) into two portions; and Figure 2d is a similar diagram showing the hammerhead divided by Jeffries and Symons (1989, Nucl. Acids. Res., 17, 1371-1371) into two portions.

Figure 3 is a diagramatic representation of the general structure of a hairpin ribozyme. Helix 2 (H2) is provided with a least 4 base pairs (*i.e.*, n is 1, 2, 3 or 4) and helix 5 can be optionally provided of length 2 or more bases (preferably 3 - 20 bases, *i.e.*, m is from 1 - 20 or more). Helix 2 and helix 5 may be covalently linked by one or more bases (*i.e.*, r is ≥ 1 base). Helix 1, 4 or 5 may also be extended by 2 or more base pairs (*e.g.*, 4 - 20 base pairs) to stabilize the ribozyme structure, and preferably is a protein binding site. In each instance, each N and N' independently is any normal or modified base and each dash represents a potential base-pairing interaction. These nucleotides may be modified at the sugar, base or phosphate. Complete base-pairing is not required in the helices, but is preferred. Helix 1 and 4 can be of any size (*i.e.*, o and p is each independently from 0 to any number, *e.g.*, 20) as long as some base-pairing is maintained. Essential bases are shown as specific bases in the structure, but those in the art will recognize that one or more may be modified chemically (abasic, base, sugar and/or phosphate modifications) or replaced with another base without significant effect. Helix 4 can be formed from two separate molecules, *i.e.*, without a connecting loop. The connecting loop when present may be a ribonucleotide with or without modifications to its base, sugar or phosphate. "q" is ≥ 2 bases. The connecting loop can also be replaced with a non-nucleotide linker molecule. H refers to bases A, U, or C. Y refers to pyrimidine bases. "_" refers to a covalent bond.

Figure 4 is a representation of the general structure of the hepatitis delta virus ribozyme domain known in the art.

Figure 5 is a representation of the general structure of the VS RNA ribozyme domain.

Figure 6 is a schematic representation of an RNAseH accessibility assay. Specifically, the left side of Figure 6 is a diagram of complementary DNA oligonucleotides bound to accessible sites on the target RNA. Complementary DNA oligonucleotides are represented by broad lines labeled A, B, and C. Target RNA is represented by the thin, twisted line. The right side of Figure 6 is a schematic of a gel separation of uncut target RNA from a cleaved target RNA. Detection of target RNA is by autoradiography of body-labeled, T7 transcript. The bands common to each lane represent uncleaved target RNA; the bands unique to each lane represent the cleaved products.

Figure 7 shows the effect of hammerhead ribozymes targeted against flt-1 receptor on the binding of VEGF to the surface of human microvascular endothelial cells. Sequences of the ribozymes used are shown in Table II; the length of stem II region is 3 bp. The hammerhead ribozymes were chemically modified such that the ribozyme consists of ribose residues at five positions (see Figure 11); U4 and U7 positions contain 2'-$NH_2$ modifications, the remaining nucleotide positions contain 2'-*O*-methyl substitutions; four nucleotides at the 5' terminus contains phosphorothioate substitutions. Additionally, the 3' end of the ribozyme contains a 3'-3' linked inverted abasic deoxyribose. The results of two separate experiments are shown as separate bars for each set. Each bar represents the average of triplicate samples. The standard deviation is shown with error bars. For the flt-1 data, 500 nM ribozyme (3:1 charge ratio with LipofectAMINE®) was used. Control 1-10 is the control for ribozymes 307-2797, control 11-20 is the control for ribozymes 3008-5585. The Control 1-10 and Control 11-20 represent the treatment of cells with LipofectAMINE® alone without any ribozymes.

Figure 8 shows the specificity of hammerhead ribozymes targeted against flt-1 receptor. Inhibition of the binding of VEGF, urokinase plasminogen activator (UPA) and fibroblast growth factor (FGF) to their corresponding receptors as a function of anti-FLT ribozymes is shown. The sequence and description of the ribozymes used are as described under Figure 7 above. The average of triplicate samples is given; percent inhibition as calculated below.

Figure 9 shows *in vitro* cleavage of flt-1 RNA by hammerhead ribozymes. A) diagrammatic representation of hammerhead ribozymes targeted against flt-1 RNA. The hammerhead (HH) ribozymes were chemically modified such that the ribozyme consists of ribose residues at five positions; U4 and U7 positions contain 2'-$NH_2$ modifications, the remaining nucleotide positions contain 2'-O-methyl substitutions; four nucleotides at the 5' terminus contains phosphorothioate substitutions. Additionally, the 3' end of the ribozyme contains a 3'-3' linked inverted abasic deoxyribose (designated as 3'-iH). 1358 HH-A and 4229 HH-A contain 3 base-paired stem II region. 1358 HH-B and 4229 HH-B contain 4 base-paired stem II region. B) and C) shows *in vitro* cleavage kinetics of HH ribozymes targeted against sites 1358 and 4229 within the flt-1 RNA.

Figure 10 shows inhibition of human microvascular endothelial cell proliferation mediated by anti-flt-1 hammerhead ribozymes. A) Diagrammatic representation of hammerhead (HH) ribozymes targeted against sites 1358 and 4229 within the the flt-1 RNA. B) Graphical representation of the inhibition of cell proliferation mediated by 1358HH and 4229HH ribozymes.

Figure 11 shows *in vitro* cleavage of RNA by hammerhead ribozymes that are targeted to sites that are conserved between flt-1 and KDR RNA. The hammerhead (HH) ribozymes were chemically modified such that the ribozyme consists of ribose residues at five positions; U4 and U7 positions contain 2'-$NH_2$ modifications, the remaining nucleotide positions contain 2'-*O*-methyl substitutions. Additionally, the 3' end of the ribozyme contains a 3'-3' linked inverted abasic deoxyribose (designated as 3'-iH). FLT/KDR-I HH ribozyme was synthesized with either a 4 base-paired or a 3 base-paired stem II region. FLT/KDR-I HH can cleave site 3388 within flt-1 RNA and site 3151 within KDR RNA. Percent *in vitro* cleavage kinetics as a function of time of HH ribozymes targeted against sites 3702 and 3950 within the KDR RNA is shown.

Figure 12 shows inhibition of human microvascular endothelial cell proliferation mediated by anti-KDR and anti-flt-1 hammerhead ribozymes. The figure is a graphical representation of the inhibition of cell proliferation mediated by hammerhead ribozymes targeted against sites KDR sites-527, 726 or 3950 or flt-1 site 4229. The figure also shows enhanced inhibition of cell proliferation by a combination of flt-1 and KDR hammerhead ribozymes. 4229+527, indicates the treatment of cells with both the flt 4229 and the KDR 527 ribozymes. 4229+726, indicates the treatment of cells with both the flt 4229 and the KDR 726 ribozymes. 4229+3950, indicates the treatment of cells with both the flt 4229 and the KDR 3950 ribozymes. VEGF -, indicates the basal level of cell proliferation in the absence of VEGF. A, indicates catalytically active ribozyme; I, indicates catalytically inactive ribozyme. All of these ribozymes were chemically modified such that the ribozyme consists of ribose residues at five positions; U4 and U7 positions contain 2'-$NH_2$ modifications, the remaining nucleotide positions contain 2'-*O*-methyl substitutions; four nucleotides at the 5' termini contain phosphorothioate substitutions. Additionally, the 3' end of the ribozyme contains a 3'-3' linked inverted abasic deoxyribose (3'-iH).

Figure 13 shows the inhibition of VEGF-induced angiogenesis in rat cornea mediated by anti-flt-1 hammerhead ribozyme. All of these ribozymes were chemically modified such that the ribozyme consists of ribose residues at five positions; U4 position contains 2'-*C*-allyl modifications, the remaining nucleotide positions contain 2'-*O*-methyl substitutions; four nucleotides at the 5' termini contain phosphorothioate substitutions. Additionally, the 3' end of the ribozyme contains a 3'-3' linked inverted abasic deoxyribose (3'-iH). A decrease in the Surface Area corresponds to a reduction in angiogenesis. VEGF alone, corresponds to treatment of the cornea with VEGF and no ribozymes. Vehicle alone, corresponds to the treatment of the cornea with the carrier alone and no VEGF. This control gives a basal level of Surface Area. Active 4229 HH, corresponds to the treatment of cornea with the flt-1 4229 HH ribozyme in the absence of any VEGF. This control also gives a basal level of Surface Area. Active 4229 HH + VEGF, corresponds to the co-treatment of cornea with the flt-1 4229 HH ribozyme and VEGF. Inactive 4229 HH + VEGF, corresponds to the co-treatment of cornea with a catalytically inactive version of 4229 HH ribozyme and VEGF.

Ribozymes

Ribozymes of this invention block to some extent VEGF-R (specifically flt-1) production and can be used to treat disease or diagnose such disease. Ribozymes will be delivered to cells in culture, to cells or tissues in animal models of angiogenesis and/or RA and to human cells or tissues *ex vivo* or *in vivo*. Ribozyme cleavage of VEGF-R RNAs (specifically RNAs that encode flt-1) in these systems may alleviate disease symptoms.

Target sites

Targets for useful ribozymes can be determined as disclosed in Draper et al., International PCT Publication No. WO 95/13380. Other examples include the following PCT applications which concern inactivation of expression of disease-related genes: WO 95/23225, WO 95/13380, WO 94/02595. Rather than repeat the guidance provided in those documents here, below are provided specific examples of such methods, not limiting to those in the art. Ribozymes to such targets are designed as described in those applications and synthesized to be tested *in vitro* and *in vivo,* as also described.

The sequence of human flt-1 mRNA was screened for optimal ribozyme target sites using a computer folding algorithm. Hammerhead or hairpin ribozyme cleavage sites were identified. These sites are shown in Tables II to III (all sequences are 5' to 3' in the tables; X can be any base-paired sequence, the actual sequence is not relevant here). The nucleotide base position is noted in the Tables as that site to be cleaved by the designated type of ribozyme. The nucleotide base position is noted in the Tables as that site to be cleaved by the designated type of ribozyme.

Hammerhead or hairpin ribozymes were designed that could bind and cleave target RNA in a sequence-specific manner. The ribozymes were individually analyzed by computer folding (Jaeger et al., 1989 Proc. Natl. Acad. Sci. USA, 86, 7706) to assess whether the ribozyme sequences fold into the appropriate secondary structure. Those ribozymes with unfavorable intramolecular interactions between the binding arms and the catalytic core were eliminated from consideration. Varying binding arm lengths can be chosen to optimize activity.

Referring to Figure 6, mRNA is screened for accessible cleavage sites by the method described generally in Draper et al., PCT WO93/23569. Briefly, DNA oligonucleotides complementary to potential hammerhead or hairpin ribozyme cleavage sites were synthesized. A polymerase chain reaction is used to generate substrates for T7 RNA polymerase transcription from human flt-1 cDNA clones. Labeled RNA transcripts are synthesized *in vitro* from the templates. The oligonucleotides and the labeled transcripts were annealed, RNAseH was added and the mixtures were incubated for the designated times at 37°C. Reactions are stopped and RNA separated on sequencing polyacrylamide gels. The percentage of the substrate cleaved is determined by autoradiographic quantitation using a PhosphorImaging system. From these data, hammerhead or hairpin ribozyme sites are chosen as the most accessible.

Ribozymes of the hammerhead or hairpin motif were designed to anneal to various sites in the mRNA message. The binding arms are complementary to the target site sequences described above. The ribozymes were chemically synthesized. The method of synthesis used follows the procedure for normal RNA synthesis as described in Usman et al., 1987 J. Am. Chem. Soc., 109, 7845; Scaringe et al., 1990 Nucleic Acids Res., 18, 5433; and Wincott et al., 1995 Nucleic Acids Res. 23, 2677-2684 and makes use of common nucleic acid protecting and coupling groups, such as dimethoxytrityl at the 5'-end, and phosphoramidites at the 3'-end. Small scale synthesis were conducted on a 394 Applied Biosystems, Inc. synthesizer using a modified 2.5 μmol scale protocol with a 5 min coupling step for alkylsilyl protected nucleotides and 2.5 min coupling step for 2'-*O*-methylated nucleotides. Table V outlines the amounts, and the contact times, of the reagents used in the synthesis cycle. A 6.5-fold excess (163 μL of 0.1 M = 16.3 μmol) of phosphoramidite and a 24-fold excess of S-ethyl tetrazole (238 μL of 0.25 M = 59.5 μmol) relative to polymer-bound 5'-hydroxyl was used in each coupling cycle. Average coupling yields on the 394 Applied Biosystems, Inc. synthesizer, determined by colorimetric quantitation of the trityl fractions, were 97.5-99%. Other oligonucleotide synthesis reagents for the 394 Applied Biosystems, Inc. synthesizer: detritylation solution was 2% TCA in methylene chloride (ABI); capping was performed with 16% *N*-methyl imidazole in THF (ABI) and 10% acetic anhydride/10% 2,6-lutidine in THF (ABI); oxidation solution was 16.9 mM $I_2$, 49 mM pyridine, 9% water in THF (Millipore). B & J Synthesis Grade acetonitrile was used directly from the reagent bottle. *E*-Ethyl tetrazole solution (0.25 M in acetonitrile) was made up from the solid obtained from American International Chemical, Inc.

Deprotection of the RNA was performed as follows. The polymer-bound oligoribonucleotide, trityl-off, was transferred from the synthesis column to a 4mL glass screw top vial and suspended in a solution of methylamine (MA) at 65 °C for 10 min. After cooling to -20 °C, the supernatant was removed from the polymer support. The support was washed three times with 1.0 mL of EtOH:MeCN:$H_2O$/3:1:1, vortexed and the supernatant was then added to the first supernatant. The combined supernatants, containing the oligoribonucleotide, were dried to a white powder.

The base-deprotected oligoribonucleotide was resuspended in anhydrous TEA•HF/NMP solution (250 μL of a solution of 1.5mL *N*-methylpyrrolidinone, 750 μL TEA and 1.0 mL TEA•3HF to provide a 1.4M HF concentration) and heated to 65°C for 1.5 h. The resulting, fully deprotected, oligomer was quenched with 50 mM TEAB (9 mL) prior to anion exchange desalting.

For anion exchange desalting of the deprotected oligomer, the TEAB solution was loaded onto a Qiagen 500® anion exchange cartridge (Qiagen Inc.) that was prewashed with 50 mM TEAB (10 mL). After washing the loaded cartridge with 50 mM TEAB (10 mL), the RNA was eluted with 2 M TEAB (10 mL) and dried down to a white powder.

Inactive hammerhead ribozymes were synthesized by substituting a U for $G_5$ and a U for $A_{14}$ (numbering from Hertel, K. J., et al., 1992, Nucleic Acids Res., 20, 3252).

The average stepwise coupling yields were >98% (Wincott et al., 1995 Nucleic Acids Res. 23, 2677-2684).

Hairpin ribozymes are synthesized in two parts and annealed to reconstruct the active ribozyme (Chowrira and Burke, 1992 Nucleic Acids Res., 20, 2835-2840). Ribozymes are also synthesized from DNA templates using bacteriophage T7 RNA polymerase (Milligan and Uhlenbeck, 1989, Methods Enzymol. 180, 51).

All ribozymes are modified extensively to enhance stability by modification with nuclease resistant groups, for example, 2'-amino, 2'-*C*-allyl, 2'-flouro, 2'-*O*-methyl, 2'-H (for a review see Usman and Cedergren, 1992 TIBS 17, 34;

Usman et al., 1994 Nucleic Acids Symp. Ser. 31, 163). Ribozymes are purified by gel electrophoresis using general methods or are purified by high pressure liquid chromatography (HPLC; See Usman et al., PCT Publication No. WO95/23225 and are resuspended in water.

The sequences of the ribozymes that are chemically synthesized, useful in this study, are shown in Tables II to III. Those in the art will recognize that these sequences are representative only of many more such sequences where the enzymatic portion of the ribozyme (all but the binding arms) is altered to affect activity. Stem-loop IV sequence of hairpin ribozymes listed in for example Table III (5'-CACGUUGUG-3') can be altered (substitution, deletion, and/or insertion) to contain any sequence, provided a minimum of two base-paired stem structure can form. The sequences listed in Tables II to III may be formed of ribonucleotides or other nucleotides or non-nucleotides. Such ribozymes are equivalent to the ribozymes described specifically in the Tables.

optimizing Ribozyme Activity

Ribozyme activity can be optimized as described by Stinchcomb *et al*., supra. The details will not be repeated here, but include altering the length of the ribozyme binding arms (stems I and III, see Figure 2c), or chemically synthesizing ribozymes with modifications that prevent their degradation by serum ribonucleases (see *e.g.*, Eckstein et al., International Publication No. WO 92/07065; Perrault et al., 1990 Nature 344, 565; Pieken et al., 1991 Science 253, 314; Usman and Cedergren, 1992 Trends in Biochem. Sci. 17, 334; Usman *et al*., International Publication No. WO 93/15187; Rossi et al., International Publication No. WO 91/03162; Beigelman et al., 1995 J. Biol Chem. in press; as well as Sproat, US Patent No. 5,334,711 which describe various chemical modifications that can be made to the sugar moieties of enzymatic RNA molecules). Modifications which enhance their efficacy in cells, and removal of stem II bases to shorten RNA synthesis times and reduce chemical requirements are desired.

Sullivan, *et al*., *supra*, describes the general methods for delivery of enzymatic RNA molecules. Ribozymes may be administered to cells by a variety of methods known to those familiar to the art, including, but not restricted to, encapsulation in liposomes, by ionto-phoresis, or by incorporation into other vehicles, such as hydrogels, cyclodextrins, biodegradable nanocapsules, and bioadhesive microspheres. For some indications, ribozymes may be directly delivered *ex vivo* to cells or tissues with or without the aforementioned vehicles. Alternatively, the RNA/vehicle combination is locally delivered by direct injection or by use of a catheter, infusion pump or stent. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of ribozyme delivery and administration are provided in Sullivan *et al*., supra and Draper *et al., supra*.

Another means of accumulating high concentrations of a ribozyme(s) within cells is to incorporate the ribozyme-encoding sequences into a DNA or RNA expression vector. Transcription of the ribozyme sequences are driven from a promoter for eukaryotic RNA polymerase I (pol I), RNA polymerase II (pol II), or RNA polymerase III (pol III). Transcripts from pol II or pol III promoters will be expressed at high levels in all cells; the levels of a given pol II promoter in a given cell type will depend on the nature of the gene regulatory sequences (enhancers, silencers, etc.) present nearby. Prokazyotic RNA polymerase promoters are also used, providing that the prokaryotic RNA polymerase enzyme is expressed in the appropriate cells (Elroy-Stein and Moss, 1990 Proc. Natl. Acad. Sci. U S A, 87, 6743-7; Gao and Huang 1993 Nucleic Acids Res., 21, 2867-72; Lieber et al., 1993 Methods Enzymol., 217, 47-66; Zhou et al., 1990 Mol. Cell. Biol., 10, 4529-37; Thompson *et al*., 1995 *supra*). Several investigators have demonstrated that ribozymes expressed from such promoters can function in mammalian cells (*e.g.* Kashani-Sabet et al., 1992 Antisense Res. Dev., 2, 3-15; Ojwang et al., 1992 Proc. Natl. Acad. Sci. U S A, 89, 10802-6; Chen et al., 1992 Nucleic Acids Res., 20, 4581-9; Yu et al., 1993 Proc. Natl. Acad. Sci. U S A, 90, 6340-4; L'Huillier et al., 1992 EMBO J. 11, 4411-8; Lisziewicz et al., 1993 Proc. Natl. Acad. Sci. U. S. A., 90, 8000-4; Thompson et al., 1995 Nucleic Acids Res. 23, 2259). The above ribozyme transcription units can be incorporated into a variety of vectors for introduction into mammalian cells, including but not restricted to, plasmid DNA vectors, viral DNA vectors (such as adenovirus or adeno-associated virus vectors), or viral RNA vectors (such as retroviral or alphavirus vectors).

Disclosed herein is transcription unit expressing a ribozyme that cleaves RNAs that encode flt-1, KDR and/or flk-1 that is inserted into a plasmid DNA vector or an adenovirus or adeno-associated virus DNA viral vector or a retroviral RNA vector. Viral vectors have been used to transfer genes and lead to either transient or long term gene expression (Zabner et al., 1993 Cell 75, 207; Carter, 1992 Curr. Opi. Biotech. 3, 533). The adenovirus, AAV or retroviral vector is delivered as recombinant viral particles. The DNA may be delivered alone or complexed with vehicles (as described for RNA above). The recombinant adenovirus or AAV or retroviral particles are locally administered to the site of treatment, e.g., through incubation or inhalation *in vivo* or by direct application to cells or tissues *ex vivo*. Retroviral vectors have also been used to express ribozymes in mammalian cells (Ojwang *et al*., 1992 *supra;* Thompson *et al*., 1995 *supra*).

flt-1, KDR and/or flk-1 are attractive nucleic acid-based therapeutic targets by several criteria. The interaction between VEGF and VEGF-R is well-established. Efficacy can be tested in well-defined and predictive animal models. Finally, the disease conditions are serious and current therapies are inadequate. Whereas protein-based therapies would inhibit VEGF activity nucleic acid-based therapy provides a direct and elegant approach to directly modulate flt-1, KDR and/or flk-1 expression.

Because flt-1 and KDR mRNAs are highly homologous in certain regions, some ribozyme target sites are also homologous (see Table IV). In this case, a single ribozyme will target both flt-1 and KDR mRNAs. At partially homologous sites, a single ribozyme can sometimes be designed to accomodate a site on both mRNAs by including G/U basepairing. For example, if there is a G present in a ribozyme target site in KDR mRNA at the same position there is an A in the flt-1 ribozyme target site, the ribozyme can be synthesized with a U at the complementary position and it will bind both to sites. The advantage of one ribozyme that targets both VEGF-R mRNAs is clear, especially in cases where both VEGF receptors may contribute to the progression of angiogenesis in the disease state.

"Angiogenesis" refers to formation of new blood vessels which is an essential process in reproduction, development and wound repair. "Tumor angiogenesis" refers to the induction of the growth of blood vessels from surrounding tissue into a solid tumor. Tumor growth and tumor metastasis are dependent on angiogenesis (for a review see Folkman, 1985 *supra*; Folkman 1990 J. Natl. Cancer Inst., 82, 4; Folkman and Shing, 1992 J. Biol. Chem. 267, 10931).

Angiogenesis plays an important role in other diseases such as arthritis wherein new blood vessels have been shown to invade the joints and degrade cartilage (Folkman and Shing, *supra*).

"Retinopathy" refers to inflammation of the retina and/or degenerative condition of the retina which may lead to occlusion of the retina and eventual blindness. In "diabetic retinopathy" angiogenesis causes the capillaries in the retina to invade the vitreous resulting in bleeding and blindness which is also seen in neonatal retinopathy (for a review see Folkman, 1985 *supra*; Folkman 1990 *supra*; Folkman and Shing, 1992 *supra).*

Example 1: flt-1, KDR and/or flk-1 ribozymes

By engineering ribozyme motifs applicant has designed several ribozymes directed against flt-1, KDR and/or flk-1 encoded mRNA sequences. These ribozymes were synthesized with modifications that improve their nuclease resistance (Beigelman et al., 1995 J Biol. Chem. 270, 25702) and enhance their activity in cells. The ability of ribozymes to cleave target sequences *in vitro* was evaluated essentially as described in Thompson et al., PCT Publication No. WO 93/23057; Draper et al., PCT Publication No. WO 95/04818.

Example 2: Effect of ribozymes on the binding of VEGF to flt-1, KDR and/or flk-1 receptors

Several common human cell lines are available that express endogenous flt-1, KDR and/or flk-1. flt-1, KDR and/or flk-1 can be detected easily with monoclonal antibodies. Use of appropriate fluorescent reagents and fluorescence-activated cell-sorting (FACS) will permit direct quantitation of surface flt-1, KDR and/or flk-1 on a cell-by-cell basis. Active ribozymes are expected to directly reduce flt-1, KDR and/or flk-1 expression and thereby reduce VEGF binding to the cells. In this example, human umbelical cord microvascular endothelial cells were used.

Cell Preparation:

Plates are coated with 1.5% gelatin and allowed to stand for one hour. Cells (e.g., microvascular endothelial cells derived from human umbilical cord vein) are plated at 20,000 cells/well (24 well plate) in 200 ml growth media and incubated overnight (- 1 doubling) to yield -40,000 cells (75-80% confluent).

Ribozyme treatment:

Media is removed from cells and the cells are washed two times with 300 ml 1X PBS: $Ca^{2+}$: $Mg^{2+}$ mixture. A complex of 200-500 nM ribozyme and LipofectAMINE® (3:1 lipid: phosphate ratio) in 200 ml OptiMEM® (5% FBS) was added to the cells. The cells are incubated for 6 hr (equivalent to 2-3 VEGF-R turnovers).

$^{125}$I VEGF binding assay:

The assay is carried out on ice to inhibit internalization of VEGF during the experiment. The media containing the ribozyme is removed from the cells and the cells are washed twice with with 300 ml 1X PBS: $Ca^{2+}$: $Mg^{2+}$ mixture containing 1% BSA. Appropriate $^{125}$I VEGF solution (100,000 cpm/well, +/- 10 X cold 1X PBS, 1% BSA) was applied to the cells. The cells are incubated on ice for 1 h. $^{125}$I VEGF-containing solution is removed and the cells are washed three times with with 300 ml 1X PBS: $Ca^{2+}$: $Mg^{2+}$ mixture containing 1% BSA. To each well 300 ml of 100 mM Tris-HCl, pH 8.0, 0.5% Triton X-100 was added and the the mixture was incubated for 2 min. The $^{125}$I VEGF-binding was quantitated using standard scintillation counting techniques. Percent inhibition was calculated as follows:

$$\text{Percent Inhibition} = \frac{\text{cpm } ^{125}\text{I VEGF bound by the ribozyme-treated samples}}{\text{cpm } ^{125}\text{I VEGF bound by the Control sample}} \times 100$$

Example 3: Effect of hammerhead ribozymes targeted against flt-1 receptor on the binding of VEGF

Hammerhead ribozymes targeted to twenty sites within flt-1 RNA were synthesized as described above. Sequence of the ribozymes used are shown in Table II; the length of stem II region is 3 bp. The hammerhead ribozymes were chemically modified such that the ribozyme consists of ribose residues at five positions; U4 and U7 positions contain 2'-$NH_2$ modifications, the remaining nucleotide positions contain 2'-*O*-methyl substitutions; four nucleotides at the 5' terminus contains phosphorothioate substitutions. Additionally, 3' end of the ribozyme contains a 3'-3' linked inverted abasic ribose.

Referring to Figure 7, the effect of hammerhead ribozymes targeted against flt-1 receptor on the binding of VEGF to flt-1 on the surface of human microvascular endothelial cells is shown. The majority of the ribozymes tested were able to inhibit the expression of flt-1 and thereby were able to inhibit the binding of VEGF.

In order to determine the specificity of ribozymes targeted against flt-1 RNA, the effect of five anti-flt-1 ribozymes on the binding of VEGF, UPA (urokinase plasminogen activator) and FGF (fibroblast growth factor) to their corresponding receptors were assayed. As shown in Figure 9, there was significant inhibition of VEGF binding to its receptors on cells treated with anti-flt-1 ribozymes. There was no specific inhibition of the binding of UPA and FGF to their corresponding receptors. These data strongly suggest that anti-flt-1 ribozymes specifically cleave flt-1 RNA and not RNAs encoding the receptors for UPA and FGF, resulting in the inhibition of flt-1 receptor expression on the surface of the cells. Thus the ribozymes are responsible for the inhibition of VEGF binding but not the binding of UPA and FGF.

Example 4: Effect of ribozvmes targeted against VEGF receptors on cell proliferation

Cell Preparation:

24-well plates are coated with 1.5% gelatin (porcine skin 300 bloom). After 1 hr, excess gelatin is washed off of the plate. Microvascular endothelial cells are plated at 5,000 cells/well (24 well plate) in 200 ml growth media. The cells are allowed to grow for - 18 hr (- 1 doubling) to yield -10,000 cells (25-30% confluent).

Ribozyme treatment:

Media is removed from the cells, and the cells are washed two times with 300 ml 1X PBS: $Ca^{2+}$: $Mg^{2+}$ mixture.

For anti-flt-1 HH ribozyme experiment (Figure 12) a complex of 500 nM ribozyme; 15 mM LFA (3:1 lipid: phosphate ratio) in 200 ml OptiMEM (5% FCS) media was added to the cells. Incubation of cells is carried out for 6 hr (equivalent to 2-3 VEGF receptor turnovers).

Proliferation:

After three or six hours, the media is removed from the cells and the cells are washed with 300 ml 1X PBS: $Ca^{2-}$: $Mg^{2+}$ mixture. Maintenance media (contains dialyzed 10% FBS) +/- VEGF or basic FGF at 10 ng/ml is added to the cells. The cells are incubated for 48 or 72 h. The cells are trypsinized and counted (Coulter counter). Trypan blue is added on one well of each treatment as control.

As shown in Figure 10B, VEGF and basic FGF can stimulate human microvascular endothelial cell proliferation. However, treatment of cells with 1358 HH or 4229 HH ribozymes, targeted against flt-1 mRNA, results in a significant decrease in the ability of VEGF to stimulate endothelial cell proliferation. These ribozymes do not inhibit the FGF-mediated stimulation of endothelial cell proliferation.

These results strongly suggest that hammerhead ribozymes targeted against flt-1 mRNA can specifically inhibit VEGF-mediated induction of endothelial cell proliferation.

Example 5: *In vitro* cleavage of flt-1 RNA by hammerhead ribozymes

Referring to Figure 9A, hammerhead ribozymes (HH) targeted against sites 1358 and 4229 within the flt-1 RNA were synthesized as described above.

RNA cleavage assay *in vitro*:

Substrate RNA was 5' end-labeled using [g-$^{32}$P] ATP and T4 polynucleotide kinase (US Biochemicals). Cleavage reactions were carried out under ribozyme "excess" conditions. Trace amount (s 1 nM) of 5' end-labeled substrate and 40 nM unlabeled ribozyme were denatured and renatured separately by heating to 90°C for 2 min and snap-cooling on ice for 10-15 min. The ribozyme and substrate were incubated, separately, at 37°C for 10 min in a buffer containing 50 mM Tris-HCl and 10 mM $MgCl_2$. The reaction was initiated by mixing the ribozyme and substrate solutions and incubating at 37°C. Aliquots of 5 ml are taken at regular intervals of time and the reaction is quenched by mixing with equal volume of 2X formamide stop mix. The samples are resolved on 20 % denaturing polyacrylamide gels. The results were quantified and percentage of target RNA cleaved is plotted as a function of time.

Referring to Figure 9B and 9C, hammerhead ribozymes targeted against sites 1358 and 4229 within the flt-1 RNA are capable of cleaving target RNA efficiently *in vitro.*

Example 6: *In* vitro cleavage of RNA by hammerhead ribozymes targeted against cleavage sites that are homologous between KDR and flt-1 mRNA

Because flt-1 and KDR mRNAs are highly homologous in certain regions, some ribozyme target sites are also homologous (see Table X). In this case, a single ribozyme will target both flt-1 and KDR mRNAs. Hammerhead ribozyme (FLT/KDR-I) targeted against one of the homologous sites between flt-1 and KDR (flt-1 site 3388 and KDR site 3151) was synthesized as described above. Ribozymes with either a 3 bp stem II or a 4 bp stem II were synthesized. RNA cleavage reactions were carried out *in vitro* essentially as described under Example 5.

Referring to Figure 11, FLT/KDR-I ribozyme with either a 3 or a 4 bp stem II was able to cleave its target RNA efficiently *in vitro.*

Example 7: Effect of multiple ribozymes targeted against both flt-1 and KDR RNA on cell proliferation

Since both flt-1 and KDR receptors of VEGF are involved in angiogenesis, the inhibition of the expression of both of these genes may be an effective approach to inhibit angiogenesis.

Human microvascular endothalial cells were treated with Hammerhead ribozymes targeted against sites flt-1 4229 alone, KDR 527 alone, KDR 726 alone, KDR 3950 alone, flt-1 4229 + KDR 527, flt-1 4229 + KDR 726 or flt-1 4229 + KDR 3950. As shown in Figure 12, all the combinations of active ribozymes (A) caused significant inhibition of VEGF-mediated induction of cell proliferation. No significant inhibition of cell proliferation was observed when the cells were treated with a catalytically inactive (I) hammerhead ribozymes. Additionally, cells treated with ribozymes targeted against both flt-1 and KDR RNAs-flt-1 4229 + KDR 527; flt-1 4229 + KDR 726; flt-1 4229 + KDR 3950, were able to cause a greater inhibition of VEGF-mediated induction of cell proliferation when compared with individual ribozymes targeted against either flt-1 or KDR RNA (see flt-1 4229 alone; KDR 527 alone; KDR 726 alone; KDR 3950 alone). This strongly suggests that treatment of cells with multiple ribozymes may be a more effective means of inhibition of gene expression.

Animal Models

There are several animal models in which the anti-angiogenesis effect of nucleic acids of the present invention, such as ribozymes, directed against VEGF-R mRNAs can be tested. Typically a corneal model has been used to study angiogenesis in rat and rabbit since recruitment of vessels can easily be followed in this normally avascular tissue (Pandey et al., 1995 Science 268: 567-569). In these models, a small Teflon or Hydron disk pretreated with an angiogenesis factor (e.g. bFGF or VEGF) is inserted into a pocket surgically created in the cornea. Angiogenesis is monitored 3 to 5 days later. Ribozymes directed against VEGF-R mRNAs would be delivered in the disk as well, or dropwise to the eye over the time course of the experiment. In another eye model, hypoxia has been shown to cause both increased expression of VEGF and neovascularization in the retina (Pierce et al., 1995 Proc. Natl. Acad. Sci. USA. 92: 905-909; Shweiki et al., 1992 J. Clin. Invest. 91: 2235-2243).

In human glioblastomas, it has been shown that VEGF is at least partially responsible for tumor angiogenesis (Plate et al., 1992 Nature 359, 845). Animal models have been developed in which glioblastoma cells are implanted subcutaneously into nude mice and the progress of tumor growth and angiogenesism is studied (Kim *et al*., 1993 *supra*; Millauer *et al*., 1994 *supra*).

Another animal model that addresses neovascularization involves Matrigel, an extract of basement membrane that becomes a solid gel when injected subcutaneously (Passaniti et al., 1992 Lab. Invest. 67: 519-528). When the Matrigel is supplemented with angiogenesis factors such as VEGF, vessels grow into the Matrigel over a period of 3 to 5 days and angiogenesis can be assessed. Again, ribozymes directed against VEGF-R mRNAs would be delivered in the Matrigel.

Several animal models exist for screening of anti-angiogenic agents. These include corneal vessel formation following corneal injury (Burger et al., 1985 Cornea 4: 35-41; Lepri, et al., 1994 J. Ocular Pharmacol. 10: 273-280; Ormerod et al., 1990 Am. J. Pathol. 137: 1243-1252) or intracorneal growth factor implant (Grant et al., 1993 Diabetologia 36: 282-291; Pandey *et al*. 1995 *supra;* Zieche et al., 1992 Lab. Invest. 67: 711-715), vessel growth into Matrigel matrix containing growth factors (Passaniti *et al*., 1992 *supra*), female reproductive organ neovascularization following hormonal manipulation (Shweiki et al., 1993 Clin. Invest. 91: 2235-2243), several models involving inhibition of tumor growth in highly vascularized solid tumors (O'Reilly et al., 1994 Cell 79: 315-328; Senger et al., 1993 Cancer and Metas. Rev. 12: 303-324; Takahasi et al., 1994 Cancer Res. 54: 4233-4237; Kim *et al*., 1993 *supra*), and transient hypoxia-induced neovascularization in the mouse retina (Pierce et al., 1995 Proc. Natl. Acad. Sci. USA. 92: 905-909).

The cornea model, described in Pandey *et al*. *supra*, is the most common and well characterized anti-angiogenic agent efficacy screening model. This model involves an avascular tissue into which vessels are recruited by a stimulating agent (growth factor, thermal or alkalai burn, endotoxin). The corneal model would utilize the intra-stromal corneal implantation of a Teflon pellet soaked in a VEGF-Hydron solution to recruit blood vessels toward the pellet which can be quantitated using standard microscopic and image analysis techniques. To evaluate their anti-angiogenic efficacy, ribozymes are applied topically to the eye or bound within Hydron on the Teflon pellet itself. This avascular cornea as well as the Matrigel (see below) provide for low background assays. While the corneal model has been performed extensively in the rabbit, studies in the rat have also been conducted.

The mouse model (Passaniti et al., *supra*) is a non-tissue model which utilizes Matrigel, an extract of basement membrane (Kleinman et al., 1986) or Millipore® filter disk, which can be impregnated with growth factors and anti-angiogenic agents in a liquid form prior to injection. Upon subcutaneous administration at body temperature, the Matrigel or Millipore® filter disk forms a solid implant. VEGF embedded in the Matrigel or Millipore® filter disk would be used to recruit vessels within the matrix of the Matrigel or Millipore® filter disk which can be processed histologically for endothelial cell specific vWF (factor VIII antigen) immunohistochemistry, Trichrome-Masson stain, or hemoglobin content. Like the cornea, the Matrigel or Millipore® filter disk are avascular; however, it is not tissue. In the Matrigel or Millipore® filter disk model, ribozymes are administered within the matrix of the Matrigel or Millipore® filter disk to test their anti-angiogenic efficacy. Thus, delivery issues in this model, as with delivery of ribozymes by Hydron-coated Teflon pellets in the rat cornea model, may be less problematic due to the homogeneous presence of the ribozyme within the respective matrix.

These models offer a distinct advantage over several other angiogenic models listed previously. The ability to use VEGF as a pro-angiogenic stimulus in both models is highly desirable since ribozymes will target only VEGFr mRNA. In other words, the involvement of other non-specific types of stimuli in the cornea and Matrigel models is not advantageous from the standpoint of understanding the pharmacologic mechanism by which the anti-VEGFr mRNA ribozymes produce their effects. In addition, the models will allow for testing the specificity of the anti-VEGFr mRNA ribozymes by using either a- or bFGF as a pro-angiogenic factor. Vessel recruitment using FGF should not be affected in either model by anti-VEGFr mRNA ribozymes. Other models of angiogenesis including vessel formation in the female reproductive system using hormonal manipulation (Shweiki *et al*., 1993 *supra*); a variety of vascular solid tumor models which involve indirect correlations with angiogenesis (O'Reilly *et al,*, 1994 *supra*; Senger *et al*., 1993 *supra*; Takahasi *et al*., 1994 *supra*; Kim *et al*., 1993 *supra*); and retinal neovascularization following transient hypoxia (Pierce *et al*., 1995 *supra*) were not selected for efficacy screening due to their non-specific nature, although there is a correlation between VEGF and angiogenesis in these models.

Other model systems to study tumor angiogenesis is reviewed by Folkman, 1985 Adv. Cancer. Res.. 43, 175.

flt-1, KDR and/or flk-1 protein levels can be measured clinically or experimentally by FACS analysis flt-1, KDR and/or flk-1 encoded mRNA levels will be assessed by Northern analysis, RNase-protection, primer extension analysis and/or quantitative RT-PCR. Ribozymes that block flt-1, KDR and/or flk-1 protein encoding mRNAs and therefore result in decreased levels of flt-1, KDR and/or flk-1 activity by more than 20% *in vitro* will be identified.

Ribozymes and/or genes encoding them are delivered by either free delivery, liposome delivery, cationic lipid delivery, adeno-associated virus vector delivery, adenovirus vector delivery, retrovirus vector delivery or plasmid vector delivery in these animal model experiments (see above).

Patients can be treated by locally administering nucleic acids targeted against VEGF-R by direct injection. Routes of administration may include, but are not limited to, intravascular, intramuscular, subcutaneous, intra-articular, aerosol inhalation, oral (tablet, capsule or pill form), topical, systemic, ocular, intraperitoneal and/or intrathecal delivery.

Example 8: Ribozyme -mediated inhibition of angiogenesis *in vivo*

The purpose ot this study was to assess the anti-angiogenic activity of hammerhead ribozymes targeted against flt-1 4229 site in the rat cornea model of VEGF induced angiogenesis (see above). These ribozymes have either active or inactive catalytic core and either bind and cleave or just bind to VEGF-R mRNA of the flt-1 subtype. The active ribozymes, that are able to bind and cleave the target RNA, have been shown to inhibit ($^{125}$I-labeled) VEGF binding in cultured endothelial cells and produce a dose-dependent decrease in VEGF induced endothelial cell proliferation in these cells (see Examples 3-4 above). The catalytically inactive forms of these ribozymes, wherein the ribozymes can only bind to the RNA but cannot catalyze RNA cleavage, fail to show these characteristics. The ribozymes and VEGF were co-delivered using the filter disk method: Nitrocellulose filter disks (Millipore®) of 0.057 diameter were immersed in appropriate solutions and were surgically implanted in rat cornea as described by Pandey *et al*., *supra*. This delivery method has been shown to deliver rhodamine-labeled free ribozyme to scleral cells and, in all likelihood cells of the pericorneal vascular plexus. Since the active ribozymes show cell culture efficacy and can be delivered to the target site using the disk method, it is essential that these ribozymes be assessed for *in vivo* anti-angiogenic activity.

The stimulus for angiogenesis in this study was the treatment of the filter disk with 30 mM VEGF which is implanted within the cornea's stroma. This dose yields reproducible neovascularization stemming from the pericorneal vascular plexus growing toward the disk in a dose-response study 5 days following implant. Filter disks treated only with the vehicle for VEGF show no angiogenic response. The ribozymes was co-adminstered with VEGF on a disk in two different ribozyme concentrations. One concern with the simultaneous administration is that the ribozymes will not be able to inhibit angiogenesis since VEGF receptors can be stimulated. However, we have observed that in low VEGF doses, the neovascular response reverts to normal suggesting that the VEGF stimulus is essential for maintaining the angiogenic response. Blocking the production of VEGF receptors using simultaneous administration of anti-VEGF-R mRNA ribozymes could attenuate the normal neovascularization induced by the filter disk treated with VEGF.

Materials and Methods:

1. Stock hammerhead ribozyme solutions:

a. flt-1 4229 (786 μM)- Active
b. flt-1 4229 (736 μM)- Inactive

2. Experimantal solutions/groups:

| | | |
|---|---|---|
| Group 1 | Solution 1 | Control VEGF solution: 30 μM in 82mM Tris base |
| Group 2 | Solution 2 | flt-1 4229 (1 μg/μL) in 30 μM VEGF/82 mM Tris base |
| Group 3 | Solution 3 | flt-1 4229 (10 μg/μL) in 30 μM VEGF/82 mM Tris base |
| Group 4 | Solution 4 | No VEGF, flt-1 4229 (10 μg/μL) in 82 mM Tris base |
| Group 5 | Solution 5 | No VEGF, No ribozyme in 82 mM Tris base |

10 eyes per group, 5 animals (Since they have similar molecular weights, the molar concentrations should be essentially similar).

Each solution (VEGF and RIBOZYMES) were prepared as a 2X solution for 1:1 mixing for final concentrations above, with the exception of solution 1 in which VEGF was 2X and diluted with ribozyme diluent (sterile water).

3. VEGF Solutions

The 2X VEGF solution (60 μM) was prepared from a stock of 0.82 μg/μL in 50 mM Tris base. 200 μL of VEGF stock was concentrated by speed vac to a final volume of 60.8 μL, for a final concentration of 2.7 μg/μL or 60 μM. Six 10 μL aliquots was prepared for daily mixing. 2X solutions for VEGF and Ribozyme was stored at 4°C until the day of the surgery. Solutions were mixed for each day of surgery. Original 2X solutions was prepared on the day before the first day of the surgery.

4. Surgical Solutions:

Anesthesia:

stock ketamine hydrochloride 100 mg/mL
stock xylazine hydrochloride 20 mg/mL

stock acepromazine 10 mg/mL

Final anesthesia solution: 50 mg/mL ketamine, 10 mg/mL xylazine, and 0.5 mg/mL acepromazine

5% povidone iodine for opthalmic surgical wash
2% lidocaine (sterile) for opthalmic administration (2 drops per eye)
sterile 0.9% NaCl for opthalmic irrigation

5. Surgical Methods:

Standard surgical procedure as described in Pandey et al., supra. Filter disks were incubated in 1 μL of each solution for approximately 30 minutes prior to implantation.

5. Experimental Protocol:

The animal cornea were treated with the treatment groups as described above. Animals were allowed to recover for 5 days after treatment with daily observation (scoring 0 - 3). On the fifth day animals were euthanized and digital images of each eye was obtained for quantitaion using Image Pro Plus. Quantitated neovascular surface area were analyzed by ANOVA followed by two post-hoc tests including Dunnets and Tukey-Kramer tests for significance at the 95% confidence level. Dunnets provide information on the significance between the differences within the means of treatments vs. controls while Tukey-Kramer provide information on the significance of differences within the means of each group.

Results are graphically represented in Figure 13. As shown in the figure, flt-1 4229 active hammerhead ribozyme at both concentrations was effective at inhibiting angiogenesis while the inactive ribozyme did not show any significant reduction in angiogenesis. A statistically signifiant reduction in neovascular surface area was observed only with active ribozymes. This result clearly shows that the ribozymes are capable of significantly inhibiting angiogenesis *in vivo.* Specifically, the mechanism of inhibition appears to be by the binding and cleavage of target RNA by ribozymes.

Diagnostic uses

The ribozymes disclosed herein may be used as diagnostic tools to examine genetic drift and mutations within diseased cells or to detect the presence of flt-1, KDR and/or flk-1 RNA in a cell. The close relationship between ribozyme activity and the structure of the target RNA allows the detection of mutations in any region of the molecule which alters the base-pairing and three-dimensional structure of the target RNA. By using multiple ribozymes disclosed herein, one may map nucleotide changes which are important to RNA structure and function in vitro, as well as in cells and tissues. Cleavage of target RNAs with ribozymes may be used to inhibit gene expression and define the role (essentially) of specified gene products in the progression of disease. In this manner, other genetic targets may be defined as important mediators of the disease. These experiments will lead to better treatment of the disease progression by affording the possibility of combinational therapies (e.g., multiple ribozymes targeted to different genes, ribozymes coupled with known small molecule inhibitors, or intermittent treatment with combinations of ribozymes and/or other chemical or biological molecules). Other *in vitro* uses of ribozymes disclosed herein are well known in the art, and include detection of the presence of mRNAs associated with flt-1, KDR and/or flk-1 related condition. Such RNA is detected by determining the presence of a cleavage product after treatment with a ribozyme using standard methodology.

In a specific example, ribozymes which can cleave only wild-type or mutant forms of the target RNA are used for the assay. The first ribozyme is used to identify wild-type RNA present in the sample and the second ribozyme will be used to identify mutant RNA in the sample. As reaction controls, synthetic substrates of both wild-type and mutant RNA will be cleaved by both ribozymes to demonstrate the relative ribozyme efficiencies in the reactions and the absence of cleavage of the "non-targeted" RNA species. The cleavage products from the synthetic substrates will also serve to generate size markers for the analysis of wild-type and mutant RNAs in the sample population. Thus each analysis will require two ribozymes, two substrates and one unknown sample which will be combined into six reactions. The presence of cleavage products will be determined using an RNAse protection assay so that full-length and cleavage fragments of each RNA can be analyzed in one lane of a polyacrylamide gel. It is not absolutely required to quantify the results to gain insight into the expression of mutant RNAs and putative risk of the desired phenotypic changes in target cells. The expression of mRNA whose protein product is implicated in the development of the phenotype (i.e., flt-1, KDR and/or flk-1) is adequate to establish risk. If probes of comparable specific activity are used for both transcripts, then a qualitative comparison of RNA levels will be adequate and will decrease the cost of the initial diagnosis. Higher mutant form to wild-type ratios will be correlated with higher risk whether RNA levels are compared qualitatively or quantitatively.

Other embodiments are within the following claims.

Table I

Characteristics of Ribozymes

Group I Introns

Size: -200 to >1000 nucleotides
Requires a U in the target sequence immediately 5' of the cleavage site.
Binds 4-6 nucleotides at 5' side of cleavage site.
Over 75 known members of this class. Found in Tetrahymena *thermophila* rRNA, fungal mitochondria, chloroplasts, phage T4, blue-green algae, and others.

RNAseP RNA (M1 RNA)

Size: -290 to 400 nucleotides
RNA portion of a ribonucleoprotein enzyme. Cleaves tRNA precursors to form mature tRNA.
Roughly 10 known members of this group all are bacterial in origin.

Hammerhead Ribozyme

Size: ~13 to 40 nucleotides.
Requires the target sequence UH immediately 5' of the cleavage site.
Binds a variable number of nucleotides on both sides of the cleavage site.
14 known members of this class. Found in a number of plant pathogens (virusoids) that use RNA as the infectious agent (Figure 1 and 2)

Hairpin Ribozyme

Size: ~50 nucleotides.
Requires the target sequence GUC immediately 3' of the cleavage site.
Binds 4-6 nucleotides at 5' side of the cleavage site and a variable number to the 3' side of the cleavage site. Only 3 known member of this class. Found in three plant pathogen (satellite RNAs of the tobacco ringspot virus, arabis mosaic virus and chicory yellow mottle virus) which uses RNA as the infectious agent (Figure 3).

Hepatitis Delta Virus (HDV) Ribozyme

Size: 50-60 nucleotides (at present)
Sequence requirements not fully determined.
Binding sites and structural requirements not fully determined, although no sequences 5' of cleavage site are required.
Only 1 known member of this class. Found in human HDV (Figure 4).

*Neurospora* VS RNA Ribozyme

Size: -144 nucleotides (at present)
Cleavage of target RNAs recently demonstrated.
Sequence requirements not fully determined.
Binding sites and structural requirements not fully determined. Only 1 known member of this class. Found in Neurospora VS RNA (Figure 5).

Table II: Human flt1 VEGF Receptor-Hammerhead Ribozyme and Substrate Sequence

| nt. Position | HH Ribozyme | Substrate |
|---|---|---|
| 10 | GCCGAGAG CUGAUGA X GAA AGUGUCCG | CGGACACUC CUCUCGGC |
| 13 | GGAGCCGA CUGAUGA X GAA AGGAGUGU | ACACUCCUC UCGGCUCC |
| 15 | GAGGAGCC CUGAUGA X GAA AGAGGAGU | ACUCCUCUC GGCUCCUC |
| 20 | CCGGGGAG CUGAUGA X GAA AGCCGAGA | UCUCGGCUC CUCCCCGG |
| 23 | CUGCCGGG CUGAUGA X GAA AGGAGCCG | CGGCUCCUC CCCGGCAG |

(continued)

| nt. Position | HH Ribozyme | Substrate |
|---|---|---|
| 43 | CCCGCUCC CUGAUGA X GAA AGCCGCCG | CGGCGGCUC GGAGCGGG |
| 54 | GAGCCCCG CUGAUGA X GAA AGCCCGCU | AGCGGGCUC CGGGGCUC |
| 62 | CUGCACCC CUGAUGA X GAA AGCCCCGG | CCGGGGCUC GGGUGCAG |
| 97 | CCCCGGGU CUGAUGA X GAA AUCCUCGC | GCGAGGAUU ACCCGGGG |
| 98 | UCCCCGGG CUGAUGA X GAA AAUCCUCG | CGAGGAUUA CCCGGGGA |
| 113 | CAGGAGAC CUGAUGA X GAA ACCACUUC | GAAGUGGUU GUCUCCUG |
| 116 | AGCCAGGA CUGAUGA X GAA ACAACCAC | GUGGUUGUC UCCUGGCU |
| 118 | CCAGCCAG CUGAUGA X GAA AGACAACC | GGUUGUCUC CUGGCUGG |
| 145 | CGCGCCCU CUGAUGA X GAA AGCGCCCG | CGGGCGCUC AGGGCGCG |
| 185 | GGCCGCCA CUGAUGA X GAA AGUCCGUC | GACGGACUC UGGCGGCC |
| 198 | CGGCCAAC CUGAUGA X GAA ACCCGGCC | GGCCGGGUC GUUGGCCG |
| 201 | CCCCGGCC CUGAUGA X GAA ACGACCCG | CGGGUCGUU GGCCGGGG |
| 240 | GUGAGCGC CUGAUGA X GAA ACGCGGCC | GGCCGCGUC GCGCUCAC |
| 246 | ACCAUGGU CUGAUGA X GAA AGCGCGAC | GUCGCGCUC ACCAUGGU |
| 255 | CAGUAGCU CUGAUGA X GAA ACCAUGGU | ACCAUGGUC AGCUACUG |
| 260 | UGUCCCAG CUGAUGA X GAA AGCUGACC | GGUCAGCUA CUGGGACA |
| 276 | CACAGCAG CUGAUGA X GAA ACCCCGGU | ACCGGGGUC CUGCUGUG |
| 294 | AGACAGCU CUGAUGA X GAA AGCAGCGC | GCGCUGCUC AGCUGUCU |
| 301 | GAGAAGCA CUGAUGA X GAA ACAGCUGA | UCAGCUGUC UGCUUCUC |
| 306 | CCUGUGAG CUGAUGA X GAA AGCAGACA | UGUCUGCUU CUCACAGG |
| 307 | UCCUGUGA CUGAUGA X GAA AAGCAGAC | GUCUGCUUC UCACAGGA |
| 309 | GAUCCUGU CUGAUGA X GAA AGAAGCAG | CUGCUUCUC ACAGGAUC |
| 317 | CUGAACUA CUGAUGA X GAA AUCCUGUG | CACAGGAUC UAGUUCAG |
| 319 | ACCUGAAC CUGAUGA X GAA AGAUCCUG | CAGGAUCUA GUUCAGGU |
| 322 | UGAACCUG CUGAC1GA X GAA ACUAGAUC | GAUCUAGUU CAGGUUCA |
| 323 | UUGAACCU CUGAUGA X GAA AACUAGAU | AUCUAGUUC AGGUUCAA |
| 328 | UAAUUUUG CUGAUGA X GAA ACCUGAAC | GUUCAGGUU CAAAAUUA |
| 329 | UUAAUUUU CUGAUGA X GAA AACCUGAA | UUCAGGUUC AAAAUUAA |
| 335 | GAUCUUUU CUGAUGA X GAA AUUUUGAA | UUCAAAAUU AAAAGAUC |
| 336 | GGAUCUUU CUGAUGA X GAA AAUUUUGA | UCAAAAUUA AAAGAUCC |
| 343 | CAGUUCAG CUGAUGA X GAA AUCUUUUA | UAAAAGAUC CUGAACUG |
| 355 | GCCUUUUA CUGAUGA X GAA ACUCAGUU | AACUGAGUU UAAAAGGC |
| 356 | UGCCUUUU CUGAUGA X GAA AACUCAGU | ACUGAGUUU AAAAGGCA |
| 357 | GUGCCUUU CUGAUGA X GAA AAACUCAG | CUGAGUUUA AAAGGCAC |
| 375 | GCUUGCAU CUGAUGA X GAA AUGUGCUG | CAGCACAUC AUGCAAGC |
| 400 | GCAUUGGA CUGAUGA X GAA AUGCAGUG | CACUGCAUC UCCAAUGC |
| 402 | CUGCAUUG CUGAUGA X GAA AGAUGCAG | CUGCAUCUC CAAUGCAG |
| 427 | AGACCAUU CUGAUGA X GAA AUGGGCUG | CAGCCCAUA AAUGGUCU |
| 434 | CAGGCAAA CUGAUGA X GAA ACCAUUUA | UAAAUGGUC UUUGCCUG |
| 436 | UUCAGGCA CUGAUGA X GAA AGACCAUU | AAUGGUCUU UGCCUGAA |
| 437 | UUUCAGGC CUGAUGA X GAA AAGACCAU | AUGGUCUUU GCCUGAAA |
| 454 | GCUUUCCU CUGAUGA X GAA ACUCACCA | UGGUGAGUA AGGAAAGC |
| 477 | GAUUUAGU CUGAUGA X GAA AUGCUCAG | CUGAGCAUA ACUAAAUC |
| 481 | GGCAGAUU CUGAUGA X GAA AGUUAUGC | GCAUAACUA AAUCUGCC |
| 485 | CACAGGCA CUGAUGA X GAA AUUUAGUU | AACUAAAUC UGCCUGUG |
| 512 | UACUGCAG CUGAUGA X GAA AUUGUUUG | CAAACAAUU CUGCAGUA |
| 513 | GUACUGCA CUGAUGA X GAA AAUUGUUU | AAACAAUUC UGCAGUAC |
| 520 | GGUUAAAG CUGAUGA X GAA ACUGCAGA | UCUGCAGUA CUUUAACC |
| 523 | CAAGGUUA CUGAUGA X GAA AGUACUGC | GCAGUACUU UAACCUUG |
| 524 | UCAAGGUU CUGAUGA X GAA AAGUACUG | CAGUACUUU AACCUUGA |

(continued)

| nt. Position | HH Ribozyme | Substrate |
|---|---|---|
| 525 | UUCAAGGU CUGAUGA X GAA AAAGUACU | AGUACUUUA ACCUUGAA |
| 530 | CUGUGUUC CUGAUGA X GAA AGGUUAAA | UUUAACCUU GAACACAG |
| 541 | GUUUGCUU CUGAUGA X GAA AGCUGUGU | ACACAGCUC AAGCAAAC |
| 560 | AGCUGUAG CUGAUGA X GAA AGCCAGUG | CACUGGCUU CUACAGCU |
| 561 | CAGCUGUA CUGAUGA X GAA AAGCCAGU | ACUGGCUUC UACAGCUG |
| 563 | UGCAGCUG CUGAUGA X GAA AGAAGCCA | UGGCUUCUA CAGCUGCA |
| 575 | CAGCUAGA CUGAUGA X GAA AUUUGCAG | CUGCAAAUA UCUAGCUG |
| 577 | UACAGCUA CUGAUGA X GAA AUAUUUGC | GCAAAUAUC UAGCUGUA |
| 579 | GGUACAGC CUGAUGA X GAA AGAUAUUU | AAAUAUCUA GCUGUACC |
| 585 | GAAGUAGG CUGAUGA X GAA ACAGCUAG | CUAGCUGUA CCUACUUC |
| 589 | CUUUGAAG CUGAUGA X GAA AGGUACAG | CUGUACCUA CUUCAAAG |
| 592 | CUUCUUUG CUGAUGA X GAA AGUAGGUA | UACCUACUU CAAAGAAG |
| 593 | UCUUCUUU CUGAUGA X GAA AAGUAGGU | ACCUACUUC AAAGAAGA |
| 614 | AGAUUGCA CUGAUGA X GAA AUUCUGUU | AACAGAAUC UGCAAUCU |
| 621 | AAUAUAUA CUGAUGA X GAA AUUGCAGA | UCUGCAAUC UAUAUAUU |
| 623 | UAAAUAUA CUGAUGA X GAA AGAUUGCA | UGCAAUCUA UAUAUUUA |
| 625 | AAUAAAUA CUGAUGA X GAA AUAGAUUG | CAAUCUAUA UAUUUAUU |
| 627 | CUAAUAAA CUGAUGA X GAA AUAUAGAU | AUCUAUAUA UUUAUUAG |
| 629 | CACUAAUA CUGAUGA X GAA AUAUAUAG | CUAUAUAUU UAUUAGUG |
| 630 | UCACUAAU CUGAUGA X GAA AAUAUAUA | UAUAUAUUU AUUAGUGA |
| 631 | AUCACUAA CUGAUGA X GAA AAAUAUAU | AUAUAUUUA UUAGUGAU |
| 633 | GUAUCACU CUGAUGA X GAA AUAAAUAU | AUAUUUAUU AGUGAUAC |
| 634 | UGUAUCAC CUGAUGA X GAA AAUAAAUA | UAUUUAUUA GUGAUACA |
| 640 | UCUACCUG CUGAUGA X GAA AUCACUAA | UUAGUGAUA CAGGUAGA |
| 646 | GAAAGGUC CUGAUGA X GAA ACCUGUAU | AUACAGGUA GACCUUUC |
| 652 | CUCUACGA CUGAUGA X GAA AGGUCUAC | GUAGACCUU UCGUAGAG |
| 653 | UCUCUACG CUGAUGA X GAA AAGGUCUA | UAGACCUUU CGUAGAGA |
| 654 | AUCUCUAC CUGAUGA X GAA AAAGGUCU | AGACCUUUC GUAGAGAU |
| 657 | UACAUCUC CUGAUGA X GAA ACGAAAGG | CCUUUCGUA GAGAUGUA |
| 665 | UUUCACUG CUGAUGA X GAA ACAUCUCU | AGAGAUGUA CAGUGARA |
| 675 | AUUUCGGG CUGAUGA X GAA AUUUCACU | AGUGAAAUC CCCGAAAU |
| 684 | AUGUGUAU CUGAUGA X GAA AUUUCGGG | CCCGAAAUU AUACACAU |
| 685 | CAUGUGUA CUGAUGA X GAA AAUUUCGG | CCGAAAUUA UACACAUG |
| 687 | GUCAUGUG CUGAUGA X GAA AUAAUUUC | GAAAUUAUA CACAUGAC |
| 711 | GGAAUGAC CUGAUGA X GAA AGCUCCCU | AGGGAGCUC GUCAUUCC |
| 714 | CAGGGAAU CUGAUGA X GAA ACGAGCUC | GAGCUCGUC AUUCCCUG |
| 717 | CGGCAGGG CUGAUGA X GAA AUGACGAG | CUCGUCAUU CCCUGCCG |
| 718 | CCGGCAGG CUGAUGA X GAA AAUGACGA | UCGUCAUUC CCUGCCGG |
| 729 | GGUGACGU CUGAUGA X GAA ACCCGGCA | UGCCGGGUU ACGUCACC |
| 730 | AGGUGACG CUGAUGA X GAA AACCCGGC | GCCGGGUUA CGUCACCU |
| 734 | UGUUAGGU CUGAUGA X GAA ACGUAACC | GGUUACGUC ACCUAACA |
| 739 | AGUGAUGU CUGAUGA X GAA AGGUGACG | CGUCACCUA ACAUCACU |
| 744 | GUAACAGU CUGAUGA X GAA AUGUUAGG | CCUAACAUC ACUGUUAC |
| 750 | UUUAAAGU CUGAUGA X GAA ACAGUGAU | AUCACUGUU ACUUUAAA |
| 751 | UUUUAAAG CUGAUGA X GAA AACAGUGA | UCACUGUUA CUUUAAAA |
| 754 | CUUUUUUA CUGAUGA X GAA AGUAACAG | CUGUUACUU UAAAAAAG |
| 755 | ACUUUUUU CUGAUGA X GAA AAGUAACA | UGUUACUUU AAAAAAGU |
| 756 | AACUUUUU CUGAUGA X GAA AAAGUAAC | GUUACUUUA AAAAAGUU |
| 764 | CAAGUGGA CUGAUGA X GAA ACUUUUUU | AAAAAAGUU UCCACUUG |
| 765 | UCAAGUGG CUGAUGA X GAA AACUUUUU | AAAAAGUUU CCACUUGA |

(continued)

| nt. Position | HH Ribozyme | Substrate |
|---|---|---|
| 766 | GUCAAGUG CUGAUGA X GAA AAACUUUU | AAAAGUUUC CACUUGAC |
| 771 | AAAGUGUC CUGAUGA X GAA AGUGGAAA | UUUCCACUU GACACUUU |
| 778 | AGGGAUCA CUGAUGA X GAA AGUGUCAA | UUGACACUU UGAUCCCU |
| 779 | CAGGGAUC CUGAUGA X GAA AAGUGUCA | UGACACUUU GAUCCCUG |
| 783 | CCAUCAGG CUGAUGA X GAA AUCAAAGU | ACUUUGAUC CCUGAUGG |
| 801 | UCCCAGAU CUGAUGA X GAA AUGCGUUU | AAACGCAUA AUCUGGGA |
| 804 | CUGUCCCA CUGAUGA X GAA AUUAUGCG | CGCAUAAUC UGGGACAG |
| 814 | GCCCUUUC CUGAUGA X GAA ACUGUCCC | GGGACAGUA GAAAGGGC |
| 824 | AUAUGAUG CUGAUGA X GAA AGCCCUUU | AAAGGGCUU CAUCAUAU |
| 825 | GAUAUGAU CUGAUGA X GAA AAGCCCUU | AAGGGCUUC AUCAUAUC |
| 828 | UUUGAUAU CUGAUGA X GAA AUGAAGCC | GGCUUCAUC AUAUCAAA |
| 831 | GCAUUUGA CUGAUGA X GAA AUGAUGAA | UUCAUCAUA UCAAAUGC |
| 833 | UUGCAUUU CUGAUGA X GAA AUAUGAUG | CAUCAUAUC AAAUGCAA |
| 845 | UUUCUUUG CUGAUGA X GAA ACGUUGCA | UGCAACGUA CAAAGAAA |
| 855 | AGAAGCCC CUGAUGA X GAA AUUUCUUU | AAAGAAAUA GGGCUUCU |
| 861 | CAGGUCAG CUGAUGA X GAA AGCCCUAU | AUAGGGCUU CUGACCUG |
| 862 | ACAGGUCA CUGAUGA X GAA AAGCCCUA | UAGGGCUUC UGACCUGU |
| 882 | UGCCCAUU CUGAUGA X GAA ACUGUUGC | GCAACAGUC AAUGGGCA |
| 892 | CUUAUACA CUGAUGA X GAA AUGCCCAU | AUGGGCAW UGUAUAAG |
| 893 | UCUUAUAC CUGAUGA X GAA AAUGCCCA | UGGGCAUUU GUAUAAGA |
| 896 | UUGUCUUA CUGAUGA X GAA ACAAAUGC | GCAUUUGUA UAAGACAA |
| 898 | GUUUGUCU CUGAUGA X GAA AUACAAAU | AUUUGUAUA AGACAAAC |
| 908 | GUGUGAGA CUGAUGA X GAA AGUUUGUC | GACAAACUA UCUCACAC |
| 910 | AUGUGUGA CUGAUGA X GAA AUAGUUUG | CAAACUAUC UCACACAU |
| 912 | CGAUGUGU CUGAUGA X GAA AGAUAGUU | AACUAUCUC ACACAUCG |
| 919 | GGUUUGUC CUGAUGA X GAA AUGUGUGA | UCACACAUC GACAAACC |
| 931 | UAUGAUUG CUGAUGA X GAA AUUGGUUU | AAACCAAUA CAAUCAUA |
| 936 | ACAUCUAU CUGAUGA X GAA AUUGUAUU | AAUACAAUC AUAGAUGU |
| 939 | UGGACAUC CUGAUGA X GAA AUGAUUGU | ACAAUCAUA GAUGUCCA |
| 945 | CUUAUUUG CUGAUGA X GAA ACAUCUAU | AUAGAUGUC CAAAUAAG |
| 951 | GGUGUGCU CUGAUGA X GAA AUUUGGAC | GUCCAAAUA AGCACACC |
| 969 | AGUAAUUU CUGAUGA X GAA ACUGGGCG | CGCCCAGUC AAAUUACU |
| 974 | CUCUAAGU CUGAUGA X GAA AUUUGACU | AGUCAAAUU ACUUAGAG |
| 975 | CCUCUAAG CUGAUGA X GAA AAUUUGAC | GUCAAAUUA CUUAGAGG |
| 978 | UGGCCUCU CUGAUGA X GAA AGUAAUUU | AAAUUACUU AGAGGCCA |
| 979 | AUGGCCUC CUGAUGA X GAA AAGUAAUU | AAUUACUUA GAGGCCAU |
| 988 | GACAAGAG CUGAUGA X GAA AUGGCCUC | GAGGCCAUA CUCUUGUC |
| 991 | GAGGACAA CUGAUGA X GAA AGUAUGGC | GCCAUACUC UUGUCCUC |
| 993 | UUGAGGAC CUGAUGA X GAA AGAGUAUG | CAUACUCUU GUCCUCAA |
| 996 | CAAUUGAG CUGAUGA X GAA ACAAGAGU | ACUCUUGUC CUCAAUUG |
| 999 | GUACAAUU CUGAUGA X GAA AGGACAAG | CUUGUCCUC AAUUGUAC |
| 1003 | AGCAGUAC CUGAUGA X GAA AUUGAGGA | UCCUCAAUU GUACUGCU |
| 1006 | GGUAGCAG CUGAUGA X GAA ACAAUUGA | UCAAUUGUA CUGCUACC |
| 1012 | GGGAGUGG CUGAUGA X GAA AGCAGUAC | GUACUGCUA CCACUCCC |
| 1018 | GUUCAAGG CUGAUGA X GAA AGUGGUAG | CUACCACUC CCUUGAAC |
| 1022 | UCGUGUUC CUGAUGA X GAA AGGGAGUG | CACUCCCUU GAACACGA |
| 1035 | GUCAUUUG CUGAUGA X GAA ACUCUCGU | ACGAGAGUU CAAAUGAC |
| 1036 | GGUCAUUU CUGAUGA X GAA AACUCUCG | CGAGAGUUC AAAUGACC |
| 1051 | AUCAGGGU CUGAUGA X GAA ACUCCAGG | CCUGGAGUU ACCCUGAU |
| 1052 | CAUCAGGG CUGAUGA X GAA AACUCCAG | CUGGAGUUA CCCUGAUG |

(continued)

| nt. Position | HH Ribozyme | Substrate |
|---|---|---|
| 1069 | AGCUCUCU CUGAUGA X GAA AUUUUUUU | AAAAAAAUA AGAGAGCU |
| 1078 | CCUUACGG CUGAUGA X GAA AGCUCUCU | AGAGAGCUU CCGUAAGG |
| 1079 | GCCUUACG CUGAUGA X GAA AAGCUCUC | GAGAGCUUC CGUAAGGC |
| 1083 | CGUCGCCU CUGAUGA X GAA ACGGAAGC | GCUUCCGUA AGGCGACG |
| 1095 | CUUUGGUC CUGAUGA X GAA AUUCGUCG | CGACGAAUU GACCAAAG |
| 1108 | GGCAUGGG CUGAUGA X GAA AUUGCUUU | AAAGCAAUU CCCAUGCC |
| 1109 | UGGCAUGG CUGAUGA X GAA AAUUGCUU | AAGCAAUUC CCAUGCCA |
| 1122 | CUGUAGAA CUGAUGA X GAA AUGUUGGC | GCCAACAUA UUCUACAG |
| 1124 | CACUGUAG CUGAUGA X GAA AUAUGUUG | CAACAUAUU CUACAGUG |
| 1125 | ACACUGUA CUGAUGA X GAA AAUAUGUU | AACAUAUUC UACAGUGU |
| 1127 | GAACACUG CUGAUGA X GAA AGAAUAUG | CAUAUUCUA CAGUGUUC |
| 1134 | AUAGUAAG CUGAUGA X GAA ACACUGUA | UACAGUGUU CUUACUAU |
| 1135 | AAUAGUAA CUGAUGA X GAA AACACUGU | ACAGUGUUC UUACUAUU |
| 1137 | UCAAUAGU CUGAUGA X GAA AGAACACU | AGUGUUCUU ACUAUUGA |
| 1138 | GUCAAUAG CUGAUGA X GAA AAGAACAC | GUGUUCUUA CUAUUGAC |
| 1141 | UUUGUCAA CUGAUGA X GAA AGUAAGAA | UUCUUACUA UUGACAAA |
| 1143 | AUUUUGUC CUGAUGA X GAA AUAGUAAG | CUUACUAUU GACAAAAU |
| 1173 | CAAGUAUA CUGAUGA X GAA AGUCCUUU | AAAGGACUU UAUACUUG |
| 1174 | ACAAGUAU CUGAUGA X GAA AAGUCCUU | AAGGACUUU AUACUUGU |
| 1175 | GACAAGUA CUGAUGA X GAA AAAGUCCU | AGGACUUUA UACUUGUC |
| 1177 | ACGACAAG CUGAUGA X GAA AUAAAGUC | GACUUUAUA CUUGUCGU |
| 1180 | UACACGAC CUGAUGA X GAA AGUAUAAA | UUUAUACUU GUCGUGUA |
| 1183 | CCUUACAC CUGAUGA X GAA ACAAGUAU | AUACUUGUC GUGUAAGG |
| 1188 | CCACUCCU CUGAUGA X GAA ACACGACA | UGUCGUGUA AGGAGUGG |
| 1202 | AUUUGAAU CUGAUGA X GAA AUGGUCCA | UGGACCAUC AUUCAAAU |
| 1205 | CAGAUUUG CUGAUGA X GAA AUGAUGGU | ACCAUCAUU CAAAUCUG |
| 1206 | ACAGAUUU CUGAUGA X GAA AAUGAUGG | CCAUCAUUC AAAUCUGU |
| 1211 | UGUUAACA CUGAUGA X GAA AUUUGAAU | AUUCAAAUC UGUUAACA |
| 1215 | GAGGUGUU CUGAUGA X GAA ACAGAUUU | AAAUCUGUU AACACCUC |
| 1216 | UGAGGUGU CUGAUGA X GAA AACAGAUU | AAUCUGUUA ACACCUCA |
| 1223 | UAUGCACU CUGAUGA X GAA AGGUGUUA | UAACACCUC AGUGCAUA |
| 1231 | AUCAUAUA CUGAUGA X GAA AUGCACUG | CAGUGCAUA UAUAUGAU |
| 1233 | UUAUCAUA CUGAUGA X GAA AUAUGCAC | GUGCAUAUA UAUGAUAA |
| 1235 | CUUUAUCA CUGAUGA X GAA AUAUAUGC | GCAUAUAUA UGAUAAAG |
| 1240 | GAAUGCUU CUGAUGA X GAA AUCAUAUA | UAUAUGAUA AAGCAUUC |
| 1247 | CAGUGAUG CUGAUGA X GAA AUGCUUUA | UAAAGCAUU CAUCACUG |
| 1248 | ACAGUGAU CUGAUGA X GAA AAUGCUUU | AAAGCAUUC AUCACUGU |
| 1251 | UUCACAGU CUGAUGA X GAA AUGAAUGC | GCAUUCAUC ACUGUGAA |
| 1264 | CUGUUUUC CUGAUGA X GAA AUGUUUCA | UGAAACAUC GAAAACAG |
| 1281 | ACGGUUUC CUGAUGA X GAA AGCACCUG | CAGGUGCUU GAAACCGU |
| 1290 | UUGCCAGC CUGAUGA X GAA ACGGUUUC | GAAACCGUA GCUGGCAA |
| 1304 | GCCGGUAA CUGAUGA X GAA ACCGCUUG | CAAGCGGUC UUACCGGC |
| 1306 | GAGCCGGU CUGAUGA X GAA AGACCGCU | AGCGGUCUU ACCGGCUC |
| 1307 | AGAGCCGG CUGAUGA X GAA AAGACCGC | GCGGUCUUA CCGGCUCU |
| 1314 | UUCAUAGA CUGAUGA X GAA AGCCGGUA | UACCGGCUC UCUAUGAA |
| 1316 | CUUUCAUA CUGAUGA X GAA AGAGCCGG | CCGGCUCUC UAUGAAAG |
| 1318 | CACUUUCA CUGAUGA X GAA AGAGAGCC | GGCUCUCUA UGAAAGUG |
| 1334 | GCGAGGGA CUGAUGA X GAA AUGCCUUC | GAAGGCAUU UCCCUCGC |
| 1335 | GGCGAGGG CUGAUGA X GAA AAUGCCUU | AAGGCAUUU CCCUCGCC |
| 1336 | CGGCGAGG CUGAUGA X GAA AAAUGCCU | AGGCAUUUC CCUCGCCG |

(continued)

| nt. Position | HH Ribozyme | Substrate |
|---|---|---|
| 1340 | CUUCCGGC CUGAUGA X GAA AGGGAAAU | AUUUCCCUC GCCGGAAG |
| 1350 | AACCAUAC CUGAUGA X GAA ACWCCGG | CCGGAAGUU GUAUGGUU |
| 1353 | UUUAACCA CUGAUGA X GAA ACAACUUC | GAAGUUGUA UGGUUAAA |
| 1358 | CAUCUUUU CUGAUGA X GAA ACCAUACA | UGUAUGGUU AAAAGAUG |
| 1359 | CCAUCUUU CUGAUGA X GAA AACCAUAC | GUAUGGUUA AAAGAUGG |
| 1370 | UCGCAGGU CUGAUGA X GAA ACCCAUCU | AGAUGGGUU ACCUGCGA |
| 1371 | GUCGCAGG CUGAUGA X GAA AACCCAUC | GAUGGGUUA CCUGCGAC |
| 1388 | AGCGAGCA CUGAUGA X GAA AUUUCUCA | UGAGAAAUC UGCUCGCU |
| 1393 | CAAAUAGC CUGAUGA X GAA AGCAGAUU | AAUCUGCUC GCUAUUUG |
| 1397 | GAGUCAAA CUGAUGA X GAA AGCGAGCA | UGCUCGCUA UUUGACUC |
| 1399 | ACGAGUCA CUGAUGA X GAA AUAGCGAG | CUCGCUAUU UGACUCGU |
| 1400 | CACGAGUC CUGAUGA X GAA AAUAGCGA | UCGCUAUUU GACUCGUG |
| 1405 | GUAGCCAC CUGAUGA X GAA AGUCAAAU | AUUUGACUC GUGGCUAC |
| 1412 | UUAACGAG CUGAUGA X GAA AGCCACGA | UCGUGGCUA CUCGUUAA |
| 1415 | UAAUUAAC CUGAUGA X GAA AGUAGCCA | UGGCUACUC GUUAAUUA |
| 1418 | UGAUAAUU CUGAUGA X GAA ACGAGUAG | CUACUCGUU AAUUAUCA |
| 1419 | UUGAUAAU CUGAUGA X GAA AACGAGUA | UACUCGUUA AUUAUCAA |
| 1422 | UCCUUGAU CUGAUGA X GAA AUUAACGA | UCGUUAAUU AUCAAGGA |
| 1423 | GUCCUUGA CUGAUGA X GAA AAUUAACG | CGUUAAUUA UCAAGGAC |
| 1425 | ACGUCCUU CUGAUGA X GAA AUAAUUAA | UUAAUUAUC AAGGACGU |
| 1434 | UCUUCAGU CUGAUGA X GAA ACGUCCUU | AAGGACGUA ACUGAAGA |
| 1456 | GAUUGUAU CUGAUGA X GAA AUUCCCUG | CAGGGAAUU AUACAAUC |
| 1457 | AGAUUGUA CUGAUGA X GAA AAUUCCCU | AGGGAAUUA UACAAUCU |
| 1459 | CAAGAUUG CUGAUGA X GAA AUAAUUCC | GGAAUUAUA CAAUCUUG |
| 1464 | CUCAGCAA CUGAUGA X GAA AUUGUAUA, | UAUACAAUC UUGCUGAG |
| 1466 | UGCUCAGC CUGAUGA X GAA AGAUUGUA | UACAAUCUU GCUGAGCA |
| 1476 | GACUGUUU CUGAUGA X GAA AUGCUCAG | CUGAGCAUA AAACAGUC |
| 1484 | ACACAUUU CUGAUGA X GAA ACUGUUUU | AAAACAGUC AAAUGUGU |
| 1493 | GGUUUUUA CUGAUGA X GAA ACACAUUU | AAAUGUGUU UAAAAACC |
| 1494 | AGGUUUUU CUGAUGA X GAA AACACAUU | AAUGUGUUU AAAAACCU |
| 1495 | GAGGUUUU CUGAUGA X GAA AAACACAU | AUGUGUUUA AAAACCUC |
| 1503 | GUGGCAGU CUGAUGA X GAA AGGUUUUU | AAAAACCUC ACUGCCAC |
| 1513 | GACAAUUA CUGAUGA X GAA AGUGGCAG | CUGCCACUC UAAUUGUC |
| 1515 | UUGACAAU CUGAUGA X GAA AGAGUGGC | GCCACUCUA AUUGUCAA |
| 1518 | ACAUUGAC CUGAUGA X GAA AUUAGAGU | ACUCUAAUU GUCAAUGU |
| 1521 | UUCACAUU CUGAUGA X GAA ACAAUUAG | CUAAUUGUC AAUGUGAA |
| 1539 | UUUUCGUA CUGAUGA X GAA AUCUGGGG | CCCCAGAUU UACGAAAA |
| 1540 | CUUUUCGU CUGAUGA X GAA AAUCUGGG | CCCAGAUUU ACGAAAAG |
| 1541 | CCUUUUCG CUGAUGA X GAA AAAUCUGG | CCAGAUUUA CGAAAAGG |
| 1556 | GAAACGAU CUGAUGA X GAA ACACGGCC | GGCCGUGUC AUCGUUUC |
| 1559 | CUGGAAAC CUGAUGA X GAA AUGACACG | CGUGUCAUC GUUUCCAG |
| 1562 | GGUCUGGA CUGAUGA X GAA ACGAUGAC | GUCAUCGUU UCCAGACC |
| 1563 | GGGUCUGG CUGAUGA X GAA AACGAUGA | UCAUCGUUU CCAGACCC |
| 1564 | CGGGUCUG CUGAUGA X GAA AAACGAUG | CAUCGUUUC CAGACCCG |
| 1576 | UGGGUAGA CUGAUGA X GAA AGCCGGGU | ACCCGGCUC UCUACCCA |
| 1578 | AGUGGGUA CUGAUGA X GAA AGAGCCGG | CCGGCUCUC UACCCACU |
| 1580 | CCAGUGGG CUGAUGA X GAA AGAGAGCC | GGCUCUCUA CCCACUGG |
| 1602 | CAAGUCAG CUGAUGA X GAA AUUUGUCU | AGACAAAUC CUGACUUG |
| 1609 | UGCGGUAC CUGAUGA X GAA AGUCAGGA | UCCUGACUU GUACCGCA |
| 1612 | AUAUGCGG CUGAUGA X GAA ACAAGUCA | UGACUUGUA CCGCAUAU |

(continued)

| nt. Position | HH Ribozyme | Substrate |
|---|---|---|
| 1619 | GGAUACCA CUGAUGA X GAA AUGCGGUA | UACCGCAUA UGGUAUCC |
| 1624 | UUGAGGGA CUGAUGA X GAA ACCAUAUG | CAUAUGGUA UCCCUCAA |
| 1626 | GGUUGAGG CUGAUGA X GAA AUACCAUA | UAUGGUAUC CCUCAACC |
| 1630 | UGUAGGUU CUGAUGA X GAA AGGGAUAC | GUAUCCCUC AACCUACA |
| 1636 | CUUGAUUG CUGAUGA X GAA AGGUUGAG | CUCAACCUA CAAUCAAG |
| 1641 | AACCACUU CUGAUGA X GAA AUUGUAGG | CCUACAAUC AAGUGGUU |
| 1649 | GGUGCCAG CUGAUGA X GAA ACCACUUG | CAAGUGGUU CUGGCACC |
| 1650 | GGGUGCCA CUGAUGA X GAA AACCACUU | AAGUGGUUC UGGCACCC |
| 1663 | AUUAUGGU CUGAUGA X GAA ACAGGGGU | ACCCCUGUA ACCAUAAU |
| 1669 | GGAAUGAU CUGAUGA X GAA AUGGUUAC | GUAACCAUA AUCAUUCC |
| 1672 | UUCGGAAU CUGAUGA X GAA AUUAUGGU | ACCAUAAUC AUUCCGAA |
| 1675 | UGCUUCGG CUGAUGA X GAA AUGAUUAU | AUAAUCAUU CCGAAGCA |
| 1676 | UUGCUUCG CUGAUGA X GAA AAUGAUUA | UAAUCAUUC CGAAGCAA |
| 1694 | UGGAACAA CUGAUGA X GAA AGUCACAC | GUGUGACUU UUGUUCCA |
| 1695 | UUGGAACA CUGAUGA X GAA AAGUCACA | UGUGACUUU UGUUCCAA |
| 1696 | AUUGGAAC CUGAUGA X GAA AAAGUCAC | GUGACUUUU GUUCCAAU |
| 1699 | AUUAUUGG CUGAUGA X GAA ACAAAAGU | ACUUUUGUU CCAAUAAU |
| 1700 | CAUUAUUG CUGAUGA X GAA AACAAAAG | CUUUUGUUC CAAUAAUG |
| 1705 | CUCUUCAU CUGAUGA X GAA AUUGGAAC | GUUCCAAUA AUGAAGAG |
| 1715 | GGAUAAAG CUGAUGA X GAA ACUCUUCA | UGAAGAGUC CUUUAUCC |
| 1718 | CCAGGAUA CUGAUGA X GAA AGGACUCU | AGAGUCCUU UAUCCUGG |
| 1719 | UCCAGGAU CUGAUGA X GAA AAGGACUC | GAGUCCUUU AUCCUGGA |
| 1720 | AUCCAGGA CUGAUGA X GAA AAAGGACU | AGUCCUUUA UCCUGGAU |
| 1722 | GCAUCCAG CUGAUGA X GAA AUAAAGGA | UCCUUUAUC CUGGAUGC |
| 1755 | AUGCUCUC CUGAUGA X GAA AUUCUGUU | AACAGAAUU GAGAGCAU |
| 1764 | CGCUGAGU CUGAUGA X GAA AUGCUCUC | GAGAGCAUC ACUCAGCG |
| 1768 | CAUGCGCU CUGAUGA X GAA AGUGAUGC | GCAUCACUC AGCGCAUG |
| 1782 | CCUUCUAU CUGAUGA X GAA AUUGCCAU | AUGGCAAUA AUAGAAGG |
| 1785 | UUUCCUUC CUGAUGA X GAA AUUAUUGC | GCAAUAAUA GAAGGAAA |
| 1798 | AGCCAUCU CUGAUGA X GAA AUUCUUUC | GAAAGAAUA AGAUGGCU |
| 1807 | CAAGGUGC CUGAUGA X GAA AGCCAUCU | AGAUGGCUA GCACCUUG |
| 1814 | CCACAACC CUGAUGA X GAA AGGUGCUA | UAGCACCUU GGUUGUGG |
| 1818 | UCAGCCAC CUGAUGA X GAA ACCAAGGU | ACCUUGGUU GUGGCUGA |
| 1829 | AAAUUCUA CUGAUGA X GAA AGUCAGCC | GGCUGACUC UAGAAUUU |
| 1831 | AGAAAUUC CUGAUGA X GAA AGAGUCAG | CUGACUCUA GAAUUUCU |
| 1836 | AUUCCAGA CUGAUGA X GAA AUUCUAGA | UCUAGAAUU UCUGGAAU |
| 1837 | GAUUCCAG CUGAUGA X GAA AAUUCUAG | CUAGAAUUU CUGGAAUC |
| 1838 | AGAUUCCA CUGAUGA X GAA AAAUUCUA | UAGAAUUUC UGGAAUCU |
| 1845 | CAAAUGUA CUGAUGA X GAA AUUCCAGA | UCUGGAAUC UACAUUUG |
| 1847 | UGCAAAUG CUGAUGA X GAA AGAUUCCA | UGGAAUCUA CAUUUGCA |
| 1851 | GCUAUGCA CUGAUGA X GAA AUGUAGAU | AUCUACAUU UGCAUAGC |
| 1852 | AGCUAUGC CUGAUGA X GAA AAUGUAGA | UCUACAUUU GCAUAGCU |
| 1857 | UUGGAAGC CUGAUGA X GAA AUGCAAAU | AUUUGCAUA GCUUCCAA |
| 1861 | UUUAUUGG CUGAUGA X GAA AGCUAUGC | GCAUAGCUU CCAAUAAA |
| 1862 | CUUUAUUG CUGAUGA X GAA AAGCUAUG | CAUAGCUUC CAAUAAAG |
| 1867 | CCCAACUU CUGAUGA X GAA AUUGGAAG | CUUCCAAUA AAGUUGGG |
| 1872 | ACAGUCCC CUGAUGA X GAA ACUUUAUU | AAUAAAGUU GGGACUGU |
| 1893 | UAAAAGCU CUGAUGA X GAA AUGUUUCU | AGAAACAUA AGCUUUUA |
| 1898 | UGAUAUAA CUGAUGA X GAA AGCUUAUG | CAUAAGCUU UUAUAUCA |
| 1899 | GUGAUAUA CUGAUGA X GAA AAGCUUAU | AUAAGCUUU UAUAUCAC |

(continued)

| nt. Position | HH Ribozyme | Substrate |
|---|---|---|
| 1900 | UGUGAUAU CUGAUGA X GAA AAAGCUUA | UAAGCUUUU AUAUCACA |
| 1901 | CUGUGAUA CUGAUGA X GAA AAAAGCUU | AAGCUUUUA UAUCACAG |
| 1903 | AUCUGUGA CUGAUGA X GAA AUAAAAGC | GCUUUUAUA UCACAGAU |
| 1905 | ACAUCUGU CUGAUGA X GAA AUAUAAAA | UUUUAUAUC ACAGAUGU |
| 1925 | UAACAUGA CUGAUGA X GAA ACCCAUUU | AAAUGGGUU UCAUGUUA |
| 1926 | UUAACAUG CUGAUGA X GAA AACCCAUU | AAUGGGUUU CAUGUUAA |
| 1927 | GWAACAU CUGAUGA X GAA AAACCCAU | AUGGGUUUC AUGWAAC |
| 1932 | UCCAAGUU CUGAUGA X GAA ACAUGAAA | UUUCAUGUU AACUUGGA |
| 1933 | UUCCAAGU CUGAUGA X GAA AACAUGAA | UUCAUGUUA ACUUGGAA |
| 1937 | UUUUUUCC CUGAUGA X GAA AGUUAACA | UGUUAACUU GGAAAAAA |
| 1976 | CUGUGCAA CUGAUGA X GAA ACAGUUUC | GAAACUGUC UUGCACAG |
| 1978 | AACUGUGC CUGAUGA X GAA AGACAGUU | AACUGUCUU GCACAGUU |
| 1986 | AACUUGUU CUGAUGA X GAA ACUGUGCA | UGCACAGUU AACAAGUU |
| 1987 | GAACUUGU CUGAUGA X GAA AACUGUGC | GCACAGUUA ACAAGUUC |
| 1994 | UGUAUAAG CUGAUGA X GAA ACUUGUUA | UAACAAGUU CUUAUACA |
| 1995 | CUGUAUAA CUGAUGA X GAA AACUUGUU | AACAAGWC WAUACAG |
| 1997 | CUCUGUAU CUGAUGA X GAA AGAACUUG | CAAGUUCUU AUACAGAG |
| 1998 | UCUCUGUA CUGAUGA X GAA AAGAACUU | AAGUUCUUA UACAGAGA |
| 2000 | CGUCUCUG CUGAUGA X GAA AUAAGAAC | GUUCUUAUA CAGAGACG |
| 2010 | AUCCAAGU CUGAUGA X GAA ACGUCUCU | AGAGACGUU ACUUGGAU |
| 2011 | AAUCCAAG CUGAUGA X GAA AACGUCUC | GAGACGUUA CUUGGAUU |
| 2014 | UAAAAUCC CUGAUGA X GAA AGUAACGU | ACGUUACUU GGAUUUUA |
| 2019 | CGCAGUAA CUGAUGA X GAA AUCCAAGU | ACUUGGAUU UUACUGCG |
| 2020 | CCGCAGUA CUGAUGA X GAA AAUCCAAG | CUUGGAUUU UACUGCGG |
| 2021 | UCCGCAGU CUGAUGA X GAA AAAUCCAA | UUGGAUUUU ACUGCGGA |
| 2022 | GUCCGCAG CUGAUGA X GAA AAAAUCCA | UGGAUUUUA CUGCGGAC |
| 2034 | CUGUUAUU CUGAUGA X GAA ACUGUCCG | CGGACAGUU AAUAACAG |
| 2035 | UCUGUUAU CUGAUGA X GAA AACUGUCC | GGACAGUUA AUAACAGA |
| 2038 | UGUUCUGU CUGAUGA X GAA AUUAACUG | CAGUUAAUA ACAGAACA |
| 2054 | UAAUACUG CUGAUGA X GAA AGUGCAUU | AAUGCACUA CAGUAUUA |
| 2059 | CUUGCUAA CUGAUGA X GAA ACUGUAGU | ACUACAGUA WAGCAAG |
| 2061 | UGCUUGCU CUGAUGA X GAA AUACUGUA | UACAGUAUU AGCAAGCA |
| 2062 | UUGCUUGC CUGAUGA X GAA AAUACUGU | ACAGUAUUA GCAAGCAA |
| 2082 | UCCUUAGU CUGAUGA X GAA AUGGCCAU | AUGGCCAUC ACUAAGGA |
| 2086 | GUGCUCCU CUGAUGA X GAA AGUGAUGG | CCAUCACUA AGGAGCAC |
| 2096 | GAGUGAUG CUGAUGA X GAA AGUGCUCC | GGAGCACUC CAUCACUC |
| 2100 | WAAGAGU CUGAUGA X GAA AUGGAGUG | CACUCCAUC ACUCUUAA |
| 2104 | AAGAUUAA CUGAUGA X GAA AGUGAUGG | CCAUCACUC UUAAUCUU |
| 2106 | GUAAGAUU CUGAUGA X GAA AGAGUGAU | AUCACUCUU AAUCUUAC |
| 2107 | GGUAAGAU CUGAUGA X GAA AAGAGUGA | UCACUCUUA AUCUUACC |
| 2110 | GAUGGUAA CUGAUGA X GAA AUUAAGAG | CUCUUAAUC UUACCAUC |
| 2112 | AUGAUGGU CUGAUGA X GAA AGAUUAAG | CUUAAUCUU ACCAUCAU |
| 2113 | CAUGAUGG CUGAUGA X GAA AAGAUUAA | UUAAUCUUA CCAUCAUG |
| 2118 | ACAUUCAU CUGAUGA X GAA AUGGUAAG | CUUACCAUC AUGAAUGU |
| 2127 | UGCAGGGA CUGAUGA X GAA ACAUUCAU | AUGAAUGUU UCCCUGCA |
| 2128 | UUGCAGGG CUGAUGA X GAA AACAUUCA | UGAAUCUUU CCCUGCAA |
| 2129 | CUUGCAGG CUGAUGA X GAA AAACAUUC | GAAUGUUUC CCUGCAAG |
| 2140 | GGUGCCUG CUGAUGA X GAA AUCUUGCA | UGCAAGAUU CAGGCACC |
| 2141 | AGGUGCCU CUGAUGA X GAA AAUCUUGC | GCAAGAUUC AGGCACCU |
| 2150 | UGCAGGCA CUGAUGA X GAA AGGUGCCU | AGGCACCUA UGCCUGCA |

(continued)

| nt. Position | HH Ribozyme | Substrate |
|---|---|---|
| 2172 | CCUGUGUA CUGAUGA X GAA ACAWCCU | AGGAAUGUA UACACAGG |
| 2174 | CCCCUGUG CUGAUGA X GAA AUACAUUC | GAAUGUAUA CACAGGGG |
| 2190 | UUCUGGAG CUGAUGA X GAA AUUUCUUC | GAAGAAAUC CUCCAGAA |
| 2193 | UUCUUCUG CUGAUGA X GAA AGGAUUUC | GAAAUCCUC CAGAAGAA |
| 2208 | CUGAUUGU CUGAUGA X GAA AUUUCUUU | AAAGAAAUU ACAAUCAG |
| 2209 | UCUGAUUG CUGAUGA X GAA AAUUUCUU | AAGAAAUUA CAAUCAGA |
| 2214 | UGAUCUCU CUGAUGA X GAA AUUGUAAU | AUUACAAUC AGAGAUCA |
| 2221 | UGCUUCCU CUGAUGA X GAA AUCUCUGA | UCAGAGAUC AGGAAGCA |
| 2234 | GCAGGAGG CUGAUGA X GAA AUGGUGCU | AGCACCAUA CCUCCUGC |
| 2238 | WUCGCAG CUGAUGA X GAA AGGUAUGG | CCAUACCUC CUGCGAAA |
| 2250 | UGAUCACU CUGAUGA X GAA AGGUWCG | CGAAACCUC AGUGAUCA |
| 2257 | CACUGUGU CUGAUGA X GAA AUCACUGA | UCAGUGAUC ACACAGUG |
| 2271 | GAACUGCU CUGAUGA X GAA AUGGCCAC | GUGGCCAUC AGCAGUUC |
| 2278 | AGUGGUGG CUGAUGA X GAA ACUGCUGA | UCAGCAGUU CCACCACU |
| 2279 | AAGUGGUG CUGAUGA X GAA AACUGCUG | CAGCAGUUC CACCACUU |
| 2287 | ACAGUCUA CUGAUGA X GAA AGUGGUGG | CCACCACUU UAGACUGU |
| 2288 | GACAGUCU CUGAUGA X GAA AAGUGGUG | CACCACUUU AGACUGUC |
| 2289 | UGACAGUC CUGAUGA X GAA AAAGUGGU | ACCACUUUA GACUGUCA |
| 2296 | AUUAGCAU CUGAUGA X GAA ACAGUCUA | UAGACUGUC AUGCUAAU |
| 2302 | GACACCAU CUGAUGA X GAA AGCAUGAC | GUCAUGCUA AUGGUGUC |
| 2310 | GGCUCGGG CUGAUGA X GAA ACACCAUU | AAUGGUGUC CCCGAGCC |
| 2320 | AGUGAUCU CUGAUGA X GAA AGGCUCGG | CCGAGCCUC AGAUCACU |
| 2325 | AACCAAGU CUGAUGA X GAA AUCUGAGG | CCUCAGAUC ACUUGGUU |
| 2329 | WUAAACC CUGAUGA X GAA AGUGAUCU | AGAUCACUU GGUUUAAA |
| 2333 | UGUUUUUA CUGAUGA X GAA ACCAAGUG | CACUUGGUU UAAAAACA |
| 2334 | UUGUUUUU CUGAUGA X GAA AACCAAGU | ACUUGGUUU AAAAACAA |
| 2335 | GUUGUUUU CUGAUGA X GAA AAACCAAG | CUUGGUUUA AAAACAAC |
| 2352 | UCUUGUUG CUGAUGA X GAA AUUUUGUG | CACAAAAUA CAACAAGA |
| 2370 | CCUAAAAU CUGAUGA X GAA AUUCCAGG | CCUGGAAUU AUUUUAGG |
| 2371 | UCCUAAAA CUGAUGA X GAA AAUUCCAG | CUGGAAUUA UUUUAGGA |
| 2373 | GGUCCUAA CUGAUGA X GAA AUAAUUCC | GGAAUUAUU UUAGGACC |
| 2374 | UGGUCCUA CUGAUGA X GAA AAUAAUUC | GAAWAUW UAGGACCA |
| 2375 | CUGGUCCU CUGAUGA X GAA AAAUAAUU | AAUUAUUUU AGGACCAG |
| 2376 | CCUGGUCC CUGAUGA X GAA AAAAUAAU | AUUAUUUUA GGACCAGG |
| 2399 | WUCAAUA CUGAUGA X GAA ACAGCGUG | CACGCUGUU UAUUGAAA |
| 2400 | CUUUCAAU CUGAUGA X GAA AACAGCGU | ACGCUGUUU AUUGAAAG |
| 2401 | UCUUUCAA CUGAUGA X GAA AAACAGCG | CGCUGUUUA UUGAAAGA |
| 2403 | ACUCUUUC CUGAUGA X GAA AUAAACAG | CUGUUUAUU GAAAGAGU |
| 2412 | UCUUCUGU CUGAUGA X GAA ACUCUUUC | GAAAGAGUC ACAGAAGA |
| 2433 | CAGUGAUA CUGAUGA X GAA ACACCUUC | GAAGGUGUC UAUCACUG |
| 2435 | UGCAGUGA CUGAUGA X GAA AGACACCU | AGGUGUCUA UCACUGCA |
| 2437 | UUUGCAGU CUGAUGA X GAA AUAGACAC | GUGUCUAUC ACUGCAAA |
| 2465 | UUUCCACA CUGAUGA X GAA AGCCCUUC | GAAGGGCUC UGUGGAAA |
| 2476 | GUAUGCUG CUGAUGA X GAA ACUUUCCA | UGGAAAGUU CAGCAUAC |
| 2477 | GGUAUGCU CUGAUGA X GAA AACUUUCC | GGAAAGUUC AGCAUACC |
| 2483 | CAGUGAGG CUGAUGA X GAA AUGCUGAA | UUCAGCAUA CCUCACUG |
| 2487 | UGAACAGU CUGAUGA X GAA AGGUAUGC | GCAUACCUC ACUGUUCA |
| 2493 | GUUCCUUG CUGAUGA X GAA ACAGUGAG | CUCACUGUU CAAGGAAC |
| 2494 | GGUUCCUU CUGAUGA X GAA AACAGUGA | UCACUGUUC AAGGAACC |
| 2504 | ACUUGUCC CUGAUGA X GAA AGGUUCCU | AGGAACCUC GGACAAGU |

(continued)

| nt. Position | HH Ribozyme | Substrate |
|---|---|---|
| 2513 | CCAGAUUA CUGAUGA X GAA ACUUGUCC | GGACAAGUC UAAUCUGG |
| 2515 | CUCCAGAU CUGAUGA X GAA AGACUUGU | ACAACUCUA AUCUGGAG |
| 2518 | CAGCUCCA CUGAUGA X GAA AUUAGACU | AGUCUAAUC UGGAGCUG |
| 2529 | GUUAGAGU CUGAUGA X GAA AUCAGCUC | GAGCUGAUC ACUCUAAC |
| 2533 | GCAUGUUA CUGAUGA X GAA AGUGAUCA | UGAUCACUC UAACAUGC |
| 2535 | GUGCAUGU CUGAUGA X GAA AGAGUGAU | AUCACUCUA ACAUGCAC |
| 2560 | CCAGAAGA CUGAUGA X GAA AGUCGCAG | CUGCGACUC UCUUCUGG |
| 2562 | AGCCAGAA CUGAUGA X GAA AGAGUCGC | GCGACUCUC UUCUGGCU |
| 2564 | GGAGCCAG CUGAUGA X GAA AGAGAGUC | GACUCUCUU CUGGCUCC |
| 2565 | AGGAGCCA CUGAUGA X GAA AAGAGAGU | ACUCUCUUC UGGCUCCU |
| 2571 | GUUAAUAG CUGAUGA X GAA AGCCAGAA | UUCUGGCUC CUAUUAAC |
| 2574 | AGGGUUAA CUGAUGA X GAA AGGAGCCA | UGGCUCCUA UUAACCCU |
| 2576 | GGAGGGUU CUGAUGA X GAA AUAGGAGC | GCUCCUAUU AACCCUCC |
| 2577 | AGGAGGGU CUGAUGA X GAA AAUAGGAG | CUCCUAUUA ACCCUCCU |
| 2583 | CGGAUAAG CUGAUGA X GAA AGGGUUAA | UUAACCCUC CUUAUCCG |
| 2586 | UUUCGGAU CUGAUGA X GAA AGGAGGGU | ACCCUCCUU AUCCGAAA |
| 2587 | UUUUCGGA CUGAUGA X GAA AAGGAGGG | CCCUCCUUA UCCGAAAA |
| 2589 | AUUUUUCG CUGAUGA X GAA AUAAGGAG | CUCCUUAUC CGAAAAAU |
| 2606 | CAGAAGAA CUGAUGA X GAA ACCUUUUC | GAAAAGGUC UUCUUCUG |
| 2608 | UUCAGAAG CUGAUGA X GAA AGACCUUU | AAAGGUCUU CUUCUGAA |
| 2609 | UUUCAGAA CUGAUGA X GAA AAGACCUU | AAGGUCUUC UUCUGAAA |
| 2611 | UAUUUCAG CUGAUGA X GAA AGAAGACC | GGUCUUCUU CUGAAAUA |
| 2612 | UUAUUUCA CUGAUGA X GAA AAGAAGAC | GUCUUCUUC UGAAAUAA |
| 2619 | UCAGUCUU CUGAUGA X GAA AUUUCAGA | UCUGAAAUA AAGACUGA |
| 2630 | UUGAUAGG CUGAUGA X GAA AGUCAGUC | GACUGACUA CCUAUCAA |
| 2634 | AUAAUUGA CUGAUGA X GAA AGGUAGUC | GACUACCUA UCAAUUAU |
| 2636 | UUAUAAUU CUGAUGA X GAA AUAGGUAG | CUACCUAUC AAUUAUAA |
| 2640 | UCCAUUAU CUGAUGA X GAA AUUGAUAG | CUAUCAAUU AUAAUGGA |
| 2641 | GUCCAUUA CUGAUGA X GAA AAUUGAUA | UAUCAAUUA UAAUGGAC |
| 2643 | GGGUCCAU CUGAUGA X GAA AUAAUUGA | UCAAUUAUA AUGGACCC |
| 2661 | UCCAAAGG CUGAUGA X GAA ACUUCAUC | GAUGAAGUU CCUUUGGA |
| 2662 | AUCCAAAG CUGAUGA X GAA AACUUCAU | AUGAAGUUC CUUUGGAU |
| 2665 | CUCAUCCA CUGAUGA X GAA AGGAACUU | AAGUUCCUU UGGAUGAG |
| 2666 | GCUCAUCC CUGAUGA X GAA AAGGAACU | AGUUCCUUU GGAUGAGC |
| 2688 | UCAUAAGG CUGAUGA X GAA AGCCGCUC | GAGCGGCUC CCUUAUGA |
| 2692 | GGCAUCAU CUGAUGA X GAA AGGGAGCC | GGCUCCCUU AUGAUGCC |
| 2693 | UGGCAUCA CUGAUGA X GAA AAGGGAGC | GCUCCCUUA UGAUGCCA |
| 2714 | CCCGGGCA CUGAUGA X GAA ACUCCCAC | GUGGGAGUU UGCCCGGG |
| 2715 | UCCCGGGC CUGAUGA X GAA AACUCCCA | UGGGAGUUU GCCCGGGA |
| 2730 | CCCAGUUU CUGAUGA X GAA AGUCUCUC | GAGAGACUU AAACUGGG |
| 2731 | GCCCAGUU CUGAUGA X GAA AAGUCUCU | AGAGACUUA AACUGGGC |
| 2744 | UUCCAAGU CUGAUGA X GAA AUUUGCCC | GGGCAAAUC ACUUGGAA |
| 2748 | CCUCUUCC CUGAUGA X GAA AGUGAUUU | AAAUCACUU GGAAGAGG |
| 2761 | UUUUCCAA CUGAUGA X GAA AGCCCCUC | GAGGGGCUU UUGGAAAA |
| 2762 | CUUUUCCA CUGAUGA X GAA AAGCCCCU | AGGGGCUUU UGGAAAAG |
| 2763 | ACUUUUCC CUGAUGA X GAA AAAGCCCC | GGGGCUUUU GGAAAAGU |
| 2775 | GAUGCUUG CUGAUGA X GAA ACCACUUU | AAAGUGGUU CAAGCAUC |
| 2776 | UGAUGCUU CUGAUGA X GAA AACCACUU | AAGUGGUUC AAGCAUCA |
| 2783 | CAAAUGCU CUGAUGA X GAA AUGCUUGA | UCAAGCAUC AGCAUUUG |
| 2789 | UAAUGCCA CUGAUGA X GAA AUGCUGAU | AUCAGCAUU UGGCAUUA |

(continued)

| nt. Position | HH Ribozyme | Substrate |
|---|---|---|
| 2790 | UUAAUGCC CUGAUGA X GAA AAUGCUGA | UCAGCAUUU GGCAUUAA |
| 2796 | GAUUUCUU CUGAUGA X GAA AUGCCAAA | UUUGGCAUU AAGAAAUC |
| 2797 | UGAUUUCU CUGAUGA X GAA AAUGCCAA | UUGGCAUUA AGAAAUCA |
| 2804 | ACGUAGGU CUGAUGA X GAA AUUUCUUA | UAAGAAAUC ACCUACGU |
| 2809 | CCGGCACG CUGAUGA X GAA AGGUGAUU | AAUCACCUA CGUGCCGG |
| 2864 | GAGCUUUG CUGAUGA X GAA ACUCGCUG | CAGCGAGUA CAAAGCUC |
| 2872 | AGUCAUCA CUGAUGA X GAA AGCUUUGU | ACAAAGCUC UGAUGACU |
| 2886 | AAGAUUUU CUGAUGA X GAA AGCUCAGU | ACUGAGCUA AAAAUCUU |
| 2892 | UGGGUCAA CUGAUGA X GAA AUUUUUAG | CUAAAAAUC UUGACCCA |
| 2894 | UGUGGGUC CUGAUGA X GAA AGAUUUW | AAAAAUCW GACCCACA |
| 2904 | UGGUGGCC CUGAUGA X GAA AUGUGGGU | ACCCACAUU GGCCACCA |
| 2914 | CACGUUCA CUGAUGA X GAA AUGGUGGC | GCCACCAUC UGAACGUG |
| 2925 | AGCAGGUU CUGAUGA X GAA ACCACGUU | AACGUGGUU AACCUGCU |
| 2926 | CAGCAGGU CUGAUGA X GAA AACCACGU | ACGUGGUUA ACCUGCUG |
| 2962 | CACCAUCA CUGAUGA X GAA AGGCCCUC | GAGGGCCUC UGAUGGUG |
| 2973 | UAUUCAAC CUGAUGA X GAA AUCACCAU | AUGGUGAUU GUUGAAUA |
| 2976 | CAGUAUUC CUGAUGA X GAA ACAAUCAC | GUGAUUGUU GAAUACUG |
| 2981 | AUUUGCAG CUGAUGA X GAA AUUCAACA | UGUUGAAUA CUGCAAAU |
| 2990 | GAUUUCCA CUGAUGA X GAA AUUUGCAG | CUGCAAAUA UGGAAAUC |
| 2998 | GUUGGAGA CUGAUGA X GAA AUUUCCAU | AUGGAAAUC UCUCCAAC |
| 3000 | UAGUUGGA CUGAUGA X GAA AGAUUUCC | GGAAAUCUC UCCAACUA |
| 3002 | GGUAGUUG CUGAUGA X GAA AGAGAUUU | AAAUCUCUC CAACUACC |
| 3008 | UCUUGAGG CUGAUGA X GAA AGUUGGAG | CUCCAACUA CCUCAAGA |
| 3012 | UUGCUCUU CUGAUGA X GAA AGGUAGUU | AACUACCUC AAGAGCAA |
| 3029 | GAAAAAAU CUGAUGA X GAA AGUCACGU | ACGUGACUU AUUUUUUC |
| 3030 | AGAAAAAA CUGAUGA X GAA AAGUCACG | CGUGACUUA UUUUUUCU |
| 3032 | UGAGAAAA CUGAUGA X GAA AUAAGUCA | UGACUUAUU UUUUCUCA |
| 3033 | UUGAGAAA CUGAUGA X GAA AAUAAGUC | GACUUAUUU UUUCUCAA |
| 3034 | GUUGAGAA CUGAUGA X GAA AAAUAAGU | ACUUAUUUU UUCUCAAC |
| 3035 | UGUUGAGA CUGAUGA X GAA AAAAUAAG | CUUAUUUUU UCUCAACA |
| 3036 | UUGUUGAG CUGAUGA X GAA AAAAAUAA | UUAUUUUUU CUCAACAA |
| 3037 | CUUGUUGA CUGAUGA X GAA AAAAAAUA | UAUUUUUUC UCAACAAG |
| 3039 | UCCUUGUU CUGAUGA X GAA AGAAAAAA | UUUUUUCUC AACAAGGA |
| 3057 | UCCAUGUG CUGAUGA X GAA AGUGCUGC | GCAGCACUA CACAUGGA |
| 3070 | UUCUUUCU CUGAUGA X GAA AGGCUCCA | UGGAGCCUA AGAAAGAA |
| 3120 | ACGCUAUC CUGAUGA X GAA AGUCUUGG | CCAAGACUA GAUAGCGU |
| 3124 | GGUGACGC CUGAUGA X GAA AUCUAGUC | GACUAGAUA GCGUCACC |
| 3129 | CUGCUGGU CUGAUGA X GAA ACGCUAUC | GAUAGCGUC ACCAGCAG |
| 3146 | AGCUCGCA CUGAUGA X GAA AGCUUUCG | CGAAAGCUU UGCGAGCU |
| 3147 | GAGCUCGC CUGAUGA X GAA AAGCUUUC | GAAAGCUUU GCGAGCUC |
| 3155 | GAAAGCCG CUGAUGA X GAA AGCUCGCA | UGCGAGCUC CGGCUUUC |
| 3161 | CUUCCUGA CUGAUGA X GAA AGCCGGAG | CUCCGGCUU UCAGGAAG |
| 3162 | UCUUCCUG CUGAUGA X GAA AAGCCGGA | UCCGGCUUU CAGGAAGA |
| 3163 | AUCUUCCU CUGAUGA X GAA AAAGCCGG | CCGGCUUUC AGGAAGAU |
| 3172 | CAGACUUU CUGAUGA X GAA AUCUUCCU | AGGAAGAUA AAAGUCUG |
| 3178 | AUCACUCA CUGAUGA X GAA ACUUUUAU | AUAAAAGUC UGAGUGAU |
| 3189 | UCUUCCUC CUGAUGA X GAA ACAUCACU | AGUGAUGUU GAGGAAGA |
| 3205 | ACCGUCAG CUGAUGA X GAA AUCCUCCU | AGGAGGAUU CUGACGGU |
| 3206 | AACCGUCA CUGAUGA X GAA AAUCCUCC | GGAGGAUUC UGACGGUU |
| 3214 | CUUGUAGA CUGAUGA X GAA ACCGUCAG | CUGACGGUU UCUACAAG |

(continued)

| nt. Position | HH Ribozyme | Substrate |
|---|---|---|
| 3215 | CCUUGUAG CUGAUGA X GAA AACCGUCA | UGACGGUUU CUACAAGG |
| 3216 | UCCUUGUA CUGAUGA X GAA AAACCGUC | GACGGUUUC UACAAGGA |
| 3218 | GCUCCUUG CUGAUGA X GAA AGAAACCG | CGGUUUCUA CAAGGAGC |
| 3231 | UCCAUAGU CUGAUGA X GAA AUGGGCUC | GAGCCCAUC ACUAUGGA |
| 3235 | AUCUUCCA CUGAUGA X GAA AGUGAUGG | CCAUCACUA UGGAAGAU |
| 3244 | AGAAAUCA CUGAUGA X GAA AUCUUCCA | UGGAAGAUC UGAUUUCU |
| 3249 | CUGUAAGA CUGAUGA X GAA AUCAGAUC | GAUCUGAUU UCUUACAG |
| 3250 | ACUGUAAG CUGAUGA X GAA AAUCAGAU | AUCUGAUUU CUUACAGU |
| 3251 | AACUGUAA CUGAUGA X GAA AAAUCAGA | UCUGAUUUC UUACAGUU |
| 3253 | AAAACUGU CUGAUGA X GAA AGAAAUCA | UGAUUUCUU ACAGUUUU |
| 3254 | GAAAACUG CUGAUGA X GAA AAGAAAUC | GAUUUCUUA CAGUUUUC |
| 3259 | CACUUGAA CUGAUGA X GAA ACUGUAAG | CUUACAGUU UUCAAGUG |
| 3260 | CCACUUGA CUGAUGA X GAA AACUGUAA | UUACAGUUU UCAAGUGG |
| 3261 | GCCACUUG CUGAUGA X GAA AAACUGUA | UACAGUUUU CAAGUGGC |
| 3262 | GGCCACUU CUGAUGA X GAA AAAACUGU | ACAGUUUUC AAGUGGCC |
| 3284 | AAGACAGG CUGAUGA X GAA ACUCCAUG | CAUGGAGUU CCUGUCUU |
| 3285 | GAAGACAG CUGAUGA X GAA AACUCCAU | AUGGAGUUC CUGUCUUC |
| 3290 | UUCUGGAA CUGAUGA X GAA ACAGGAAC | GUUCCUGUC UUCCAGAA |
| 3292 | CUUUCUGG CUGAUGA X GAA AGACAGGA | UCCUGUCUU CCAGAAAG |
| 3293 | ACUUUCUG CUGAUGA X GAA AAGACAGG | CCUGUCUUC CAGAAAGU |
| 3306 | UCCCGAUG CUGAUGA X GAA AUGCACUU | AAGUGCAUU CAUCGGGA |
| 3307 | GUCCCGAU CUGAUGA X GAA AAUGCACU | AGUGCAUUC AUCGGGAC |
| 3310 | CAGGUCCC CUGAUGA X GAA AUGAAUGC | GCAUUCAUC GGGACCUG |
| 3333 | GAUAAAAG CUGAUGA X GAA AUGUUUCU | AGAAACAUU CUUUUAUC |
| 3334 | AGAUAAAA CUGAUGA X GAA AAUGUUUC | GAAACAUUC UUUUAUCU |
| 3336 | UCAGAUAA CUGAUGA X GAA AGAAUGUU | AACAUUCUU UUAUCUGA |
| 3337 | CUCAGAUA CUGAUGA X GAA AAGAAUGU | ACAUUCUUU UAUCUGAG |
| 3338 | UCUCAGAU CUGAUGA X GAA AAAGAAUG | CAUUCUUUU AUCUGAGA |
| 3339 | UUCUCAGA CUGAUGA X GAA AAAAGAAU | AUUCUUUUA UCUGAGAA |
| 3341 | UGUUCUCA CUGAUGA X GAA AUAAAAGA | UCUUUUAUC UGAGAACA |
| 3363 | AAAUCACA CUGAUGA X GAA AUCUUCAC | GUGAAGAUU UGUGAUUU |
| 3364 | AAAAUCAC CUGAUGA X GAA AAUCUUCA | UGAAGAUUU GUGAUUUU |
| 3370 | AAGGCCAA CUGAUGA X GAA AUCACAAA | UUUGUGAUU UUGGCCUU |
| 3371 | CAAGGCCA CUGAUGA X GAA AAUCACAA | UUGUGAUUU UGGCCUUG |
| 3372 | GCAAGGCC CUGAUGA X GAA AAAUCACA | UGUGAUUUU GGCCUUGC |
| 3378 | UCCCGGGC CUGAUGA X GAA AGGCCAAA | UUUGGCCUU GCCCGGGA |
| 3388 | CUUAUAAA CUGAUGA X GAA AUCCCGGG | CCCGGGAUA UUUAUAAG |
| 3390 | UUCUUAUA CUGAUGA X GAA AUAUCCCG | CGGGAUAUU UAUAAGAA |
| 3391 | GUUCUUAU CUGAUGA X GAA AAUAUCCC | GGGAUAUUU AUAAGAAC |
| 3392 | GGUUCUUA CUGAUGA X GAA AAAUAUCC | GGAUAUUUA UAAGAACC |
| 3394 | GGGGUUCU CUGAUGA X GAA AUAAAUAU | AUAUUUAUA AGAACCCC |
| 3406 | UCUCACAU CUGAUGA X GAA AUCGGGGU | ACCCCGAUU AUGUGAGA |
| 3407 | UUCUCACA CUGAUGA X GAA AAUCGGGG | CCCCGAUUA UGUGAGAA |
| 3424 | AAGUCGAG CUGAUGA X GAA AUCUCCUU | AAGGAGAUA CUCGACUU |
| 3427 | AGGAAGUC CUGAUGA X GAA AGUAUCUC | GAGAUACUC GACUUCCU |
| 3432 | UUCAGAGG CUGAUGA X GAA AGUCGAGU | ACUCGACUU CCUCUGAA |
| 3433 | UUUCAGAG CUGAUGA X GAA AAGUCGAG | CUCGACUUC CUCUGAAA |
| 3436 | CCAUUUCA CUGAUGA X GAA AGGAAGUC | GACUUCCUC UGAAAUGG |
| 3451 | AGAUUCGG CUGAUGA X GAA AGCCAUCC | GGAUGGCUC CCGAAUCU |
| 3458 | CAAAGAUA CUGAUGA X GAA AUUCGGGA | UCCCGAAUC UAUCUUUG |

(continued)

| nt. Position | HH Ribozyme | Substrate |
|---|---|---|
| 3460 | GUCAAAGA CUGAUGA X GAA AGAUUCGG | CCGAAUCUA UCUUUGAC |
| 3462 | UUGUCAAA CUGAUGA X GAA AUAGAUUC | GAAUCUAUC UUUGACAA |
| 3464 | UUWGUCA CUGAUGA X GAA AGAUAGAU | AUCUAUCUU UGACAAAA |
| 3465 | AUUUUGUC CUGAUGA X GAA AAGAUAGA | UCUAUCUUU GACAAAAU |
| 3474 | GUGCUGUA CUGAUGA X GAA AUUUUGUC | GACAAAAUC UACAGCAC |
| 3476 | UGGUGCUG CUGAUGA X GAA AGAUUUUG | CAAAAUCUA CAGCACCA |
| 3500 | CUCCGUAA CUGAUGA X GAA ACCACACG | CGUGUGGUC UUACGGAG |
| 3502 | UACUCCGU CUGAUGA X GAA AGACCACA | UGUGGUCUU ACGGAGUA |
| 3503 | AUACUCCG CUGAUGA X GAA AAGACCAC | GUGGUCUUA CGGAGUAU |
| 3510 | CACAGCAA CUGAUGA X GAA ACUCCGUA | UACGGAGUA UUGCUGUG |
| 3512 | CCCACAGC CUGAUGA X GAA AUACUCCG | CGGAGUAUU GCUGUGGG |
| 3525 | AAGGAGAA CUGAUGA X GAA AUUUCCCA | UGGGAAAUC UUCUCCUU |
| 3527 | CUAAGGAG CUGAUGA X GAA AGAUUUCC | GGAAAUCUU CUCCUUAG |
| 3528 | CCUAAGGA CUGAUGA X GAA AAGAUUUC | GAAAUCUUC UCCUUAGG |
| 3530 | CACCUAAG CUGAUGA X GAA AGAAGAUU | AAUCUUCUC CUUAGGUG |
| 3533 | ACCCACCU CUGAUGA X GAA AGGAGAAG | CUUCUCCUU AGGUGGGU |
| 3534 | GACCCACC CUGAUGA X GAA AAGGAGAA | UUCUCCUU GGUGGGUC |
| 3542 | GGUAUGGA CUGAUGA X GAA ACCCACCU | AGGUGGGUC UCCAUACC |
| 3544 | UGGGUAUG CUGAUGA X GAA AGACCCAC | GUGGGUCUC CAUACCCA |
| 3548 | CUCCUGGG CUGAUGA X GAA AUGGAGAC | GUCUCCAUA CCCAGGAG |
| 3558 | UCCAUUUG CUGAUGA X GAA ACUCCUGG | CCAGGAGUA CAAAUGGA |
| 3575 | GACUGCAA CUGAUGA X GAA AGUCCUCA | UGAGGACUU UUGCAGUC |
| 3576 | CGACUGCA CUGAUGA X GAA AAGUCCUC | GAGGACUUU UGCAGUCG |
| 3577 | GCGACUGC CUGAUGA X GAA AAAGUCCU | AGGACUUUU GCAGUCGC |
| 3583 | CCUCAGGC CUGAUGA X GAA ACUGCAAA | UUUGCAGUC GCCUGAGG |
| 3613 | GUACUCAG CUGAUGA X GAA AGCUCUCA | UGAGAGCUC CUGAGUAC |
| 3620 | GAGUAGAG CUGAUGA X GAA ACUCAGGA | UCCUGAGUA CUCUACUC |
| 3623 | CAGGAGUA CUGAUGA X GAA AGUACUCA | UGAGUACUC UACUCCUG |
| 3625 | UUCAGGAG CUGAUGA X GAA AGAGUACU | AGUACUCUA CUCCUGAA |
| 3628 | GAUUUCAG CUGAUGA X GAA AGUAGAGU | ACUCUACUC CUGAAAUC |
| 3636 | AUCUGAUA CUGAUGA X GAA AUUUCAGG | CCUGAAAUC UAUCAGAU |
| 3638 | UGAUCUGA CUGAUGA X GAA AGAUUUCA | UGAAAUCUA UCAGAUCA |
| 3640 | CAUGAUCU CUGAUGA X GAA AUAGAUUU | AAAUCUAUC AGAUCAUG |
| 3645 | UCCAGCAU CUGAUGA X GAA AUCUGAUA | UAUCAGAUC AUGCUGGA |
| 3689 | GUUCUGCA CUGAUGA X GAA AUCUUGGC | GCCAAGAUU UGCAGAAC |
| 3690 | AGUUCUGC CUGAUGA X GAA AAUCUUGG | CCAAGAUUU GCAGAACU |
| 3699 | UUUUCCAC CUGAUGA X GAA AGUUCUGC | GCAGAACUU GUGGAAAA |
| 3711 | AAAUCACC CUGAUGA X GAA AGUUUUUC | GAAAAACUA GGUGAUUU |
| 3718 | UUGAAGCA CUGAUGA X GAA AUCACCUA | UAGGUGAUU UGCUUCAA |
| 3719 | CUUGAAGC CUGAUGA X GAA AAUCACCU | AGGUGAUUU GCUUCAAG |
| 3723 | UUUGCUUG CUGAUGA X GAA AGCAAAUC | GAUUUGCUU CAAGCAAA |
| 3724 | AUUUGCUU CUGAUGA X GAA AAGCAAAU | AUUUGCUUC AAGCAAAU |
| 3735 | UCCUGUUG CUGAUGA X GAA ACAUUUGC | GCAAAUGUA CAACAGGA |
| 3748 | GUAGUCUU CUGAUGA X GAA ACCAUCCU | AGGAUGGUA AAGACUAC |
| 3755 | UUGGGAUG CUGAUGA X GAA AGUCUUUA | UAAAGACUA CAUCCCAA |
| 3759 | UUGAUUGG CUGAUGA X GAA AUGUAGUC | GACUACAUC CCAAUCAA |
| 3765 | AUGGCAUU CUGAUGA X GAA AUUGGGAU | AUCCCAAUC AAUGCCAU |
| 3774 | CCUGUCAG CUGAUGA X GAA AUGGCAUU | AAUGCCAUA CUGACAGG |
| 3787 | AAACCCAC CUGAUGA X GAA AUUUCCUG | CAGGAAAUA GUGGGUUU |
| 3794 | AGUAUGUA CUGAUGA X GAA ACCCACUA | UAGUGGGUU UACAUACU |

(continued)

| nt. Position | HH Ribozyme | Substrate |
|---|---|---|
| 3795 | GAGUAUGU CUGAUGA X GAA AACCCACU | AGUGGGUUU ACAUACUC |
| 3796 | UGAGUAUG CUGAUGA X GAA AAACCCAC | GUGGGUUUA CAUACUCA |
| 3800 | GAGUUGAG CUGAUGA X GAA AUGUAAAC | GUUUACAUA CUCAACUC |
| 3803 | CAGGAGUU CUGAUGA X GAA AGUAUGUA | UACAUACUC AACUCCUG |
| 3808 | GAAGGCAG CUGAUGA X GAA AGUUGAGU | ACUCAACUC CUGCCUUC |
| 3815 | CCUCAGAG CUGAUGA X GAA AGGCAGGA | UCCUGCCUU CUCUGAGG |
| 3816 | UCCUCAGA CUGAUGA X GAA AAGGCAGG | CCUGCCUUC UCUGAGGA |
| 3818 | AGUCCUCA CUGAUGA X GAA AGAAGGCA | UGCCUUCUC UGAGGACU |
| 3827 | CCUUGAAG CUGAUGA X GAA AGUCCUCA | UGAGGACUU CUUCAAGG |
| 3828 | UCCUUGAA CUGAUGA X GAA AAGUCCUC | GAGGACUUC UUCAAGGA |
| 3830 | UUUCCUUG CUGAUGA X GAA AGAAGUCC | GGACUUCUU CAAGGAAA |
| 3831 | CUUUCCUU CUGAUGA X GAA AAGAAGUC | GACUUCUUC AAGGAAAG |
| 3841 | AGCUGAAA CUGAUGA X GAA ACUUUCCU | AGGAAAGUA UUUCAGCU |
| 3843 | GGAGCUGA CUGAUGA X GAA AUACUUUC | GAAAGUAUU UCAGCUCC |
| 3844 | CGGAGCUG CUGAUGA X GAA AAUACUUU | AAAGUAUUU CAGCUCCG |
| 3845 | UCGGAGCU CUGAUGA X GAA AAAUACUU | AAGUAUUUC AGCUCCGA |
| 3850 | AAACUUCG CUGAUGA X GAA AGCUGAAA | UUUCAGCUC CGAAGUUU |
| 3857 | CUGAAUUA CUGAUGA X GAA ACUUCGGA | UCCGAAGUU UAAUUCAG |
| 3858 | CCUGAAUU CUGAUGA X GAA AACUUCGG | CCGAAGUUU AAUUCAGG |
| 3859 | UCCUGAAU CUGAUGA X GAA AAACUUCG | CGAAGUUUA AUUCAGGA |
| 3862 | GCUUCCUG CUGAUGA X GAA AUUAAACU | AGUUUAAUU CAGGAAGC |
| 3863 | AGCUUCCU CUGAUGA X GAA AAUUAAAC | GUUUAAUUC AGGAAGCU |
| 3872 | CAUCAUCA CUGAUGA X GAA AGCUUCCU | AGGAAGCUC UGAUGAUG |
| 3882 | ACAUAUCU CUGAUGA X GAA ACAUCAUC | GAUGAUGUC AGAUAUGU |
| 3887 | CAUUUACA CUGAUGA X GAA AUCUGACA | UGUCAGAUA UGUAAAUG |
| 3891 | AAAGCAUU CUGAUGA X GAA ACAUAUCU | AGAUAUGUA AAUGCUUU |
| 3898 | GAACUUGA CUGAUGA X GAA AGCAUUUA | UAAAUGCUU UCAAGUUC |
| 3899 | UGAACUUG CUGAUGA X GAA AAGCAUUU | AAAUGCUUU CAAGUUCA |
| 3900 | AUGAACUU CUGAUGA X GAA AAAGCAUU | AAUGCUUUC AAGUUCAU |
| 3905 | GGCUCAUG CUGAUGA X GAA ACUUGAAA | UUUCAAGUU CAUGAGCC |
| 3906 | AGGCUCAU CUGAUGA X GAA AACUUGAA | UUCAAGUUC AUGAGCCU |
| 3924 | AAGGUUUU CUGAUGA X GAA AUUCUUUC | GAAAGAAUC AAAACCUU |
| 3932 | GUUCUUCA CUGAUGA X GAA AGGUUUUG | CAAAACCUU UGAAGAAC |
| 3933 | AGUUCUUC CUGAUGA X GAA AAGGUUUU | AAAACCUUU GAAGAACU |
| 3942 | WCGGUAA CUGAUGA X GAA AGUUCUUC | GAAGAACUU UUACCGAA |
| 3943 | AUUCGGUA CUGAUGA X GAA AAGUUCUU | AAGAACUUU UACCGAAU |
| 3944 | CAUUCGGU CUGAUGA X GAA AAAGUUCU | AGAACUUUU ACCGAAUG |
| 3945 | GCAUUCGG CUGAUGA X GAA AAAAGUUC | GAACUUUUA CCGAAUGC |
| 3959 | CAAACAUG CUGAUGA X GAA AGGUGGCA | UGCCACCUC CAUGUUUG |
| 3965 | AGUCAUCA CUGAUGA X GAA ACAUGGAG | CUCCAUGUU UGAUGACU |
| 3966 | UAGUCAUC CUGAUGA X GAA AACAUGGA | UCCAUGUUU GAUGACUA |
| 3974 | CGCCCUGG CUGAUGA X GAA AGUCAUCA | UGAUGACUA CCAGGGCG |
| 3994 | GGCCAACA CUGAUGA X GAA AGUGCUGC | GCAGCACUC UGUUGGCC |
| 3998 | GAGAGGCC CUGAUGA X GAA ACAGAGUG | CACUCUGUU GGCCUCUC |
| 4004 | GCAUGGGA CUGAUGA X GAA AGGCCAAC | GUUGGCCUC UCCCAUGC |
| 4006 | CAGCAUGG CUGAUGA X GAA AGAGGCCA | UGGCCUCUC CCAUGCUG |
| 4022 | UCCAGGUG CUGAUGA X GAA AGCGCUUC | GAAGCGCUU CACCUGGA |
| 4023 | GUCCAGGU CUGAUGA X GAA AAGCGCUU | AAGCGCUUC ACCUGGAC |
| 4052 | UCUUGAGC CUGAUGA X GAA AGGCCUUG | CAAGGCCUC GCUCAAGA |
| 4056 | UCAAUCUU CUGAUGA X GAA AGCGAGGC | GCCUCGCUC AAGAUUGA |

(continued)

| nt. Position | HH Ribozyme | Substrate |
|---|---|---|
| 4062 | CUCAAGUC CUGAUGA X GAA AUCUUGAG | CUCAAGAUU GACUUGAG |
| 4067 | UUACUCUC CUGAUGA X GAA AGUCAAUC | GAUUGACUU GAGAGUAA |
| 4074 | UUACUGGU CUGAUGA X GAA ACUCUCAA | UUGAGAGUA ACCAGUAA |
| 4081 | CUUACUUU CUGAUGA X GAA ACUGGUUA | UAACCAGUA AAAGUAAG |
| 4087 | CGACUCCU CUGAUGA X GAA ACUUUUAC | GUAAAAGUA AGGAGUCG |
| 4094 | ACAGCCCC CUGAUGA X GAA ACUCCUUA | UAAGGAGUC GGGGCUGU |
| 4103 | UGACAUCA CUGAUGA X GAA ACAGCCCC | GGGGCUGUC UGAUGUCA |
| 4110 | GGCCUGCU CUGAUGA X GAA ACAUCAGA | UCUGAUGUC AGCAGGCC |
| 4123 | AUGGCAGA CUGAUGA X GAA ACUGGGCC | GGCCCAGUU UCUGCCAU |
| 4124 | AAUGGCAG CUGAUGA X GAA AACUGGGC | GCCCAGUUU CUGCCAUU |
| 4125 | GAAUGGCA CUGAUGA X GAA AAACUGGG | CCCAGUUUC UGCCAUUC |
| 4132 | ACAGCUGG CUGAUGA X GAA AUGGCAGA | UCUGCCAUU CCAGCUGU |
| 4133 | CACAGCUG CUGAUGA X GAA AAUGGCAG | CUGCCAUUC CAGCUGUG |
| 4149 | CCUUCGCU CUGAUGA X GAA ACGUGCCC | GGGCACGUC AGCGAAGG |
| 4169 | CGUAGGUG CUGAUGA X GAA ACCUGCGC | GCGCAGGUU CACCUACG |
| 4170 | UCGUAGGU CUGAUGA X GAA AACCUGCG | CGCAGGWC ACCUACGA |
| 4175 | CGUGGUCG CUGAUGA X GAA AGGUGAAC | GWCACCUA CGACCACG |
| 4203 | CAGCACGC CUGAUGA X GAA AUUWCCU | AGGAAAAUC GCGUGCUG |
| 4214 | GGGGCGGG CUGAUGA X GAA AGCAGCAC | GUGCUGCUC CCCGCCCC |
| 4229 | CCGAGUUG CUGAUGA X GAA AGUCUGGG | CCCAGACUA CAACUCGG |
| 4235 | GGACCACC CUGAUGA X GAA AGWGUAG | CUACAACUC GGUGGUCC |
| 4242 | GAGUACAG CUGAUGA X GAA ACCACCGA | UCGGUGGUC CUGUACUC |
| 4247 | GGGUGGAG CUGAUGA X GAA ACAGGACC | GGUCCUGUA CUCCACCC |
| 4250 | GUGGGGUG CUGAUGA X GAA AGUACAGG | CCUGUACUC CACCCCAC |
| 4263 | AAACUCUA CUGAUGA X GAA AUGGGUGG | CCACCCAUC UAGAGUUU |
| 4265 | UCAAACUC CUGAUGA X GAA AGAUGGGU | ACCCAUCUA GAGWUGA |
| 4270 | UCGUGUCA CUGAUGA X GAA ACUCUAGA | UCUAGAGUU UGACACGA |
| 4271 | UUCGUGUC CUGAUGA X GAA AACUCUAG | CUAGAGUUU GACACGAA |
| 4284 | CUAGAAAU CUGAUGA X GAA AGGCUUCG | CGAAGCCW AUWCUAG |
| 4285 | UCUAGAAA CUGAUGA X GAA AAGGCWC | GAAGCCWA UWCUAGA |
| 4287 | CUUCUAGA CUGAUGA X GAA AUAAGGCU | AGCCUUAUU UCUAGAAG |
| 4288 | GCWCUAG CUGAUGA X GAA AAUAAGGC | GCCUUAUUU CUAGAAGC |
| 4289 | UGCWCUA CUGAUGA X GAA AAAUAAGG | CCWAWUC UAGAAGCA |
| 4291 | UGUGCWC CUGAUGA X GAA AGAAAUAA | WAUWCUA GAAGCACA |
| 4305 | GGUAUAAA CUGAUGA X GAA ACACAUGU | ACAUGUGUA UUUAUACC |
| 4307 | GGGGUAUA CUGAUGA X GAA AUACACAU | AUGUGUAUU UAUACCCC |
| 4308 | GGGGGUAU CUGAUGA X GAA AAUACACA | UGUGUAUUU AUACCCCC |
| 4309 | UGGGGGUA CUGAUGA X GAA AAAUACAC | GUGUAUUUA UACCCCCA |
| 4311 | CCUGGGGG CUGAUGA X GAA AUAAAUAC | GUAUWAUA CCCCCAGG |
| 4325 | GCAAAAGC CUGAUGA X GAA AGUUUCCU | AGGAAACUA GCUUUUGC |
| 4329 | ACUGGCAA CUGAUGA X GAA AGCUAGUU | AACUAGCUU UUGCCAGU |
| 4330 | UACUGGCA CUGAUGA X GAA AAGCUAGU | ACUAGCUUU UGCCAGUA |
| 4331 | AUACUGGC CUGAUGA X GAA AAAGCUAG | CUAGCUUUU GCCAGUAU |
| 4338 | AUGCAUAA CUGAUGA X GAA ACUGGCAA | UUGCCAGUA UUAUGCAU |
| 4340 | AUAUGCAU CUGAUGA X GAA AUACUGGC | GCCAGUAUU AUGCAUAU |
| 4341 | UAUAUGCA CUGAUGA X GAA AAUACUGG | CCAGUAUUA UGCAUAUA |
| 4347 | AACUUAUA CUGAUGA X GAA AUGCAUAA | UUAUGCAUA UAUAAGUU |
| 4349 | UAAACUUA CUGAUGA X GAA AUAUGCAU | AUGCAUAUA UAAGUUUA |
| 4351 | UGUAAACU CUGAUGA X GAA AUAUAUGC | GCAUAUAUA AGUUUACA |
| 4355 | AAGGUGUA CUGAUGA X GAA ACUUAUAU | AUAUAAGW UACACCUU |

(continued)

| nt. Position | HH Ribozyme | Substrate |
|---|---|---|
| 4356 | AAAGGUGU CUGAUGA X GAA AACUUAUA | UAUAAGUUU ACACCUUU |
| 4357 | UAAAGGUG CUGAUGA X GAA AAACUUAU | AUAAGUUUA CACCUUUA |
| 4363 | GAAAGAUA CUGAUGA X GAA AGGUGUAA | UUACACCUU UAUCUUUC |
| 4364 | GGAAAGAU CUGAUGA X GAA AAGGUGUA | UACACCUUU AUCUUUCC |
| 4365 | UGGAAAGA CUGAUGA X GAA AAAGGUGU | ACACCUUUA UCCUUCCA |
| 4367 | CAUGGAAA CUGAUGA X GAA AUAAAGGU | ACCUUUAUC UWCCAUG |
| 4369 | CCCAUGGA CUGAUGA X GAA AGAUAAAG | CUUUAUCUU UCCAUGGG |
| 4370 | UCCCAUGG CUGAUGA X GAA AAGAUAAA | UUUAUCUUU CCAUGGGA |
| 4371 | CUCCCAUG CUGAUGA X GAA AAAGAUAA | UUAUCUUUC CAUGGGAG |
| 4389 | AUCACAAA CUGAUGA X GAA AGCAGCUG | CAGCUGCUU UUUGUGAU |
| 4390 | AAUCACAA CUGAUGA X GAA AAGCAGCU | AGCUGCUUU UUGUGAUU |
| 4391 | AAAUCACA CUGAUGA X GAA AAAGCAGC | GCUGCUUUU UGUGAUUU |
| 4392 | AAAAUCAC CUGAUGA X GAA AAAAGCAG | CUGCUUUUU GUGAUUUU |
| 4398 | AWAAAAA CUGAUGA X GAA AUCACAAA | UUUGUGAUU UUUUUAAU |
| 4399 | UAUUAAAA CUGAUGA X GAA AAUCACAA | UUGUGAUUU UUUUAAUA |
| 4400 | CUAUUAAA CUGAUGA X GAA AAAUCACA | GUGAUUUU UUUAAUAG |
| 4401 | ACUAUUAA CUGAUGA X GAA AAAAUCAC | GUGAUUUUU UUAAUAGU |
| 4402 | CACUAUUA CUGAUGA X GAA AAAAAUCA | UGAUUUUUU UAAUAGUG |
| 4403 | GCACUAUU CUGAUGA X GAA AAAAAAUC | GAUUUUUUU AAUAGUGC |
| 4404 | AGCACUAU CUGAUGA X GAA AAAAAAAU | AUUUUUUUA AUAGUGCU |
| 4407 | AAAAGCAC CUGAUGA X GAA AWAAAAA | UUUWAAUA GUGCUUUU |
| 4413 | AAAAAAAA CUGAUGA X GAA AGCACUAU | AUAGUGCUU UUUUUUUU |
| 4414 | AAAAAAAA CUGAUGA X GAA AAGCACUA | UAGUGCUUU UUUUUUUU |
| 4415 | CAAAAAAA CUGAUGA X GAA AAAGCACU | AGUGCUUUU UUUUUUUG |
| 4416 | UCAAAAAA CUGAUGA X GAA AAAAGCAC | GUGCUUUUU UUUUUUGA |
| 4417 | GUCAAAAA CUGAUGA X GAA AAAAAGCA | UGCUUUUUU UUUUUGAC |
| 4418 | AGUCAAAA CUGAUGA X GAA AAAAAAGC | GCUUUUUUU UUUUGACU |
| 4419 | UAGUCAAA CUGAUGA X GAA AAAAAAAG | CUUUUUUUU UUUGACUA |
| 4420 | UUAGUCAA CUGAUGA X GAA AAAAAAAA | UUUUUUUUU UUGACUAA |
| 4421 | GUUAGUCA CUGAUGA X GAA AAAAAAAA | UUUUUUUUU UGACUAAC |
| 4422 | UGUUAGUC CUGAUGA X GAA AAAAAAAA | UUUUUUUUU GACUAACA |
| 4427 | AUUCUUGU CUGAUGA X GAA AGUCAAAA | UUUUGACUA ACAAGAAU |
| 4438 | UCUGGAGU CUGAUGA X GAA ACAUUCUU | AAGAAUGUA ACUCCAGA |
| 4442 | UCUAUCUG CUGAUGA X GAA AGUUACAU | AUGUAACUC CAGAUAGA |
| 4448 | UAUUUCUC CUGAUGA X GAA AUCUGGAG | CUCCAGAUA GAGAAAUA |
| 4456 | CUUGUCAC CUGAUGA X GAA AUUUCUCU | AGAGAAAUA GUGACAAG |
| 4476 | UUUAGCAG CUGAUGA X GAA AGUGUUCU | AGAACACUA CUGCUAAA |
| 4482 | UGAGGAUU CUGAUGA X GAA AGCAGUAG | CUACUGCUA AAUCCUCA |
| 4486 | AACAUGAG CUGAUGA X GAA AUUUAGCA | UGCUAAAUC CUCAUGUU |
| 4489 | AGUAACAU CUGAUGA X GAA AGGAUUUA | UAAAUCCUC AUGUUACU |
| 4494 | CACUGAGU CUGAUGA X GAA ACAUGAGG | CCUCAUGUU ACUCAGUG |
| 4495 | ACACUGAG CUGAUGA X GAA AACAUGAG | CUCAUGUUA CUCAGUGU |
| 4498 | CUAACACU CUGAUGA X GAA AGUAACAU | AUGUUACUC AGUGUUAG |
| 4504 | AUUUCUCU CUGAUGA X GAA ACACUGAG | CUCAGUGUU AGAGAAAU |
| 4505 | GAUUUCUC CUGAUGA X GAA AACACUGA | UCAGUGUUA GAGAAAUC |
| 4513 | UUAGGAAG CUGAUGA X GAA AUUUCUCU | AGAGAAAUC CUUCCUAA |
| 4516 | GGUUUAGG CUGAUGA X GAA AGGAUUUC | GAAAUCCUU CCUAAACC |
| 4517 | GGGUUUAG CUGAUGA X GAA AAGGAUUU | AAAUCCUUC CUAAACCC |
| 4520 | AUUGGGUU CUGAUGA X GAA AGGAAGGA | UCCUUCCUA AACCCAAU |
| 4533 | GAGCAGGG CUGAUGA X GAA AGUCAUUG | CAAUGACUU CCCUGCUC |

(continued)

| nt. Position | HH Ribozyme | Substrate |
|---|---|---|
| 4534 | GGAGCAGG CUGAUGA X GAA AAGUCAUU | AAUGACUUC CCUGCUCC |
| 4541 | GGGGGUUG CUGAUGA X GAA AGCAGGGA | UCCCUGCUC CAACCCCC |
| 4557 | CGUGCCCU CUGAUGA X GAA AGGUGGCG | CGCCACCUC AGGGCACG |
| 4576 | CUCAAUCA CUGAUGA X GAA ACUGGUCC | GGACCAGUU UGAUUGAG |
| 4577 | CCUCAAUC CUGAUGA X GAA AACUGGUC | GACCAGUUU GAUUGAGG |
| 4581 | AGCUCCUC CUGAUGA X GAA AUCAAACU | AGUUUGAUU GAGGAGCU |
| 4598 | CAUUGGGU CUGAUGA X GAA AUCAGUGC | GCACUGAUC ACCCAAUG |
| 4610 | GGGUACGU CUGAUGA X GAA AUGCAUUG | CAAUCCAUC ACGUACCC |
| 4615 | CAGUGGGG CUGAUGA X GAA ACGUGAUG | CAUCACGUA CCCCACUG |
| 4664 | CUGGGGCU CUGAUGA X GAA ACGGGCUU | AAGCCCGUU AGCCCCAG |
| 4665 | CCUGGGGC CUGAUGA X GAA AACGGGCU | AGCCCGUUA GCCCCAGG |
| 4678 | CAGCCAGU CUGAUGA X GAA AUCCCCUG | CAGGGGAUC ACUGGCUG |
| 4700 | ACUCCCGA CUGAUGA X GAA AUGUUGCU | AGCAACAUC UCGGGAGU |
| 4702 | GGACUCCC CUGAUGA X GAA AGAUGUUG | CAACAUCUC GGGAGUCC |
| 4709 | UGCUAGAG CUGAUGA X GAA ACUCCCGA | UCGGGAGUC CUCUAGCA |
| 4712 | GCCUGCUA CUGAUGA X GAA AGGACUCC | GGAGUCCUC UAGCAGGC |
| 4714 | AGGCCUGC CUGAUGA X GAA AGAGGACU | AGUCCUCUA GCAGGCCU |
| 4723 | ACAUGUCU CUGAUGA X GAA AGGCCUGC | GCAGGCCUA AGACAUGU |
| 4802 | GCGUCUCA CUGAUGA X GAA AUUCUUUC | GAAAGAAUU UGAGACGC |
| 4803 | UGCGUCUC CUGAUGA X GAA AAUUCUUU | AAAGAAUUU GAGACGCA |
| 4840 | GCAUUGCU CUGAUGA X GAA AGCCCCGU | ACGGGGCUC AGCAAUGC |
| 4852 | GCCACUGA CUGAUGA X GAA AUGGCAUU | AAUGCCAUU UCAGUGGC |
| 4853 | AGCCACUG CUGAUGA X GAA AAUGGCAU | AUGCCAUUU CAGUGGCU |
| 4854 | AAGCCACU CUGAUGA X GAA AAAUGGCA | UGCCAUUUC AGUGGCUU |
| 4862 | GAGCUGGG CUGAUGA X GAA AGCCACUG | CAGUGGCUU CCCAGCUC |
| 4863 | AGAGCUGG CUGAUGA X GAA AAGCCACU | AGUGGCUUC CCAGCUCU |
| 4870 | AAGGGUCA CUGAUGA X GAA AGCUGGGA | UCCCAGCUC UGACCCUU |
| 4878 | AAAUGUAG CUGAUGA X GAA AGGGUCAG | CUGACCCUU CUACAUUU |
| 4879 | CAAAUGUA CUGAUGA X GAA AAGGGUCA | UGACCCUUC UACAUUUG |
| 4881 | CUCAAAUG CUGAUGA X GAA AGAAGGGU | ACCCUUCUA CAUUUGAG |
| 4885 | GGCCCUCA CUGAUGA X GAA AUGUAGAA | UUCUACAUU UGAGGGCC |
| 4886 | GGGCCCUC CUGAUGA X GAA AAUGUAGA | UCUACAUUU GAGGGCCC |
| 4929 | AUCCAGAA CUGAUGA X GAA AUGUCCCC | GGGGACAUU UUCUGGAU |
| 4930 | AAUCCAGA CUGAUGA X GAA AAUGUCCC | GGGACAUUU UCUGGAUU |
| 4931 | GAAUCCAG CUGAUGA X GAA AAAUGUCC | GGACAUUUU CUGGAUUC |
| 4932 | AGAAUCCA CUGAUGA X GAA AAAAUGUC | GACAUUUUC UGGAUUCU |
| 4938 | CCUCCCAG CUGAUGA X GAA AUCCAGAA | UUCUGGAUU CUGGGAGG |
| 4939 | GCCUCCCA CUGAUGA X GAA AAUCCAGA | UCUGGAUUC UGGGAGGC |
| 4963 | AAAAAAGA CUGAUGA X GAA AUUUGUCC | GGACAAAUA UCUUUUUU |
| 4965 | CCAAAAAA CUGAUGA X GAA AUAUUUGU | ACAAAUAUC UUUUUUGG |
| 4967 | UUCCAAAA CUGAUGA X GAA AGAUAUUU | AAAUAUCUU UUUUGGAA |
| 4968 | GUUCCAAA CUGAUGA X GAA AAGAUAUU | AAUAUCUUU UUUGGAAC |
| 4969 | AGUUCCAA CUGAUGA X GAA AAAGAUAU | AUAUCUUUU UUGGAACU |
| 4970 | UAGUUCCA CUGAUGA X GAA AAAAGAUA | UAUCUUUUU UGGAACUA |
| 4971 | UUAGUUCC CUGAUGA X GAA AAAAAGAU | AUCUUUUUU GGAACUAA |
| 4978 | AUUUGCUU CUGAUGA X GAA AGUUCCAA | UUGGAACUA AAGCAAAU |
| 4987 | AGGUCUAA CUGAUGA X GAA AUUUGCUU | AAGCAAAUU UUAGACCU |
| 4988 | AAGGUCUA CUGAUGA X GAA AAUUUGCU | AGCAAAUUU UAGACCUU |
| 4989 | AAAGGUCU CUGAUGA X GAA AAAUUUGC | GCAAAUUUU AGACCUUU |
| 4990 | UAAAGGUC CUGAUGA X GAA AAAAUUUG | CAAAUUUUA GACCUUUA |

(continued)

| nt. Position | HH Ribozyme | Substrate |
|---|---|---|
| 4996 | CAUAGGUA CUGAUGA X GAA AGGUCUAA | UUAGACCUU UACCUAUG |
| 4997 | CCAUAGGU CUGAUGA X GAA AAGGUCUA | UAGACCUUU ACCUAUGG |
| 4998 | UCCAUAGG CUGAUGA X GAA AAAGGUCU | AGACCUUUA CCUAUGGA |
| 5002 | CACUUCCA CUGAUGA X GAA AGGUAAAG | CUUUACCUA UGGAAGUG |
| 5013 | GGACAUAG CUGAUGA X GAA ACCACUUC | GAAGUGGUU CUAUGUCC |
| 5014 | UGGACAUA CUGAUGA X GAA AACCACUU | AAGUGGUUC UAUGUCCA |
| 5016 | AAUGGACA CUGAUGA X GAA AGAACCAC | GUGGUUCUA UGUCCAUU |
| 5020 | UGAGAAUG CUGAUGA X GAA ACAUAGAA | UUCUAUGUC CAUUCUCA |
| 5024 | CGAAUGAG CUGAUGA X GAA AUGGACAU | AUGUCCAUU CUCAUUCG |
| 5025 | ACGAAUGA CUGAUGA X GAA AAUGGACA | UGUCCAUUC UCAUUCGU |
| 5027 | CCACGAAU CUGAUGA X GAA AGAAUGGA | UCCAUUCUC AUUCGUGG |
| 5030 | AUGCCACG CUGAUGA X GAA AUGAGAAU | AUUCUCAUU CGUGGCAU |
| 5031 | CAUGCCAC CUGAUGA X GAA AAUGAGAA | UUCUCAUUC GUGGCAUG |
| 5041 | CAAAUCAA CUGAUGA X GAA ACAUGCCA | UGGCAUGUU UUGAUUUG |
| 5042 | ACAAAUCA CUGAUGA X GAA AACAUGCC | GGCAUGUUU UGAUUUGU |
| 5043 | UACAAAUC CUGAUGA X GAA AAACAUGC | GCAUGUUUU GAUUUGUA |
| 5047 | GUGCUACA CUGAUGA X GAA AUCAAAAC | GUUUUGAUU UGUAGCAC |
| 5048 | AGUGCUAC CUGAUGA X GAA AAUCAAAA | UUUUGAUUU GUAGCACU |
| 5051 | CUCAGUGC CUGAUGA X GAA ACAAAUCA | UGAUUUGUA GCACUGAG |
| 5069 | UCAGAGUU CUGAUGA X GAA AGUGCCAC | GUGGCACUC AACUCUGA |
| 5074 | UGGGCUCA CUGAUGA X GAA AGUUGAGU | ACUCAACUC UGAGCCCA |
| 5084 | GCCAAAAG CUGAUGA X GAA AUGGGCUC | GAGCCCAUA CUUUUGGC |
| 5087 | GGAGCCAA CUGAUGA X GAA AGUAUGGG | CCCAUACUU UUGGCUCC |
| 5088 | AGGAGCCA CUGAUGA X GAA AAGUAUGG | CCAUACUUU UGGCUCCU |
| 5089 | GAGGAGCC CUGAUGA X GAA AAAGUAUG | CAUACUUUU GGCUCCUC |
| 5094 | UACUAGAG CUGAUGA X GAA AGCCAAAA | UUUUGGCUC CUCUAGUA |
| 5097 | UCUUACUA CUGAUGA X GAA AGGAGCCA | UGGCUCCUC UAGUAAGA |
| 5099 | CAUCUUAC CUGAUGA X GAA AGAGGAGC | GCUCCUCUA GUAAGAUG |
| 5102 | GUGCAUCU CUGAUGA X GAA ACUAGAGG | CCUCUAGUA AGAUGCAC |
| 5119 | CUCUGGCU CUGAUGA X GAA AGUUUUCA | UGAAAACUU AGCCAGAG |
| 5120 | ACUCUGGC CUGAUGA X GAA AAGUUUUC | GAAAACUUA GCCAGAGU |
| 5129 | GACAACCU CUGAUGA X GAA ACUCUGGC | GCCAGAGUU AGGUUGUC |
| 5130 | AGACAACC CUGAUGA X GAA AACUCUGG | CCAGAGUUA GGUUGUCU |
| 5134 | CUGGAGAC CUGAUGA X GAA ACCUAACU | AGUUAGGUU GUCUCCAG |
| 5137 | GGCCUGGA CUGAUGA X GAA ACAACCUA | UAGGUUGUC UCCAGGCC |
| 5139 | AUGGCCUG CUGAUGA X GAA AGACAACC | GGUUGUCUC CAGGCCAU |
| 5156 | UUCAGUGU CUGAUGA X GAA AGGCCAUC | GAUGGCCUU ACACUGAA |
| 5157 | UUUCAGUG CUGAUGA X GAA AAGGCCAU | AUGGCCUUA CACUGAAA |
| 5170 | UAGAAUGU CUGAUGA X GAA ACAUUUUC | GAAAAUGUC ACAUUCUA |
| 5175 | CAAAAUAG CUGAUGA X GAA AUGUGACA | UGUCACAUU CUAUUUUG |
| 5176 | CCAAAAUA CUGAUGA X GAA AAUGUGAC | GUCACAUUC UAUUUUGG |
| 5178 | ACCCAAAA CUGAUGA X GAA AGAAUGUG | CACAUUCUA UUUUGGGU |
| 5180 | AUACCCAA CUGAUGA X GAA AUAGAAUG | CAUUCUAUU UUGGGUAU |
| 5181 | AAUACCCA CUGAUGA X GAA AAUAGAAU | AUUCUAUUU UGGGUAUU |
| 5182 | UAAUACCC CUGAUGA X GAA AAAUAGAA | UUCUAUUUU GGGUAUUA |
| 5187 | UAUAUUAA CUGAUGA X GAA ACCCAAAA | UUUUGGGUA UUAAUAUA |
| 5189 | UAUAUAUU CUGAUGA X GAA AUACCCAA | UUGGGUAUU AAUAUAUA |
| 5190 | CUAUAUAU CUGAUGA X GAA AAUACCCA | UGGGUAUUA AUAUAUAG |
| 5193 | GGACUAUA CUGAUGA X GAA AUUAAUAC | GUAUUAAUA UAUAGUCC |
| 5195 | CUGGACUA CUGAUGA X GAA AUAUUAAU | AUUAAUAUA UAGUCCAG |

(continued)

| nt. Position | HH Ribozyme | Substrate |
|---|---|---|
| 5197 | GUCUGGAC CUGAUGA X GAA AUAUAUUA | UAAUAUAUA GUCCAGAC |
| 5200 | AGUGUCUG CUGAUGA X GAA ACUAUAUA | UAUAUAGUC CAGACACU |
| 5209 | AUUGAGUU CUGAUGA X GAA AGUGUCUG | CAGACACUU AACUCAAU |
| 5210 | AAUUGAGU CUGAUGA X GAA AAGUGUCU | AGACACUUA ACUCAAUU |
| 5214 | AAGAAAUU CUGAUGA X GAA AGUUAAGU | ACUUAACUC AAUUUCUU |
| 5218 | UACCAAGA CUGAUGA X GAA AUUGAGUU | AACUCAAUU UCUUGGUA |
| 5219 | AUACCAAG CUGAUGA X GAA AAUUGAGU | ACUCAAUUU CUUGGUAU |
| 5220 | AAUACCAA CUGAUGA X GAA AAAUUGAG | CUCAAUUUC UUGGUAUU |
| 5222 | AUAAUACC CUGAUGA X GAA AGAAAUUG | CAAUUUCUU GGUAUUAU |
| 5226 | CAGAAUAA CUGAUGA X GAA ACCAAGAA | UUCUUGGUA UUAUUCUG |
| 5228 | AACAGAAU CUGAUGA X GAA AUACCAAG | CUUGGUAUU AUUCUGUU |
| 5229 | AAACAGAA CUGAUGA X GAA AAUACCAA | UUGGUAUUA UUCUGUUU |
| 5231 | CAAAACAG CUGAUGA X GAA AUAAUACC | GGUAUUAUU CUGUUUUG |
| 5232 | GCAAAACA CUGAUGA X GAA AAUAAUAC | GUAUUAUUC UGUUUUGC |
| 5236 | CUGUGCAA CUGAUGA X GAA ACAGAAUA | UAUUCUGUU UUGCACAG |
| 5237 | ACUGUGCA CUGAUGA X GAA AACAGAAU | AUUCUGUUU UGCACAGU |
| 5238 | AACUGUGC CUGAUGA X GAA AAACAGAA | UUCUGUUUU GCACAGUU |
| 5246 | UCACAACU CUGAUGA X GAA ACUGUGCA | UGCACAGUU AGUUGUGA |
| 5247 | UUCACAAC CUGAUGA X GAA AACUGUGC | GCACAGUUA GUUGUGAA |
| 5250 | UCUUUCAC CUGAUGA X GAA ACUAACUG | CAGUUAGUU GUGAAAGA |
| 5284 | CUCCUCAG CUGAUGA X GAA ACUGCAUU | AAUGCAGUC CUGAGGAG |
| 5296 | AUGGAGAA CUGAUGA X GAA ACUCUCCU | AGGAGAGUU UUCUCCAU |
| 5297 | UAUGGAGA CUGAUGA X GAA AACUCUCC | GGAGAGUUU UCUCCAUA |
| 5298 | AUAUGGAG CUGAUGA X GAA AAACUCUC | GAGAGUUUU CUCCAUAU |
| 5299 | GAUAUGGA CUGAUGA X GAA AAAACUCU | AGAGUUUUC UCCAUAUC |
| 5301 | UUGAUAUG CUGAUGA X GAA AGAAAACU | AGUUUUCUC CAUAUCAA |
| 5305 | CGUUUUGA CUGAUGA X GAA AUGGAGAA | UUCUCCAUA UCAAAACG |
| 5307 | CUCGUUUU CUGAUGA X GAA AUAUGGAG | CUCCAUAUC AAAACGAG |
| 5336 | ACCUUAUU CUGAUGA X GAA ACCUUUUU | AAAAAGGUC AAUAAGGU |
| 5340 | CUUGACCU CUGAUGA X GAA AUUGACCU | AGGUCAAUA AGGUCAAG |
| 5345 | CUUCCCUU CUGAUGA X GAA ACCUUAUU | AAUAAGGUC AAGGGAAG |
| 5361 | GGUAUAGA CUGAUGA X GAA ACGGGGUC | GACCCCGUC UCUAUACC |
| 5363 | UUGGUAUA CUGAUGA X GAA AGACGGGG | CCCCGUCUC UAUACCAA |
| 5365 | GGUUGGUA CUGAUGA X GAA AGAGACGG | CCGUCUCUA UACCAACC |
| 5367 | UUGGUUGG CUGAUGA X GAA AUAGAGAC | GUCUCUAUA CCAACCAA |
| 5382 | UGUUGGUG CUGAUGA X GAA AUUGGUUU | AAACCAAUU CACCAACA |
| 5383 | GUGUUGGU CUGAUGA X GAA AAUUGGUU | AACCAAUUC ACCAACAC |
| 5395 | UGGGUCCC CUGAUGA X GAA ACUGUGUU | AACACAGUU GGGACCCA |
| 5417 | ACGUGACU CUGAUGA X GAA ACUUCCUG | CAGGAAGUC AGUCACGU |
| 5421 | GGAAACGU CUGAUGA X GAA ACUGACUU | AAGUCAGUC ACGUUUCC |
| 5426 | GAAAAGGA CUGAUGA X GAA ACGUGACU | AGUCACGUU UCCUUUUC |
| 5427 | UGAAAAGG CUGAUGA X GAA AACGUGAC | GUCACGUUU CCUUUUCA |
| 5428 | AUGAAAAG CUGAUGA X GAA AAACGUGA | UCACGUUUC CUUUUCAU |
| 5431 | UAAAUGAA CUGAUGA X GAA AGGAAACG | CGUUUCCUU UUCAUUUA |
| 5432 | UUAAAUGA CUGAUGA X GAA AAGGAAAC | GUUUCCUUU UCAUUUAA |
| 5433 | AUUAAAUG CUGAUGA X GAA AAAGGAAA | UUUCCUUUU CAUUUAAU |
| 5434 | CAUUAAAU CUGAUGA X GAA AAAAGGAA | UUCCUUUUC AWUAAUG |
| 5437 | CCCCAUUA CUGAUGA X GAA AUGAAAAG | CUUUUCAUU UAAUGGGG |
| 5438 | UCCCCAUU CUGAUGA X GAA AAUGAAAA | UUUUCAUUU AAUGGGGA |
| 5439 | AUCCCCAU CUGAUGA X GAA AAAUGAAA | UUUCAUUUA AUGGGGAU |

(continued)

| nt. Position | HH Ribozyme | Substrate |
|---|---|---|
| 5448 | GAUAGUGG CUGAUGA X GAA AUCCCCAU | AUGGGGAUU CCACUAUC |
| 5449 | AGAUAGUG CUGAUGA X GAA AAUCCCCA | UGGGGAUUC CACUAUCU |
| 5454 | GUGUGAGA CUGAUGA X GAA AGUGGAAU | AUUCCACUA UCUCACAC |
| 5456 | UAGUGUGA CUGAUGA X GAA AUAGUGGA | UCCACUAUC UCACACUA |
| 5458 | AUUAGUGU CUGAUGA X GAA AGAUAGUG | CACUAUCUC ACACUAAU |
| 5464 | UUUCAGAU CUGAUGA X GAA AGUGUGAG | CUCACACUA AUCUGAAA |
| 5467 | UCCUUUCA CUGAUGA X GAA AUUAGUGU | ACACUAAUC UGAAAGGA |
| 5489 | CGCCAGCU CUGAUGA X GAA AUGCUCUU | AAGAGCAUU AGCUGGCG |
| 5490 | GCGCCAGC CUGAUGA X GAA AAUGCUCU | AGAGCAUUA GCUGGCGC |
| 5501 | GUGCUUAA CUGAUGA X GAA AUGCGCCA | UGGCGCAUA UUAAGCAC |
| 5503 | AAGUGCUU CUGAUGA X GAA AUAUGCGC | GCGCAUAUU AAGCACUU |
| 5504 | AAAGUGCU CUGAUGA X GAA AAUAUGCG | CGCAUAUUA AGCACUUU |
| 5511 | GGAGCUUA CUGAUGA X GAA AGUGCUUA | UAAGCACUU UAAGCUCC |
| 5512 | AGGAGCUU CUGAUGA X GAA AAGUGCUU | AAGCACUUU AAGCUCCU |
| 5513 | AAGGAGCU CUGAUGA X GAA AAAGUGCU | AGCACUUUA AGCUCCUU |
| 5518 | UACUCAAG CUGAUGA X GAA AGCWAAA | UUUAAGCUC CUUGAGUA |
| 5521 | UUUUACUC CUGAUGA X GAA AGGAGCUU | AAGCUCCUU GAGUAAAA |
| 5526 | CACCUUUU CUGAUGA X GAA ACUCAAGG | CCUUGAGUA AAAAGGUG |
| 5537 | AAAUUACA CUGAUGA X GAA ACCACCUU | AAGGUGGUA UGUAAUUU |
| 5541 | GCAUAAAU CUGAUGA X GAA ACAUACCA | UGGUAUGUA AUUUAUGC |
| 5544 | CUUGCAUA CUGAUGA X GAA AUUACAUA | UAUGUAAUU UAUGCAAG |
| 5545 | CCUUGCAU CUGAUGA X GAA AAUUACAU | AUGUAAUUU AUGCAAGG |
| 5546 | ACCUUGCA CUGAUGA X GAA AAAUUACA | UGUAAUUUA UGCAAGGU |
| 5555 | UGGAGAAA CUGAUGA X GAA ACCUUGCA | UGCAAGGUA UUUCUCCA |
| 5557 | ACUGGAGA CUGAUGA X GAA AUACCUUG | CAAGGUAUU UCUCCAGU |
| 5558 | AACUGGAG CUGAUGA X GAA AAUACCUU | AAGGUAUUU CUCCAGUU |
| 5559 | CAACUGGA CUGAUGA X GAA AAAUACCU | AGGUAUUUC UCCAGUUG |
| 5561 | CCCAACUG CUGAUGA X GAA AGAAAUAC | GUAUUUCUC CAGUUGGG |
| 5566 | UGAGUCCC CUGAUGA X GAA ACUGGAGA | UCUCCAGUU GGGACUCA |
| 5573 | AAUAUCCU CUGAUGA X GAA AGUCCCAA | UUGGGACUC AGGAUAUU |
| 5579 | UUAACUAA CUGAUGA X GAA AUCCUGAG | CUCAGGAUA UUAGUUAA |
| 5581 | CAUUAACU CUGAUGA X GAA AUAUCCUG | CAGGAUAUU AGUUAAUG |
| 5582 | UCAUUAAC CUGAUGA X GAA AAUAUCCU | AGGAUAUUA GUUAAUGA |
| 5585 | GGCUCAUU CUGAUGA X GAA ACUAAUAU | AUAUUAGUU AAUGAGCC |
| 5586 | UGGCUCAU CUGAUGA X GAA AACUAAUA | UAUUAGUUA AUGAGCCA |
| 5596 | CUUCUAGU CUGAUGA X GAA AUGGCUCA | UGAGCCAUC ACUAGAAG |
| 5600 | UUUUCUUC CUGAUGA X GAA AGUGAUGG | CCAUCACUA GAAGAAAA |
| 5615 | CAGUUGAA CUGAUGA X GAA AUGGGCUU | AAGCCCAUU UUCAACUG |
| 5616 | GCAGUUGA CUGAUGA X GAA AAUGGGCU | AGCCCAUUU UCAACUGC |
| 5617 | AGCAGUUG CUGAUGA X GAA AAAUGGGC | GCCCAUUUU CAACUGCU |
| 5618 | AAGCAGUU CUGAUGA X GAA AAAAUGGG | CCCAUUUUC AACUGCUU |
| 5626 | AAGUUUCA CUGAUGA X GAA AGCAGUUG | CAACUGCUU UGAAACUU |
| 5627 | CAAGUUUC CUGAUGA X GAA AAGCAGUU | AACUGCUUU GAAACUUG |
| 5634 | CCCCAGGC CUGAUGA X GAA AGUUUCAA | UUGAAACUU GCCUGGGG |
| 5644 | CAUGCUCA CUGAUGA X GAA ACCCCAGG | CCUGGGGUC UGAGCAUG |
| 5661 | UGUCUCCC CUGAUGA X GAA AUUCCCAU | AUGGGAAUA GGGAGACA |
| 5674 | CCCUUUCC CUGAUGA X GAA ACCCUGUC | GACAGGGUA GGAAAGGG |
| 5688 | CUGAAGAG CUGAUGA X GAA AGGCGCCC | GGGCGCCUA CUCUUCAG |
| 5691 | ACCCUGAA CUGAUGA X GAA AGUAGGCG | CGCCUACUC UUCAGGGU |
| 5693 | AGACCCUG CUGAUGA X GAA AGAGUAGG | CCUACUCUU CAGGGUCU |

(continued)

| nt. Position | HH Ribozyme | Substrate |
|---|---|---|
| 5694 | UAGACCCU CUGAUGA X GAA AAGAGUAG | CUACUCUUC AGGGUCUA |
| 5700 | GAUCUUUA CUGAUGA X GAA ACCCUGAA | UUCAGGGUC UAAAGAUC |
| 5702 | UUGAUCUU CUGAUGA X GAA AGACCCUG | CAGGGUCUA AAGAUCAA |
| 5708 | GCCCACUU CUGAUGA X GAA AUCUUUAG | CUAAAGAUC AAGUGGGC |
| 5719 | AGCGAUCC CUGAUGA X GAA AGGCCCAC | GUGGGCCUU GGAUCGCU |
| 5724 | AGCUUAGC CUGAUGA X GAA AUCCAAGG | CCUUGGAUC GCUAAGCU |
| 5728 | AGCCAGCU CUGAUGA X GAA AGCGAUCC | GGAUCGCUA AGCUGGCU |
| 5737 | AUCAAACA CUGAUGA X GAA AGCCAGCU | AGCUGGCUC UGUUUGAU |
| 5741 | UAGCAUCA CUGAUGA X GAA ACAGAGCC | GGCUCUGUU UGAUGCUA |
| 5742 | AUAGCAUC CUGAUGA X GAA AACAGAGC | GCUCUGUUU GAUGCUAU |
| 5749 | UGCAUAAA CUGAUGA X GAA AGCAUCAA | UGAUGCUA UUUAUGCA |
| 5751 | CUUGCAUA CUGAUGA X GAA AUAGCAUC | GAUGCUAUU UAUGCAAG |
| 5752 | ACUUGCAU CUGAUGA X GAA AAUAGCAU | AUGCUAUUU AUGCAAGU |
| 5753 | AACUUGCA CUGAUGA X GAA AAAUAGCA | UGCUAUUUA UGCAAGUU |
| 5761 | UAGACCCU CUGAUGA X GAA ACWGCAU | AUGCAAGU AGGGUCUA |
| 5762 | AUAGACCC CUGAUGA X GAA AACUUGCA | UGCAAGUUA GGGUCUAU |
| 5767 | AAUACAUA CUGAUGA X GAA ACCCUAAC | GUUAGGGUC UAUGUAUU |
| 5769 | UAAAUACA CUGAUGA X GAA AGACCCUA | UAGGGUCUA UGUAUWA |
| 5773 | AUCCUAAA CUGAUGA X GAA ACAUAGAC | GUCUAUGUA UUUAGGAU |
| 5775 | GCAUCCUA CUGAUGA X GAA AUACAUAG | CUAUGUAUU UAGGAUGC |
| 5776 | CGCAUCCU CUGAUGA X GAA AAUACAUA | UAUGUAUUU AGGAUGCG |
| 5777 | GCGCAUCC CUGAUGA X GAA AAAUACAU | AUGUAUUUA GGAUGCGC |
| 5788 | CUGAAGAG CUGAUGA X GAA AGGCGCAU | AUGCGCCUA CUCUUCAG |
| 5791 | ACCCUGAA CUGAUGA X GAA AGUAGGCG | CGCCUACUC UUCAGGGU |
| 5793 | AGACCCUG CUGAUGA X GAA AGAGUAGG | CCUACUCUU CAGGGUCU |
| 5794 | UAGACCCU CUGAUGA X GAA AAGAGUAG | CUACUCUUC AGGGUCUA |
| 5800 | GAUCUUUA CUGAUGA X GAA ACCCUGAA | UUCAGGGU UAAAGAUC |
| 5802 | UUGAUCUU CUGAUGA X GAA AGACCCUG | CAGGGUCUA AAGAUCAA |
| 5808 | GCCCACUU CUGAUGA X GAA AUCUUUAG | CUAAAGAUC AAGUGGGC |
| 5819 | AGCGAUCC CUGAUGA X GAA AGGCCCAC | GUGGGCCUU GGAUCGCU |
| 5824 | AGCUUAGC CUGAUGA X GAA AUCCAAGG | CCUUGGAUC GCUAAGCU |
| 5828 | AGCCAGCU CUGAUGA X GAA AGCGAUCC | GGAUCGCUA AGCUGGCU |
| 5837 | AUCAAACA CUGAUGA X GAA AGCCAGCU | AGCUGGCUC UGUUUGAU |
| 5841 | UAGCAUCA CUGAUGA X GAA ACAGAGCC | GGCUCUGUU UGAUGCUA |
| 5842 | AUAGCAUC CUGAUGA X GAA AACAGAGC | GCUCUGUUU GAUGCUAU |
| 5849 | UGCAUAAA CUGAUGA X GAA AGCAUCAA | UUGAUGCUA UUUAUGCA |
| 5851 | CUUGCAUA CUGAUGA X GAA AUAGCAUC | GAUGCUAUU UAUGCAAG |
| 5852 | ACUUGCAU CUGAUGA X GAA AAUAGCAU | AUGCUAUUU AUGCAAGU |
| 5853 | AACUUGCA CUGAUGA X GAA AAAUAGCA | UGCUAUUUA UGCAAGUU |
| 5861 | UAGACCCU CUGAUGA X GAA ACUUGCAU | AUGCAAGUU AGGGUCUA |
| 5862 | AUAGACCC CUGAUGA X GAA AACUUGCA | UGCAAGUUA GGGUCUAU |
| 5867 | AAUACAUA CUGAUGA X GAA ACCCUAAC | GUUAGGGUC UAUGUAUU |
| 5869 | UAAAUACA CUGAUGA X GAA AGACCCUA | UAGGGUCUA UGUAUUUA |
| 5873 | AUCCUAAA CUGAUGA X GAA ACAUAGAC | GUCUAUGUA UUUAGGAU |
| 5875 | ACAUCCUA CUGAUGA X GAA AUACAUAG | CUAUGUAUU UAGGAUGU |
| 5876 | GACAUCCU CUGAUGA X GAA AAUACAUA | UAUGUAUUU AGGAUGUC |
| 5877 | AGACAUCC CUGAUGA X GAA AAAUACAU | AUGUAUUUA GGAUGUCU |
| 5884 | AAGGUGCA CUGAUGA X GAA ACAUCCUA | UAGGAUGUC UGCACCUU |
| 5892 | GGCUGCAG CUGAUGA X GAA AGGUGCAG | CUGCACCUU CUGCAGCC |
| 5893 | UGGCUGCA CUGAUGA X GAA AAGGUGCA | UGCACCUUC UGCAGCCA |

(continued)

| nt. Position | HH Ribozyme | Substrate |
|---|---|---|
| 5904 | CAGCUUCU CUGAUGA X GAA ACUGGCUG | CAGCCAGUC AGAAGCUG |
| 5930 | GAAGCAGC CUGAUGA X GAA AUCCACUG | CAGUGGAUU GCUGCUUC |
| 5937 | UCCCCAAG CUGAUGA X GAA AGCAGCAA | UUGCUGCUU CUUGGGGA |
| 5938 | CUCCCCAA CUGAUGA X GAA AAGCAGCA | UGCUGCUUC UUGGGGAG |
| 5940 | UUCUCCCC CUGAUGA X GAA AGAAGCAG | CUGCUUCUU GGGGAGAA |
| 5953 | AGGAAGCA CUGAUGA X GAA ACUCUUCU | AGAAGAGUA UGCUUCCU |
| 5958 | AUAAAAGG CUGAUGA X GAA AGCAUACU | AGUAUGCUU CCUUUUAU |
| 5959 | GAUAAAAG CUGAUGA X GAA AAGCAUAC | GUAUGCUUC CUUUUAUC |
| 5962 | AUGGAUAA CUGAUGA X GAA AGGAAGCA | UGCUUCCUU UUAUCCAU |
| 5963 | CAUGGAUA CUGAUGA X GAA AAGGAAGC | GCUUCCUUU UAUCCAUG |
| 5964 | ACAUGGAU CUGAUGA X GAA AAAGGAAG | CUUCCUUUU AUCCAUGU |
| 5965 | UACAUGGA CUGAUGA X GAA AAAAGGAA | UUCCUUUUA UCCAUGUA |
| 5967 | AUUACAUG CUGAUGA X GAA AUAAAAGG | CCUUUUAUC CAUGUAAU |
| 5973 | AGUUAAAU CUGAUGA X GAA ACAUGGAU | AUCCAUGUA AUUUAACU |
| 5976 | UACAGUUA CUGAUGA X GAA AUUACAUG | CAUGUAAUU UAACUGUA |
| 5977 | CUACAGUU CUGAUGA X GAA AAUUACAU | AUGUAAUUU AACUGUAG |
| 5978 | UCUACAGU CUGAUGA X GAA AAAUUACA | UGUAAUUUA ACUGUAGA |
| 5984 | UCAGGUUC CUGAUGA X GAA ACAGUUAA | UUAACUGUA GAACCUGA |
| 5996 | GUUACUUA CUGAUGA X GAA AGCUCAGG | CCUGAGCUC UAAGUAAC |
| 5998 | CGGUUACU CUGAUGA X GAA AGAGCUCA | UGAGCUCUA AGUAACCG |
| 6002 | UCUUCGGU CUGAUGA X GAA ACUUAGAG | CUCUAAGUA ACCGAAGA |
| 6015 | CAGAGGCA CUGAUGA X GAA ACAUUCUU | AAGAAUGUA UGCCUCUG |
| 6021 | UAAGAACA CUGAUGA X GAA AGGCAUAC | GUAUGCCUC UGUUCUUA |
| 6025 | CACAUAAG CUGAUGA X GAA ACAGAGGC | GCCUCUGUU CUUAUGUG |
| 6026 | GCACAUAA CUGAUGA X GAA AACAGAGG | CCUCUGUUC UUAUGUGC |
| 6028 | UGGCACAU CUGAUGA X GAA AGAACAGA | UCUGUUCUU AUGUGCCA |
| 6029 | GUGGCACA CUGAUGA X GAA AAGAACAG | CUGUUCUUA UGUGCCAC |
| 6040 | UAAACAAG CUGAUGA X GAA AUGUGGCA | UGCCACAUC CUUGUUUA |
| 6043 | CUUUAAAC CUGAUGA X GAA AGGAUGUG | CACAUCCUU GUUUAAAG |
| 6046 | AGCCUUUA CUGAUGA X GAA ACAAGGAU | AUCCUUGUU UAAAGGCU |
| 6047 | GAGCCUUU CUGAUGA X GAA AACAAGGA | UCCUUGUUU AAAGGCUC |
| 6048 | AGAGCCUU CUGAUGA X GAA AAACAAGG | CCUUGUUUA AAGGCUCU |
| 6055 | CAUACAGA CUGAUGA X GAA AGCCUUUA | UAAAGGCUC UCUGUAUG |
| 6057 | UUCAUACA CUGAUGA X GAA AGAGCCUU | AAGGCUCUC UGUAUGAA |
| 6061 | UCUCUUCA CUGAUGA X GAA ACAGAGAG | CUCUCUGUA UGAAGAGA |
| 6079 | GUGCUGAU CUGAUGA X GAA ACGGUCCC | GGGACCGUC AUCAGCAC |
| 6082 | AAUGUGCU CUGAUGA X GAA AUGACGGU | ACCGUCAUC AGCACAUU |
| 6090 | CACUAGGG CUGAUGA X GAA AUGUGCUG | CAGCACAUU CCCUAGUG |
| 6091 | UCACUAGG CUGAUGA X GAA AAUGUGCU | AGCACAUUC CCUAGUGA |
| 6095 | AGGCUCAC CUGAUGA X GAA AGGGAAUG | CAUUCCCUA GUGAGCCU |
| 6104 | GGAGCCAG CUGAUGA X GAA AGGCUCAC | GUGAGCCUA CUGGCUCC |
| 6111 | GCUGCCAG CUGAUGA X GAA AGCCAGUA | UACUGGCUC CUGGCAGC |
| 6124 | UUCCACAA CUGAUGA X GAA AGCCGCUG | CAGCGGCUU UUGUGGAA |
| 6125 | CUUCCACA CUGAUGA X GAA AAGCCGCU | AGCGGCUUU UGUGGAAG |
| 6126 | UCUUCCAC CUGAUGA X GAA AAAGCCGC | GCGGCUUUU GUGGAAGA |
| 6137 | UGGCUAGU CUGAUGA X GAA AGUCUUCC | GGAAGACUC ACUAGCCA |
| 6141 | CUUCUGGC CUGAUGA X GAA AGUGAGUC | GACUCACUA GCCAGAAG |
| 6166 | GUGGAGAG CUGAUGA X GALA ACUGUCCC | GGGACAGUC CUCUCCAC |
| 6169 | UUGGUGGA CUGAUGA X GAA AGGACUGU | ACAGUCCUC UCCACCAA |
| 6171 | UCUUGGUG CUGAUGA X GAA AGAGGACU | AGUCCUCUC CACCAAGA |

(continued)

| nt. Position | HH Ribozyme | Substrate |
|---|---|---|
| 6181 | UGGAUUUA CUGAUGA X GAA AUCUUGGU | ACCAAGAUC UAAAUCCA |
| 6183 | UUUGGAUU CUGAUGA X GAA AGAUCUUG | CAAGAUCUA AAUCCAAA |
| 6187 | UUUGUUUG CUGAUGA X GAA AUUUAGAU | AUCUAAAUC CAAACAAA |
| 6204 | UCUGGCUC CUGAUGA X GAA AGCCUGCU | AGCAGGCUA GAGCCAGA |
| 6226 | ACAACAAA CUGAUGA X GAA AUUUGUCC | GGACAAAUC UUUGUUGU |
| 6228 | GAACAACA CUGAUGA X GAA AGAUUUGU | ACAAAUCUU UGUUGUUC |
| 6229 | GGAACAAC CUGAUGA X GAA AAGAUUUG | CAAAUCUUU GUUGUUCC |
| 6232 | AGAGGAAC CUGAUGA X GAA ACAAAGAU | AUCUUUGUU GUUCCUCU |
| 6235 | AGAAGAGG CUGAUGA X GAA ACAACAAA | UUUGUUGUU CCUCUUCU |
| 6236 | AAGAAGAG CUGAUGA X GAA AACAACAA | UUGUUGUUC CUCUUCUU |
| 6239 | GUAAAGAA CUGAUGA X GAA AGGAACAA | UUGUUCCUC UUCUUUAC |
| 6241 | GUGUAAAG CUGAUGA X GAA AGAGGAAC | GUUCCUCUU CUUUACAC |
| 6242 | UGUGUAAA CUGAUGA X GAA AAGAGGAA | UUCCUCUUC UUUACACA |
| 6244 | UAUGUGUA CUGAUGA X GAA AGAAGAGG | CCUCUUCUU UACACAUA |
| 6245 | GUAUGUGU CUGAUGA X GAA AAGAAGAG | CUCUUCUUU ACACAUAC |
| 6246 | CGUAUGUG CUGAUGA X GAA AAAGAAGA | UCUUCUUUA CACAUACG |
| 6252 | GGUUUGCG CUGAUGA X GAA AUGUGUAA | UUACACAUA CGCAAACC |
| 6280 | AUUUAUAA CUGAUGA X GAA AUUGCCAG | CUGGCAAUU UUAUAAAU |
| 6281 | GAUUUAUA CUGAUGA X GAA AAUUGCCA | UGGCAAUUU UAUAAAUC |
| 6282 | UGAUUUAU CUGAUGA X GAA AAAUUGCC | GGCAAUUUU AUAAAUCA |
| 6283 | CUGAUUUA CUGAUGA X GAA AAAAUUGC | GCAAUUUUA UAAAUCAG |
| 6285 | ACCUGAUU CUGAUGA X GAA AUAAAAUU | AAUUUUAUA AAUCAGGU |
| 6289 | AGUUACCU CUGAUGA X GAA AUUUAUAA | UUAUAAAUC AGGUAACU |
| 6294 | CUUCCAGU CUGAUGA X GAA ACCUGAUU | AAUCAGGUA ACUGGAAG |
| 6308 | CUGAGUUU CUGAUGA X GAA AGCUCCUU | AAGGAGGUU AAACUCAG |
| 6309 | UCUGAGUU CUGAUGA X GAA AACCUCCU | AGGAGGUUA AACUCAGA |
| 6314 | UUUUUUCU CUGAUGA X GAA AGUUUAAC | GUUAAACUC AGAAAAAA |
| 6331 | AAUUGACU CUGAUGA X GAA AGGUCUUC | GAAGACCUC AGUCAAUU |
| 6335 | AGAGAAUU CUGAUGA X GAA ACUGAGGU | ACCUCAGUC AAUUCUCU |
| 6339 | AAGUAGAG CUGAUGA X GAA AUUGACUG | CAGUCAAUU CUCUACUU |
| 6340 | AAAGUAGA CUGAUGA X GAA AAUUGACU | AGUCAAUUC UCUACUUU |
| 6342 | AAAAAGUA CUGAUGA X GAA AGAAUUGA | UCAAUUCUC UACUUUUU |
| 6344 | AAAAAAAG CUGAUGA X GAA AGAGAAUU | AAUUCUCUA CUUUUUUU |
| 6347 | AAAAAAAA CUGAUGA X GAA AGUAGAGA | UCUCUACUU UUUUUUUU |
| 6348 | AAAAAAAA CUGAUGA X GAA AAGUAGAG | CUCUACUUU UUUUUUUU |
| 6349 | AAAAAAAA CUGAUGA X GAA AAAGUAGA | UCUACUUUU UUUUUUUU |
| 6350 | AAAAAAAA CUGAUGA X GAA AAAAGUAG | CUACUUUUU UUUUUUUU |
| 6351 | AAAAAAAA CUGAUGA X GAA AAAAAGUA | UACUUUUUU UUUUUUUU |
| 6352 | AAAAAAAA CUGAUGA X GAA AAAAAAGU | ACUUUUUUU UUUUUUUU |
| 6353 | AAAAAAAA CUGAUGA X GAA AAAAAAAG | CUUUUUUUU UUUUUUUU |
| 6354 | GAAAAAAA CUGAUGA X GAA AAAAAAAA | UUUUUUUUU UUUUUUUC |
| 6355 | GGAAAAAA CUGAUGA X GAA AAAAAAAA | UUUUUUUUU UUUUUUCC |
| 6356 | UGGAAAAA CUGAUGA X GAA AAAAAAAA | UUUUUUUUU UUUUUCCA |
| 6357 | UUGGAAAA CUGAUGA X GAA AAAAAAAA | UUUUUUUUU UUUUCCAA |
| 6358 | UUUGGAAA CUGAUGA X GAA AAAAAAAA | UUUUUUUUU UUUCCAAA |
| 6359 | AUUUGGAA CUGAUGA X GAA AAAAAAAA | UUUUUUUUU UUCCAAAU |
| 6360 | GAUUUGGA CUGAUGA X GAA AAAAAAAA | UUUUUUUUU UCCAAAUC |
| 6361 | UGAUUUGG CUGAUGA X GAA AAAAAAAA | UUUUUUUUU CCAAAUCA |
| 6362 | CUGAUUUG CUGAUGA X GAA AAAAAAAA | UUUUUUUUC CAAAUCAG |
| 6368 | UAUUAUCU CUGAUGA X GAA AUUUGGAA | UUCCAAAUC AGAUAAUA |

(continued)

| nt. Position | HH Ribozyme | Substrate |
|---|---|---|
| 6373 | UGGGCUAU CUGAUGA X GAA AUCUGAUU | AAUCAGAUA AUAGCCCA |
| 6376 | UGCUGGGC CUGAUGA X GAA AUUAUCUG | CAGAUAAUA GCCCAGCA |
| 6388 | GUUAUCAC CUGAUGA X GAA AUUUGCUG | CAGCAAAUA GUGAUAAC |
| 6394 | UUAUUUGU CUGAUGA X GAA AUCACUAU | AUAGUGAUA ACAAAUAA |
| 6401 | UAAGGUUU CUGAUGA X GAA AUUUGUUA | UAACAAAUA AAACCUUA |
| 6408 | GAACAGCU CUGAUGA X GAA AGGUUUUA | UAAAACCUU AGCUGUUC |
| 6409 | UGAACAGC CUGAUGA X GAA AAGGUUUU | AAAACCWA GCUGUUCA |
| 6415 | AAGACAUG CUGAUGA X GAA ACAGCUAA | UUAGCUGUU CAUGUCUU |
| 6416 | CAAGACAU CUGAUGA X GAA AACAGCUA | UAGCUGUUC AUGUCUUG |
| 6421 | GAAAUCAA CUGAUGA X GAA ACAUGAAC | GUUCAUGUC UUGAUUUC |
| 6423 | UUGAAAUC CUGAUGA X GAA AGACAUGA | UCAUGUCUU GAUUUCAA |
| 6427 | AUUAUUGA CUGAUGA X GAA AUCAAGAC | GUCUUGAUU UCAAUAAU |
| 6428 | AAUUAUUG CUGAUGA X GAA AAUCAAGA | UCUUGAUUU CAAUAAUU |
| 6429 | UAAUUAUU CUGAUGA X GAA AAAUCAAG | CUUGAUUUC AAUAAWA |
| 6433 | GAAUUAAU CUGAUGA X GAA AUUGAAAU | AUUUCAAUA AUUAAUUC |
| 6436 | UAAGAAUU CUGAUGA X GAA AUUAUUGA | UCAAUAAUU AAUUCUUA |
| 6437 | UUAAGAAU CUGAUGA X GAA AAUUAUUG | CAAUAAUUA AUUCUUAA |
| 6440 | UGAUUAAG CUGAUGA X GAA AUUAAUUA | UAAUUAAUU CUUAAUCA |
| 6441 | AUGAUUAA CUGAUGA X GAA AAUUAAUU | AAUUAAUUC UUAAUCAU |
| 6443 | UAAUGAUU CUGAUGA X GAA AGAAUUAA | UUAAUUCUU AAUCAUUA |
| 6444 | UUAAUGAU CUGAUGA X GAA AAGAAUUA | UAAUUCUUA AUCAUUAA |
| 6447 | CUCUUAAU CUGAUGA X GAA AUUAAGAA | UUCUUAAUC AUUAAGAG |
| 6450 | GGUCUCUU CUGAUGA X GAA AUGAUUAA | UUAAUCAUU AAGAGACC |
| 6451 | UGGUCUCU CUGAUGA X GAA AAUGAUUA | UAAUCAUUA AGAGACCA |
| 6461 | GUAUUUAU CUGAUGA X GAA AUGGUCUC | GAGACCAUA AUAAAUAC |
| 6464 | GGAGUAUU CUGAUGA X GAA AUUAUGGU | ACCAUAAUA AAUACUCC |
| 6468 | AAAAGGAG CUGAUGA X GAA AUUUAUUA | UAAUAAAUA CUCCUUUU |
| 6471 | UUGAAAAG CUGAUGA X GAA AGUAUUUA | UAAAUACUC CUUUUCAA |
| 6474 | CUCUUGAA CUGAUGA X GAA AGGAGUAU | AUACUCCUU UUCAAGAG |
| 6475 | UCUCUUGA CUGAUGA X GAA AAGGAGUA | UACUCCUUU UCAAGAGA |
| 6476 | UUCUCUUG CUGAUGA X GAA AAAGGAGU | ACUCCUUUU CAAGAGAA |
| 6477 | UUUCUCUU CUGAUGA X GAA AAAAGGAG | CUCCUUUUC AAGAGAAA |
| 6497 | ACAAUUCU CUGAUGA X GAA AUGGUUUU | AAAACCAUU AGAAUUGU |
| 6498 | AACAAUUC CUGAUGA X GAA AAUGGUUU | AAACCAUUA GAAUUGUU |
| 6503 | UGAGUAAC CUGAUGA X GAA AUUCUAAU | AUUAGAAUU GUUACUCA |
| 6506 | AGCUGAGU CUGAUGA X GAA ACAAUUCU | AGAAUUGUU ACUCAGCU |
| 6507 | GAGCUGAG CUGAUGA X GAA AACAAUUC | GAAUUGUUA CUCAGCUC |
| 6510 | AAGGAGCU CUGAUGA X GAA AGUAACAA | UUGUUACUC AGCUCCUU |
| 6515 | GUUUGAAG CUGAUGA X GAA AGCUGAGU | ACUCAGCUC CUUCAAAC |
| 6518 | UGAGUUUG CUGAUGA X GAA AGGAGCUG | AGCUCCUU CAAACUCA |
| 6519 | CUGAGUUU CUGAUGA X GAA AAGGAGCU | AGCUCCUUC AAACUCAG |
| 6525 | ACAAACCU CUGAUGA X GAA AGUUUGAA | UUCAAACUC AGGUUUGU |
| 6530 | AUGCUACA CUGAUGA X GAA ACCUGAGU | ACUCAGGUU UGUAGCAU |
| 6531 | UAUGCUAC CUGAUGA X GAA AACCUGAG | CUCAGGUUU GUAGCAUA |
| 6534 | AUGUAUGC CUGAUGA X GAA ACAAACCU | AGGUUUGUA GCAUACAU |
| 6539 | GACUCAUG CUGAUGA X GAA AUGCUACA | UGUAGCAUA CAUGAGUC |
| 6547 | GAUGGAUG CUGAUGA X GAA ACUCAUGU | ACAUGAGUC CAUCCAUC |
| 6551 | GACUGAUG CUGAUGA X GAA AUGGACUC | GAGUCCAUC CAUCAGUC |
| 6555 | CUUUGACU CUGAUGA X GAA AUGGAUGG | CCAUCCAUC AGUCAAAG |
| 6559 | CAUUCUUU CUGAUGA X GAA ACUGAUGG | CCAUCAGUC AAAGAAUG |

(continued)

| nt. Position | HH Ribozyme | Substrate |
|---|---|---|
| 6570 | CCAGAUGG CUGAUGA X GAA ACCAUUCU | AGAAUGGUU CCAUCUGG |
| 6571 | UCCAGAUG CUGAUGA X GAA AACCAUUC | GAAUGGUUC CAUCUGGA |
| 6575 | AGACUCCA CUGAUGA X GAA AUGGAACC | GGUUCCAUC UGGAGUCU |
| 6582 | UACAUUAA CUGAUGA X GAA ACUCCAGA | UCUGGAGUC UUAAUGUA |
| 6584 | UCUACAUU CUGAUGA X GAA AGACUCCA | UGGAGUCUU AAUGUAGA |
| 6585 | UUCUACAU CUGAUGA X GAA AAGACUCC | GGAGUCUUA AUGUAGAA |
| 6590 | UUUCUUUC CUGAUGA X GAA ACAUUAAG | CUUAAUGUA GAAAGAAA |
| 6609 | AUUAUUAC CUGAUGA X GAA AGUCUCCA | UGGAGACUU GUAAUAAU |
| 6612 | CUCAUUAU CUGAUGA X GAA ACAAGUCU | AGACUUGUA AUAAUGAG |
| 6615 | UAGCUCAU CUGAUGA X GAA AUUACAAG | CUUGUAAUA AUGAGCUA |
| 6623 | UUUGUAAC CUGAUGA X GAA AGCUCCUU | AAUGAGCUA GUUACAAA |
| 6626 | CACUUUGU CUGAUGA X GAA ACUAGCUC | GAGCUAGUU ACAAAGUG |
| 6627 | GCACUUUG CUGAUGA X GAA AACUAGCU | AGCUAGUUA CAAAGUGC |
| 6637 | UAAUGAAC CUGAUGA X GAA AGCACUUU | AAAGUGCUU GUUCAUUA |
| 6640 | UUUUAAUG CUGAUGA X GAA ACAAGCAC | GUGCUUGUU CAUUAAAA |
| 6641 | AUUUUAAU CUGAUGA X GAA AACAAGCA | UGCUUGUUC AUUAAAAU |
| 6644 | GCUAUUUU CUGAUGA X GAA AUGAACAA | UUGUUCAUU AAAAUAGC |
| 6645 | UGCUAUUU CUGAUGA X GAA AAUGAACA | UGUUCAUUA AAAUAGCA |
| 6650 | UUCAGUGC CUGAUGA X GAA AUUUUAAU | AUUAAAAUA GCACUGAA |
| 6662 | CAUGUUUC CUGAUGA X GAA AUUUUCAG | CUGAAAAUU GAAACAUG |
| 6674 | UAUCAGUU CUGAUGA X GAA AUUCAUGU | ACAUGAAUU AACUGAUA |
| 6675 | UUAUCAGU CUGAUGA X GAA AAUUCAUG | CAUGAAUUA ACUGAUAA |
| 6682 | UGGAAUAU CUGAUGA X GAA AUCAGUUA | UAACUGAUA AUAUUCCA |
| 6685 | GAUUGGAA CUGAUGA X GAA AUUAUCAG | CUGAUAAUA UUCCAAUC |
| 6687 | AUGAUUGG CUGAUGA X GAA AUAUUAUC | GAUAAUAUU CCAAUCAU |
| 6688 | AAUGAUUG CUGAUGA X GAA AAUAUUAU | AUAAUAUUC CAAUCAUU |
| 6693 | UGGCAAAU CUGAUGA X GAA AUUGGAAU | AUUCCAAUC AUUUGCCA |
| 6696 | AAAUGGCA CUGAUGA X GAA AUGAUUGG | CCAAUCAUU UGCCAUUU |
| 6697 | UAAAUGGC CUGAUGA X GAA AAUGAUUG | CAAUCAUUU GCCAUUUA |
| 6703 | UUGUCAUA CUGAUGA X GAA AUGGCAAA | UUUGCCAUU UAUGACAA |
| 6704 | UUUGUCAU CUGAUGA X GAA AAUGGCAA | UUGCCAUUU AUGACAAA |
| 6705 | UUUUGUCA CUGAUGA X GAA AAAUGGCA | UGCCAUUUA UGACAAAA |
| 6719 | UUAGUGCC CUGAUGA X GAA ACCAUUUU | AAAAUGGUU GGCACUAA |
| 6726 | UUCUUUGU CUGAUGA X GAA AGUGCCAA | UUGGCACUA ACAAAGAA |
| 6743 | CUGAAAGG CUGAUGA X GAA AGUGCUCG | CGAGCACUU CCUUUCAG |
| 6744 | UCUGAAAG CUGAUGA X GAA AAGUGCUC | GAGCACUUC CUUUCAGA |
| 6747 | AACUCUGA CUGAUGA X GAA AGGAAGUG | CACUUCCUU UCAGAGUU |
| 6748 | AAACUCUG CUGAUGA X GAA AAGGAAGU | ACUUCCUUU CAGAGUUU |
| 6749 | GAAACUCU CUGAUGA X GAA AAAGGAAG | CUUCCUUUC AGAGUUUC |
| 6755 | AUCUCAGA CUGAUGA X GAA ACUCUGAA | UUCAGAGUU UCUGAGAU |
| 6756 | UAUCUCAG CUGAUGA X GAA AACUCUGA | UCAGAGUUU CUGAGAUA |
| 6757 | UUAUCUCA CUGAUGA X GAA AAACUCUG | CAGAGUUUC UGAGAUAA |
| 6764 | ACGUACAU CUGAUGA X GAA AUCUCAGA | UCUGAGAUA AUGUACGU |
| 6769 | GUUCCACG CUGAUGA X GAA ACAUUAUC | GAUAAUGUA CGUGGAAC |
| 6781 | UCCACCCA CUGAUGA X GAA ACUGUUCC | GGAACAGUC UGGGUGGA |
| 6814 | AAGACACA CUGAUGA X GAA ACUUGCAC | GUGCAAGUC UGUGUCUU |
| 6820 | ACUGACAA CUGAUGA X GAA ACACAGAC | GUCUGUGUC UUGUCAGU |
| 6822 | GGACUGAC CUGAUGA X GAA AGACACAG | CUGUGUCUU GUCAGUCC |
| 6825 | CUUGGACU CUGAUGA X GAA ACAAGACA | UGUCUUGUC AGUCCAAG |
| 6829 | ACUUCUUG CUGAUGA X GAA ACUGACAA | UUGUCAGUC CAAGAAGU |

(continued)

| nt. Position | HH Ribozyme | Substrate |
|---|---|---|
| 6851 | CUAAAAUU CUGAUGA X GAA ACAUCUCG | CGAGAUGUU AAUUUUAG |
| 6852 | CCUAAAAU CUGAUGA X GAA AACAUCUC | GAGAUGUUA AUUUUAGG |
| 6855 | GUCCCUAA CUGAUGA X GAA AUUAACAU | AUGUUAAUU UUAGGGAC |
| 6856 | GGUCCCUA CUGAUGA X GAA AAUUAACA | UGUUAAUUU UAGGGACC |
| 6857 | GGGUCCCU CUGAUGA X GAA AAAUUAAC | GUUAAUUUU AGGGACCC |
| 6858 | CGGGUCCC CUGAUGA X GAA AAAAUUAA | UUAAUUUUA GGGACCCG |
| 6872 | UAGGAAAC CUGAUGA X GAA AGGCACGG | CCGUGCCUU GUUUCCUA |
| 6875 | GGCUAGGA CUGAUGA X GAA ACAAGGCA | UGCCUUGUU UCCUAGCC |
| 6876 | GGGCUAGG CUGAUGA X GAA AACAAGGC | GCCUUGUUU CCUAGCCC |
| 6877 | UGGGCUAG CUGAUGA X GAA AAACAAGG | CCUUGUUUC CUAGCCCA |
| 6880 | UUGUGGGC CUGAUGA X GAA AGGAAACA | UGUUUCCUA GCCCACAA |
| 6901 | AUCUGUUU CUGAUGA X GAA AUGUUUGC | GCAAACAUC AAACAGAU |
| 6910 | CUAGCGAG CUGAUGA X GAA AUCUGUUU | AAACAGAUA CUCGCUAG |
| 6913 | AGGCUAGC CUGAUGA X GAA AGUAUCUG | CAGAUACUC GCUAGCCU |
| 6917 | AAUGAGGC CUGAUGA X GAA AGCGAGUA | UACUCGCUA GCCUCAUU |
| 6922 | AUUUAAAU CUGAUGA X GAA AGGCUAGC | GCUAGCCUC AUUUAAAU |
| 6925 | UCAAUUUA CUGAUGA X GAA AUGAGGCU | AGCCUCAUU UAAAUUGA |
| 6926 | AUCAAUUU CUGAUGA X GAA AAUGAGGC | GCCUCAUUU AAAUUGAU |
| 6927 | AAUCAAUU CUGAUGA X GAA AAAUGAGG | CCUCAUUUA AAUUGAUU |
| 6931 | CUUUAAUC CUGAUGA X GAA AUUUAAAU | AUUUAAAUU GAUUAAAG |
| 6935 | CCUCCUUU CUGAUGA X GAA AUCAAUUU | AAAUUGAUU AAAGGAGG |
| 6936 | UUCUCCUU CUGAUGA X GAA AAUCAAUU | AAUUGAUUA AAGGAGGA |
| 6951 | CGGCCAAA CUGAUGA X GAA AUGCACUC | GAGUGCAUC UUUGGCCG |
| 6953 | GUCGGCCA CUGAUGA X GAA AGAUGCAC | GUGCAUCUU UGGCCGAC |
| 6954 | UGUCGGCC CUGAUGA X GAA AAGAUGCA | UGCAUCUUU GGCCGACA |
| 6970 | CACACAGU CUGAUGA X GAA ACACCACU | AGUGGUGUA ACUGUGUG |
| 7026 | AACACACA CUGAUGA X GAA ACACCCAC | GUGGGUGUA UGUGUGUU |
| 7034 | AUGCACAA CUGAUGA X GAA ACACACAU | AUGUGUGUU UUGUGCAU |
| 7035 | UAUGCACA CUGAUGA X GAA AACACACA | UGUGUGUUU UGUGCAUA |
| 7036 | UUAUGCAC CUGAUGA X GAA AAACACAC | GUGUGUUUU GUGCAUAA |
| 7043 | UAAAUAGU CUGAUGA X GAA AUGCACAA | UUGUGCAUA ACUAUUUA |
| 7047 | UCCUUAAA CUGAUGA X GAA AGUUAUGC | GCAUAACUA UUUAAGGA |
| 7049 | UUUCCUUA CUGAUGA X GAA AUAGUUAU | AUAACUAUU UAAGGAAA |
| 7050 | GUUUCCUU CUGAUGA X GAA AAUAGUUA | UAACUAUUU AAGGAAAC |
| 7051 | AGUUUCCU CUGAUGA X GAA AAAUAGUU | AACUAUUUA AGGAAACU |
| 7065 | AACUUUAA CUGAUGA X GAA AUUCCAGU | ACUGGAAUU UUAAAGUU |
| 7066 | UAACUUUA CUGAUGA X GAA AAUUCCAG | CUGGAAUUU UAAAGUUA |
| 7067 | GUAACUUU CUGAUGA X GAA AAAUUCCA | UGGAAUUUU AAAGUUAC |
| 7068 | AGUAACUU CUGAUGA X GAA AAAAUUCC | GGAAUUUUA AAGUUACU |
| 7073 | AUAAAAGU CUGAUGA X GAA ACUUUAAA | UUUAAAGUU ACUUUUAU |
| 7074 | UAUAAAAG CUGAUGA X GAA AACUUUAA | UUAAAGUUA CUUUUAUA |
| 7077 | UUGUAUAA CUGAUGA X GAA AGUAACUU | AAGUUACUU UUAUACAA |
| 7078 | UUUGUAUA CUGAUGA X GAA AAGUAACU | AGUUACUUU UAUACAAA |
| 7079 | GUUUGUAU CUGAUGA X GAA AAAGUAAC | GUUACUUUU AUACAAAC |
| 7080 | GGUUUGUA CUGAUGA X GAA AAAAGUAA | UUACUUUUA UACAAACC |
| 7082 | UUGGUUUG CUGAUGA X GAA AUAAAAGU | ACUUUUAUA CAAACCAA |
| 7095 | GUAGCAUA CUGAUGA X GAA AUUCUUGG | CCAAGAAUA UAUGCUAC |
| 7097 | CUGUAGCA CUGAUGA X GAA AUAUUCUU | AAGAAUAUA UGCUACAG |
| 7102 | UAUAUCUG CUGAUGA X GAA AGCAUAUA | UAUAUGCUA CAGAUAUA |
| 7108 | CUGUCUUA CUGAUGA X GAA AUCUGUAG | CUACAGAUA UAAGACAG |

(continued)

| nt. Position | HH Ribozyme | Substrate |
|---|---|---|
| 7110 | GUCUGUCU CUGAUGA X GAA AUAUCUGU | ACAGAUAUA AGACAGAC |
| 7124 | UAGGACCA CUGAUGA X GAA ACCAUGUC | GACAUGGUU UGGUCCUA |
| 7125 | AUAGGACC CUGAUGA X GAA AACCAUGU | ACAUGGUUU GGUCCUAU |
| 7129 | AAAUAUAG CUGAUGA X GAA ACCAAACC | GGUUUGGUC CUAUAUUU |
| 7132 | UAGAAAUA CUGAUGA X GAA AGGACCAA | UUGGUCCUA UAUUUCUA |
| 7134 | ACUAGAAA CUGAUGA X GAA AUAGGACC | GGUCCUAUA UUUCUAGU |
| 7136 | UGACUAGA CUGAUGA X GAA AUAUAGGA | UCCUAUAUU UCUAGUCA |
| 7137 | AUGACUAG CUGAUGA X GAA AAUAUAGG | CCUAUAUUU CUAGUCAU |
| 7138 | CAUGACUA CUGAUGA X GAA AAAUAUAG | CUAUAUUUC UAGUCAUG |
| 7140 | AUCAUGAC CUGAUGA X GAA AGAAAUAU | AUAUUUCUA GUCAUGAU |
| 7143 | UUCAUCAU CUGAUGA X GAA ACUAGAAA | UUUCUAGUC AUGAUGAA |
| 7155 | AUACAAAA CUGAUGA X GAA ACAUUCAU | AUGAAUGUA UUUUGUAU |
| 7157 | GUAUACAA CUGAUGA X GAA AUACAUUC | GAAUGUAUU UUGUAUAC |
| 7158 | GGUAUACA CUGAUGA X GAA AAUACAUU | AAUGUAUUU UGUAUACC |
| 7159 | UGGUAUAC CUGAUGA X GAA AAAUACAU | AUGUAUUUU GUAUACCA |
| 7162 | AGAUGGUA CUGAUGA X GAA ACAAAAUA | UAUUUUGUA UACCAUCU |
| 7164 | GAAGAUGG CUGAUGA X GAA AUACAAAA | UUUUGUAUA CCAUCUUC |
| 7169 | UAUAUGAA CUGAUGA X GAA AUGGUAUA | UAUACCAUC UUCAUAUA |
| 7171 | AUUAUAUG CUGAUGA X GAA AGAUGGUA | UACCAUCUU CAUAUAAU |
| 7172 | UAUUAUAU CUGAUGA X GAA AAGAUGGU | ACCAUCUUC AUAUAAUA |
| 7175 | GUAUAUUA CUGAUGA X GAA AUGAAGAU | AUCUUCAUA UAAUAUAC |
| 7177 | AAGUAUAU CUGAUGA X GAA AUAUGAAG | CUUCAUAUA AUAUACUU |
| 7180 | UUUAAGUA CUGAUGA X GAA AUUAUAUG | CAUAUAAUA UACUUAAA |
| 7182 | UUUUUAAG CUGAUGA X GAA AUAUUAUA | UAUAAUAUA CUUAAAAA |
| 7185 | AUAUUUUU CUGAUGA X GAA AGUAUAUU | AAUAUACUU AAAAAUAU |
| 7186 | AAUAUUUU CUGAUGA X GAA AAGUAUAU | AUAUACUUA AAAAUAUU |
| 7192 | UUAAGAAA CUGAUGA X GAA AUUUUUAA | UUAAAAAUA UUUCUUAA |
| 7194 | AAUUAAGA CUGAUGA X GAA AUAUUUUU | AAAAAUAUU UCUUAAUU |
| 7195 | CAAUUAAG CUGAUGA X GAA AAUAUUUU | AAAAUAUUU CUUAAUUG |
| 7196 | CCAAUUAA CUGAUGA X GAA AAAUAUUU | AAAUAUUUC UUAAUUGG |
| 7198 | UCCCAAUU CUGAUGA X GAA AGAAAUAU | AUAUUUCUU AAUUGGGA |
| 7199 | AUCCCAAU CUGAUGA X GAA AAGAAAUA | UAUUUCUUA AUUGGGAU |
| 7202 | CAAAUCCC CUGAUGA X GAA AUUAAGAA | UUCUUAAUU GGGAUUUG |
| 7208 | CGAUUACA CUGAUGA X GAA AUCCCAAU | AUUGGGAUU UGUAAUCG |
| 7209 | ACGAUUAC CUGAUGA X GAA AAUCCCAA | UUGGGAUUU GUAAUCGU |
| 7212 | GGUACGAU CUGAUGA X GAA ACAAAUCC | GGAUUUGUA AUCGUACC |
| 7215 | GUUGGUAC CUGAUGA X GAA AUUACAAA | UUUGUAAUC GUACCAAC |
| 7218 | UAAGUUGG CUGAUGA X GAA ACGAUUAC | GUAAUCGUA CCAACUUA |
| 7225 | UAUCAAUU CUGAUGA X GAA AGUUGGUA | UACCAACUU AAUUGAUA |
| 7226 | UUAUCAAU CUGAUGA X GAA AAGUUGGU | ACCAACUUA AUUGAUAA |
| 7229 | AGUUUAUC CUGAUGA X GAA AUUAAGUU | AACUUAAUU GAUAAACU |
| 7233 | GCCAAGUU CUGAUGA X GAA AUCAAUUA | UAAUUGAUA AACUUGGC |
| 7238 | CAGUUGCC CUGAUGA X GAA AGUUUAUC | GAUAAACUU GGCAACUG |
| 7249 | GAACAUAA CUGAUGA X GAA AGCAGUUG | CAACUGCUU UUAUGUUC |
| 7250 | AGAACAUA CUGAUGA X GAA AAGCAGUU | AACUGCUUU UAUGUUCU |
| 7251 | CAGAACAU CUGAUGA X GAA AAAGCAGU | ACUGCUUUU AUGUUCUG |
| 7252 | ACAGAACA CUGAUGA X GAA AAAAGCAG | CUGCUUUUA UGUUCUGU |
| 7256 | GGAGACAG CUGAUGA X GAA ACAUAAAA | UUUUAUGUU CUGUCUCC |
| 7257 | AGGAGACA CUGAUGA X GAA AACAUAAA | UUUAUGUUC UGUCUCCU |
| 7261 | UGGAAGGA CUGAUGA X GAA ACAGAACA | UGUUCUGUC UCCUUCCA |

(continued)

| nt. Position | HH Ribozyme | Substrate |
|---|---|---|
| 7263 | UAUGGAAG CUGAUGA X GAA AGACAGAA | UUCUGUCUC CUUCCAUA |
| 7266 | AUUUAUGG CUGAUGA X GAA AGGAGACA | UUCUCCUU CCAUAAAU |
| 7267 | AAUUUAUG CUGAUGA X GAA AAGGAGAC | GUCUCCUUC CAUAAAUU |
| 7271 | GAAAAAUU CUGAUGA X GAA AUGGAAGG | CCUUCCAUA AAUUUUUC |
| 7275 | UUUUGAAA CUGAUGA X GAA AUUUAUGG | CCAUAAAUU UUUCAAAA |
| 7276 | AUUUUGAA CUGAUGA X GAA AAUUUAUG | CAUAAAUUU UUCAAAAU |
| 7277 | UAUUUUGA CUGAUGA X GAA AAAUUUAU | AUAAAUUUU UCAAAAUA |
| 7278 | GUAUUUUG CUGAUGA X GAA AAAAUUUA | UAAAUUUUU CAAAAUAC |
| 7279 | AGUAUUUU CUGAUGA X GAA AAAAAUUU | AAAUUUUUC AAAAUACU |
| 7285 | UGAAUUAG CUGAUGA X GAA AUUUUGAA | UUCAAAAUA CUAAUUCA |
| 7288 | UGUUGAAU CUGAUGA X GAA AGUAUUUU | AAAAUACUA AUUCAACA |
| 7291 | CUUUGUUG CUGAUGA X GAA AUUAGUAU | AUACUAAUU CAACAAAG |
| 7292 | UCUUUGUU CUGAUGA X GAA AAUUAGUA | UACUAAUUC AACAAAGA |
| 7308 | AAAAAAAA CUGAUGA X GAA AGCUUUUU | AAAAAGCUC UUUUUUUU |
| 7310 | GGAAAAAA CUGAUGA X GAA AGAGCUUU | AAAGCUCUU UUUUUUCC |
| 7311 | AGGAAAAA CUGAUGA X GAA AAGAGCUU | AAGCUCUUU UUUUUCCU |
| 7312 | UAGGAAAA CUGAUGA X GAA AAAGAGCU | AGCUCUUUU UUUUCCUA |
| 7313 | UUAGGAAA CUGAUGA X GAA AAAAGAGC | GCUCUUUUU UUUCCUAA |
| 7314 | UUUAGGAA CUGAUGA X GAA AAAAAGAG | CUCUUUUUU UUCCUAAA |
| 7315 | UUUUAGGA CUGAUGA X GAA AAAAAAGA | UCUUUUUUU UCCUAAAA |
| 7316 | AUUUUAGG CUGAUGA X GAA AAAAAAAG | CUUUUUUUU CCUAAAAU |
| 7317 | UAUUUUAG CUGAUGA X GAA AAAAAAAA | UUUUUUUUC CUAAAAUA |
| 7320 | GUUUAUUU CUGAUGA X GAA AGGAAAAA | UUUUUCCUA AAAUAAAC |
| 7325 | UUUGAGUU CUGAUGA X GAA AUUUUAGG | CCUAAAAUA AACUCAAA |
| 7330 | AUAAAUUU CUGAUGA X GAA AGUUUAUU | AAUAAACUC AAAUUUAU |
| 7335 | CAAGGAUA CUGAUGA X GAA AUUUGAGU | ACUCAAAUU UAUCCUUG |
| 7336 | ACAAGGAU CUGAUGA X GAA AAUUUGAG | CUCAAAUUU AUCCUUGU |
| 7337 | AACAAGGA CUGAUGA X GAA AAAUUUGA | UCAAAUUUA UCCUUGUU |
| 7339 | UAAACAAG CUGAUGA X GAA AUAAAUUU | AAAUUUAUC CUUGUUUA |
| 7342 | CUCUAAAC CUGAUGA X GAA AGGAUAAA | UUUAUCCUU GUUUAGAG |
| 7345 | CUGCUCUA CUGAUGA X GAA ACAAGGAU | AUCCUUGUU UAGAGCAG |
| 7346 | UCUGCUCU CUGAUGA X GAA AACAAGGA | UCCUUGUUU AGAGCAGA |
| 7347 | CUCUGCUC CUGAUGA X GAA AAACAAGG | CCUUGUUUA GAGCAGAG |
| 7362 | UUUUUCUU CUGAUGA X GAA AUUUUUCU | AGAAAAAUU AAGAAAAA |
| 7363 | GUUUUUCU CUGAUGA X GAA AAUUUUUC | GAAAAAUUA AGAAAAAC |
| 7373 | CCAUUUCA CUGAUGA X GAA AGUUUUUC | GAAAAACUU UGAAAUGG |
| 7374 | ACCAUUUC CUGAUGA X GAA AAGUUUUU | AAAAACUUU GAAAUGGU |
| 7383 | UUUUUUGA CUGAUGA X GAA ACCAUUUC | GAAAUGGUC UCAAAAAA |
| 7385 | AAUUUUUU CUGAUGA X GAA AGACCAUU | AAUGGUCUC AAAAAAUU |
| 7393 | UAUUUAGC CUGAUGA X GAA AUUUUUUG | CAAAAAAUU GCUAAAUA |
| 7397 | AAAAUAUU CUGAUGA X GAA AGCAAUUU | AAAUUGCUA AAUAUUUU |
| 7401 | AUUGAAAA CUGAUGA X GAA AUUUAGCA | UGCUAAAUA UUUUCAAU |
| 7403 | CCAUUGAA CUGAUGA X GAA AUAUUUAG | CUAAAUAUU UUCAAUGG |
| 7404 | UCCAUUGA CUGAUGA X GAA AAUAUUUA | UAAAUAUUU UCAAUGGA |
| 7405 | UUCCAUUG CUGAUGA X GAA AAAUAUUU | AAAUAUUUU CAAUGGAA |
| 7406 | UUUCCAUU CUGAUGA X GAA AAAAUAUU | AAUAUUUUC AAUGGAAA |
| 7418 | CUAACAUU CUGAUGA X GAA AGUUUUCC | GGAAAACUA AAUGUUAG |
| 7424 | GCUAAACU CUGAUGA X GAA ACAUUUAG | CUAAAUGUU AGUUUAGC |
| 7425 | AGCUAAAC CUGAUGA X GAA AACAUUUA | UAAAUGUUA GUUUAGCU |
| 7428 | AUCAGCUA CUGAUGA X GAA ACUAACAU | AUGUUAGUU UAGCUGAU |

(continued)

| nt. Position | HH Ribozyme | Substrate |
|---|---|---|
| 7429 | AAUCAGCU CUGAUGA X GAA AACUAACA | UGUUAGUUU AGCUGAUU |
| 7430 | CAAUCAGC CUGAUGA X GAA AAACUAAC | GUUAGUUUA GCUGAUUG |
| 7437 | CCCCAUAC CUGAUGA X GAA AUCAGCUA | UAGCUGAUU GUAUGGGG |
| 7440 | AAACCCCA CUGAUGA X GAA ACAAUCAG | CUGAUUGUA UGGGGUUU |
| 7447 | GGUUCGAA CUGAUGA X GAA ACCCCAUA | UAUGGGGUU UUCGAACC |
| 7448 | AGGUUCGA CUGAUGA X GAA AACCCCAU | AUGGGGUUU UCGAACCU |
| 7449 | AAGGUUCG CUGAUGA X GAA AAACCCCA | UGGGGUUUU CGAACCUU |
| 7450 | AAAGGUUC CUGAUGA X GAA AAAACCCC | GGGGUUUUC GAACCUUU |
| 7457 | AAAAGUGA CUGAUGA X GAA AGGUUCGA | UCGAACCUU UCACUUUU |
| 7458 | AAAAAGUG CUGAUGA X GAA AAGGUUCG | CGAACCUUU CACUUUUU |
| 7459 | CAAAAAGU CUGAUGA X GAA AAAGGUUC | GAACCUUUC ACUUUUUG |
| 7463 | CAAACAAA CUGAUGA X GAA AGUGAAAG | CUUUCACUU UUUGUUUG |
| 7464 | ACAAACAA CUGAUGA X GAA AAGUGAAA | UUUCACUUU UUGUUUGU |
| 7465 | AACAAACA CUGAUGA X GAA AAAGUGAA | UUCACUUUU UGUUUGUU |
| 7466 | AAACAAAC CUGAUGA X GAA AAAAGUGA | UCACUUUUU GUUUGUUU |
| 7469 | GUAAAACA CUGAUGA X GAA ACAAAAAG | CUUUUUGUU UGUUUUAC |
| 7470 | GGUAAAAC CUGAUGA X GAA AACAAAAA | UUUUUGUUU GUUUUACC |
| 7473 | AUAGGUAA CUGAUGA X GAA ACAAACAA | UUGUUUGUU UUACCUAU |
| 7474 | AAUAGGUA CUGAUGA X GAA AACAAACA | UGUUUGUUU UACCUAUU |
| 7475 | AAAUAGGU CUGAUGA X GAA AAACAAAC | GUUUGUUUU ACCUAUUU |
| 7476 | GAAAUAGG CUGAUGA X GAA AAAACAAA | UUUGUUUUA CCUAUUUC |
| 7480 | UUGUGAAA CUGAUGA X GAA AGGUAAAA | UUUUACCUA UUUCACAA |
| 7482 | AGUUGUGA CUGAUGA X GAA AUAGGUAA | UUACCUAUU UCACAACU |
| 7483 | CAGUUGUG CUGAUGA X GAA AAUAGGUA | UACCUAUUU CACAACUG |
| 7484 | ACAGUUGU CUGAUGA X GAA AAAUAGGU | ACCUAUUUC ACAACUGU |
| 7495 | UGGCAAUU CUGAUGA X GAA ACACAGUU | AACUGUGUA AAUUGCCA |
| 7499 | UUAUUGGC CUGAUGA X GAA AUUUACAC | GUGUAAAUU GCCAAUAA |
| 7506 | ACAGGAAU CUGAUGA X GAA AUUGGCAA | UUGCCAAUA AUUCCUGU |
| 7509 | UGGACAGG CUGAUGA X GAA AUUAUUGG | CCAAUAAUU CCUGUCCA |
| 7510 | AUGGACAG CUGAUGA X GAA AAUUAUUG | CAAUAAUUC CUGUCCAU |
| 7515 | UUUUCAUG CUGAUGA X GAA ACAGGAAU | AUUCCUGUC CAUGAAAA |
| 7531 | CACUGGAU CUGAUGA X GAA AUUUGCAU | AUGCAAAUU AUCCAGUG |
| 7532 | ACACUGGA CUGAUGA X GAA AAUUUGCA | UGCAAAUUA UCCAGUGU |
| 7534 | CUACACUG CUGAUGA X GAA AUAAUUUG | CAAAUUAUC CAGUGUAG |
| 7541 | AAUAUAUC CUGAUGA X GAA ACACUGGA | UCCAGUGUA GAUAUAUU |
| 7545 | GUCAAAUA CUGAUGA X GAA AUCUACAC | GUGUAGAUA UAUUUGAC |
| 7547 | UGGUCAAA CUGAUGA X GAA AUAUCUAC | GUAGAUAUA UUUGACCA |
| 7549 | GAUGGUCA CUGAUGA X GAA AUAUAUCU | AGAUAUAUU UGACCAUC |
| 7550 | UGAUGGUC CUGAUGA X GAA AAUAUAUC | GAUAUAUUU GACCAUCA |
| 7557 | CAUAGGGU CUGAUGA X GAA AUGGUCAA | UUGACCAUC ACCCUAUG |
| 7563 | AAUAUCCA CUGAUGA X GAA AGGGUGAU | AUCACCCUA UGGAUAUU |
| 7569 | CUAGCCAA CUGAUGA X GAA AUCCAUAG | CUAUGGAUA UUGGCUAG |
| 7571 | AACUAGCC CUGAUGA X GAA AUAUCCAU | AUGGAUAUU GGCUAGUU |
| 7576 | GGCAAAAC CUGAUGA X GAA AGCCAAUA | UAUUGGCUA GUUUUGCC |
| 7579 | AAAGGCAA CUGAUGA X GAA ACUAGCCA | UGGCUAGUU UUGCCUUU |
| 7580 | UAAAGGCA CUGAUGA X GAA AACUAGCC | GGCUAGUUU UGCCUUUA |
| 7581 | AUAAAGGC CUGAUGA X GAA AAACUAGC | GCUAGUUUU GCCUUUAU |
| 7586 | GCUUAAUA CUGAUGA X GAA AGGCAAAA | UUUUGCCUU UAUUAAGC |
| 7587 | UGCUUAAU CUGAUGA X GAA AAGGCAAA | UUUGCCUUU AUUAAGCA |
| 7588 | UUGCUUAA CUGAUGA X GAA AAAGGCAA | UUGCCUUUA UUAAGCAA |

(continued)

| nt. Position | HH Ribozyme | Substrate |
|---|---|---|
| 7590 | AUUUGCUU CUGAUGA X GAA AUAAAGGC | GCCUUUAUU AAGCAAAU |
| 7591 | AAUUUGCU CUGAUGA X GAA AAUAAAGG | CCUUUAUUA AGCAAAUU |
| 7599 | CUGAAAUG CUGAUGA X GAA AUUUGCUU | AAGCAAAUU CAUUUCAG |
| 7600 | GCUGAAAU CUGAUGA X GAA AAUUUGCU | AGCAAAUUC AUUUCAGC |
| 7603 | CAGGCUGA CUGAUGA X GAA AUGAAUUU | AAAUUCAUU UCAGCCUG |
| 7604 | UCAGGCUG CUGAUGA X GAA AAUGAAUU | AAUUCAUUU CAGCCUGA |
| 7605 | UUCAGGCU CUGAUGA X GAA AAAUGAAU | AUUCAUUUC AGCCUGAA |
| 7617 | UAUAGGCA CUGAUGA X GAA ACAUUCAG | CUGAAUGUC UGCCUAUA |
| 7623 | AGAAUAUA CUGAUGA X GAA AGGCAGAC | GUCUGCCUA UAUAUUCU |
| 7625 | AGAGAAUA CUGAUGA X GAA AUAGGCAG | CUGCCUAUA UAUUCUCU |
| 7627 | GCAGAGAA CUGAUGA X GAA AUAUAGGC | GCCUAUAUA UUCUCUGC |
| 7629 | GAGCAGAG CUGAUGA X GAA AUAUAUAG | CUAUAUAUU CUCUGCUC |
| 7630 | AGAGCAGA CUGAUGA X GAA AAUAUAUA | UAUAUAUUC UCUGCUCU |
| 7632 | AAAGAGCA CUGAUGA X GAA AGAAUAUA | UAUAUUCUC UGCUCUUU |
| 7637 | AAUACAAA CUGAUGA X GAA AGCAGAGA | UCUCUGCUC UUUGUAUU |
| 7639 | AGAAUACA CUGAUGA X GAA AGAGCAGA | UCUGCUCUU UGUAUUCU |
| 7640 | GAGAAUAC CUGAUGA X GAA AAGAGCAG | CUGCUCUUU GUAUUCUC |
| 7643 | AAGGAGAA CUGAUGA X GAA ACAAAGAG | CUCUUUGUA UUCUCCUU |
| 7645 | CAAAGGAG CUGAUGA X GAA AUACAAAG | CUUUGUAUU CUCCUUUG |
| 7646 | UCAAAGGA CUGAUGA X GAA AAUACAAA | UUUGUAUUC UCCUUUGA |
| 7648 | GUUCAAAG CUGAUGA X GAA AGAAUACA | UGUAUUCUC CUUUGAAC |
| 7651 | CGGGUUCA CUGAUGA X GAA AGGAGAAU | AUUCUCCUU UGAACCCG |
| 7652 | ACGGGUUC CUGAUGA X GAA AAGGAGAA | UUCUCCUU GAACCCGU |
| 7661 | GAUGUUUU CUGAUGA X GAA ACGGGUUC | GAACCCGUU AAAACAUC |
| 7662 | GGAUGUUU CUGAUGA X GAA AACGGGUU | AACCCGUUA AAACAUCC |
| 7669 | UGCCACAG CUGAUGA X GAA AUGUUUUA | UAAAACAUC CUGUGGCA |

Where "X" represents stem II region of a HH ribozyme (Hertel et al., 1992 Nucleic Acids Res. 20 3252). The length of stem II may be ≥ 2 base-pairs.

Table III: Human *flt*1 VEGF Receptor-Hairpin Ribozyme and Substrate Sequence

| nt. Position | HP Ribozyme Sequence | Substrate |
|---|---|---|
| 16 | CGGGGAGG AGAA GAGAGG ACCAGAGAAACACACGUUGUGGUACAUUACCUGGUA | CCUCUCG GCU CCUCCCCG |
| 39 | CCGCUCCG AGAA GCCGCC ACCAGAGAAACACACGUUGUGGUACAUUACCUGGUA | GGCGGCG GCU CGGAGCGG |
| 180 | CCGCCAGA AGAA GUCCUC ACCAGAGAAACACACGUUGUGGUACAUUACCUGGUA | GAGGACG GAC UCUGGCGG |
| 190 | AACGACCC AGAA GCCAGA ACCAGAGAAACACACGUUGUGGUACAUUACCUGGUA | UCUGGCG GCC GGGUCGUU |
| 278 | GCGCGCAC AGAA GGACCC ACCAGAGAAACACACGUUGUGGUACAUUACCUGGUA | GGGUCCU GCU GUGCGCGC |
| 290 | GACAGCUG AGAA GCGCGC ACCAGAGAAACACACGUUGUGGUACAUUACCUGGUA | GCGCGCU GCU CAGCUGUC |
| 295 | AAGCAGAC AGAA GAGCAG ACCAGAGAAACACACGUUGUGGUACAUUACCUGGUA | CUGCUCA GCU GUCUGCUU |
| 298 | GAGAAGCA AGAA GCUGAG ACCAGAGAAACACACGUUGUGGUACAUUACCUGGUA | CUCAGCU GUC UGCUUCUC |
| 302 | CUGUGAGA AGAA GACAGC ACCAGAGAAACACACGUUGUGGUACAUUACCUGGUA | GCUGUCU GCU UCUCACAG |

(continued)

| nt. Position | HP Ribozyme Sequence | Substrate |
|---|---|---|
| 420 | CAUUUAUG AGAA GCUUCC ACCAGAGAAACACACGUUGUGGUACAUUACCUGGUA | GGAAGCA GCC CAUAAAUG |
| 486 | CUUCCACA AGAA GAUUUA ACCAGAGAAACACACGUUGUGGUACAUUACCUGGUA | UAAAUCU GCC UGUGGAAG |
| 537 | UUUGCUUG AGAA GUGUUC ACCAGAGAAACACACGUUGUGGUACAUUACCUGGUA | GAACACA GCU CAAGCAAA |
| 565 | AUAUUUGC AGAA GUAGAA ACCAGAGAAACACACGUUGUGGUACAUUACCUGGUA | UUCUACA GCU GCAAAUAU |
| 721 | CGUAACCC AGAA GGGAAU ACCAGAGAAACACACGUUGUGGUACAUUACCUGGUA | AUUCCCU GCC GGGUUACG |
| 786 | CGUUUUCC AGAA GGGAUC ACCAGAGAAACACACGUUGUGGUACAUUACCUGGUA | GAUCCCU GAU GGAAAACG |
| 863 | CUUCACAG AGAA GAAGCC ACCAGAGAAACACACGUUGUGGUACAUUACCUGGUA | GGCUUCU GAC CUGUGAAG |
| 1056 | UUUUUUUC AGAA GGGUAA ACCAGAGAAACACACGUUGUGGUACAUUACCUGGUA | UUACCCU GAU GAAAAAAA |
| 1301 | GCCGGUAA AGAA GCUUGC ACCAGAGAAACACACGUUGUGGUACAUUACCUGGUA | GCAAGCG GUC UUACCGGC |
| 1310 | UCAUAGAG AGAA GGUAAG ACCAGAGAAACACACGUUGUGGUACAUUACCUGGUA | CUUACCG GCU CUCUAUGA |
| 1389 | AAAUAGCG AGAA GAUUUC ACCAGAGAAACACACGUUGUGGUACAUUACCUGGUA | GAAAUCU GCU CGCUAUUU |
| 1535 | UUUCGUAA AGAA GGGGUU ACCAGAGAAACACACGUUGUGGUACAUUACCUGGUA | AACCCCA GAU UUACGAAA |
| 1566 | AGAGCCGG AGAA GGAAAC ACCAGAGAAACACACGUUGUGGUACAUUACCUGGUA | GUUUCCA GAC CCGGCUCU |
| 1572 | GGGUAGAG AGAA GGGUCU ACCAGAGAAACACACGUUGUGGUACAUUACCUGGUA | AGACCCG GCU CUCUACCC |
| 1604 | CGGUACAA AGAA GGAUUU ACCAGAGAAACACACGUUGUGGUACAUUACCUGGUA | AAAUCCU GAC UUGUACCG |
| 1824 | AUUCUAGA AGAA GCCACA ACCAGAGAAACACACGUUGUGGUACAUUACCUGGUA | UGUGGCU GAC UCUAGAAU |
| 1908 | UUUGGCAC AGAA GUGAUA ACCAGAGAAACACACGUUGUGGUACAUUACCUGGUA | UAUCACA GAU GUGCCAAA |
| 1949 | CUCCUUCC AGAA GCAUUU ACCAGAGAAACACACGUUGUGGUACAUUACCUGGUA | AAAUGCC GAC GGAAGGAG |
| 1973 | CUGUGCAA AGAA GUUUCA ACCAGAGAAACACACGUUGUGGUACAUUACCUGGUA | UGAAACU GUC UUGCACAG |
| 2275 | AGUGGUGG AGAA GCUGAU ACCAGAGAAACACACGUUGUGGUACAUUACCUGGUA | AUCAGCA GUU CCACCACU |
| 2321 | ACCAAGUG AGAA GAGGCU ACCAGAGAAACACACGUUGUGGUACAUUACCUGGUA | AGCCUCA GAU CACUUGGU |
| 2396 | UUUCAAUA AGAA GCGUGC ACCAGAGAAACACACGUUGUGGUACAUUACCUGGUA | GCACGCU GUU UAUUGAAA |
| 2490 | GUUCCUUG AGAA GUGAGG ACCAGAGAAACACACGUUGUGGUACAUUACCUGGUA | CCUCACU GUU CAAGGAAC |
| 2525 | UUAGAGUG AGAA GCUCCA ACCAGAGAAACACACGUUGUGGUACAUUACCUGGUA | UGGAGCU GAU CACUCUAA |
| 2625 | GAUAGGUA AGAA GUCUUU ACCAGAGAAACACACGUUGUGGUACAUUACCUGGUA | AAAGACU GAC UACCUAUC |
| 2652 | GGAACUUC AGAA GGGUCC ACCAGAGAAACACACGUUGUGGUACAUUACCUGGUA | GGACCCA GAU GAAGUUCC |

(continued)

| nt. Position | HP Ribozyme Sequence | Substrate |
|---|---|---|
| 2684 | CAUAAGGG AGAA GCUCAC ACCAGAGAAACACACGUUGUGGUACAUUACCUGGUA | GUGAGCG GCU CCCUUAUG |
| 2816 | CAGCCACA AGAA GGCACG ACCAGAGAAACACACGUUGUCGUACAUUACCUGGUA | CGUGCCG GAC UGUGGCUG |
| 2873 | GCUCAGUC AGAA GAGCUU ACCAGAGAAACACACGUUGUGGUACAUUACCUGGUA | AAGCUCU GAU GACUGAGC |
| 2930 | AGGCUCCC AGAA GGUUAA ACCAGAGAAACACACGUUGUGGUACAUUACCUGGUA | UUAACCU GCU GGGAGCCU |
| 2963 | CAAUCACC AGAA GAGGCC ACCAGAGAAACACACGUUGUGGUACAUUACCUGGUA | GGCCUCU GAU GGUGAUUG |
| 3157 | UUCCUGAA AGAA GGAGCU ACCAGAGAAACACACGUUGUGGUACAUUACCUGGUA | AGCUCCG GCU UUCAGGAA |
| 3207 | UAGAAACC AGAA GAAUCC ACCAGAGAAACACACGUUGUGGUACAUUACCUGGUA | GGAUUCU GAC GGUUUCUA |
| 3211 | CUUGUAGA AGAA GUCAGA ACCAGAGAAACACACGUUGUGGUACAUUACCUGGUA | UCUGACG GUU UCUACAAG |
| 3245 | UGUAAGAA AGAA GAUCUU ACCAGAGAAACACACGUUGUGGUACAUUACCUGGUA | AAGAUCU GAU UUCUUACA |
| 3256 | CACUUGAA AGAA GUAAGA ACCAGAGAAACACACGUUGUGGUACAUUACCUGGUA | UCUUACA GUU UUCAAGUG |
| 3287 | UUCUGGAA AGAA GGAACU ACCAGAGAAACACACGUUGUGGUACAUUACCUGGUA | AGUUCCU GUC UUCCAGAA |
| 3402 | CUCACAUA AGAA GGGUUC ACCAGAGAAACACACGUUGUGGUACAUUACCUGGUA | GAACCCC GAU UAUGUGAG |
| 3580 | CCUCAGGC AGAA GCAAAA ACCAGAGAAACACACGUUGUGGUACAUUACCUGGUA | UUUUGCA GUC GCCUGAGG |
| 3641 | CCAGCAUG AGAA GAUAGA ACCAGAGAAACACACGUUGUGGUACAUUACCUGGUA | UCUAUCA GAU CAUGCUGG |
| 3655 | UCUGUGCC AGAA GUCCAG ACCAGAGAAACACACGUUGUGGUACAUUACCUGGUA | CUGGACU GCU GGCACAGA |
| 3810 | UCAGAGAA AGAA GGAGUU ACCAGAGAAACACACGUUGUGGUACAUUACCUGGUA | AACUCCU GCC UUCUCUGA |
| 3846 | AACUUCGG AGAA GAAAUA ACCAGAGAAACACACGUUGUGGUACAUUACCUGGUA | UAUUUCA GCU CCGAAGUU |
| 3873 | CUGACAUC AGAA GAGCUU ACCAGAGAAACACACGUUGUGGUACAUUACCUGGUA | AAGCUCU GAU GAUGUCAG |
| 3995 | GAGAGGCC AGAA GAGUGC ACCAGAGAAACACACGUUGUGGUACAUUACCUGGUA | GCACUCU GUU GGCCUCUC |
| 4100 | UGACAUCA AGAA GCCCCG ACCAGAGAAACACACGUUGUGGUACAUUACCUGGUA | CGGGGCU GUC UGAUGUCA |
| 4104 | CUGCUGAC AGAA GACAGC ACCAGAGAAACACACGUUGUGGUACAUUACCUGGUA | GCUGUCU GAU GUCAGCAG |
| 4120 | AUGGCAGA AGAA GGGCCU ACCAGAGAAACACACGUUGUGGUACAUUACCUGGUA | AGGCCCA GUU UCUGCCAU |
| 4135 | GUGCCCAC AGAA GGAAUG ACCAGAGAAACACACGUUGUGGUACAUUACCUGGUA | CAUUCCA GCU GUGGGCAC |
| 4210 | GGGCGGGG AGAA GCACGC ACCAGAGAAACACACGUUGUGGUACAUUACCUGGUA | GCGUGCU GCU CCCCGCCC |
| 4217 | AGUCUGGG AGAA GGGAGC ACCAGAGAAACACACGUUGUGGUACAUUACCUGGUA | GCUCCCC GCC CCCAGACU |
| 4224 | GAGUUGUA AGAA GGGGGC ACCAGAGAAACACACGUUGUGGUACAUUACCUGGUA | GCCCCCA GAC UACAACUC |

(continued)

| nt. Position | HP Ribozyme Sequence | Substrate |
|---|---|---|
| 4382 | CAAAAAGC AGAA GGCUCC ACCAGAGAAACACACGUUGUGGUACAUUACCUGGUA | GGAGCCA GCU GCUUUUUG |
| 4385 | UCACAAAA AGAA GCUGGC ACCAGAGAAACACACGUUGUGGUACAUUACCUGGUA | GCCAGCU GCU UUUUGUGA |
| 4537 | GGGGUUGG AGAA GGGAAG ACCAGAGAAACACACGUUGUGGUACAUUACCUGGUA | CUUCCCU GCU CCAACCCC |
| 4573 | CUCAAUCA AGAA GGUCCU ACCAGAGAAACACACGUUGUGGUACAUUACCUGGUA | AGGACCA GUU UGAUUGAG |
| 4594 | AUUGGGUG AGAA GUGCAG ACCAGAGAAACACACGUUGUGGUACAUUACCUGGUA | CUGCACU GAU CACCCAAU |
| 4628 | GGCUGCAG AGAA GGCCCA ACCAGAGAAACACACGUUGUGGUACAUUACCUGGUA | UGGGCCA GCC CUGCAGCC |
| 4636 | GGGUUUUG AGAA GCAGGG ACCAGAGAAACACACGUUGUGGUACAUUACCUGGUA | CCCUGCA GCC CAAAACCC |
| 4866 | AGGGUCAG AGAA GGGAAG ACCAGAGAAACACACGUUGUGGUACAUUACCUGGUA | CUUCCCA GCU CUGACCCU |
| 4871 | GUAGAAGG AGAA GAGCUG ACCAGAGAAACACACGUUGUGGUACAUUACCUGGUA | CAGCUCU GAC CCUUCUAC |
| 4905 | CGCUGUCC AGAA GCUCCU ACCAGAGAAACACACGUUGUGGUACAUUACCUGGUA | AGGAGCA GAU GGACAGCG |
| 5233 | CUGUGCAA AGAA GAAUAA ACCAGAGAAACACACGUUGUGGUACAUUACCUGGUA | UUAUUCU GUU UUGCACAG |
| 5281 | CUCCUCAG AGAA GCAUUU ACCAGAGAAACACACGUUGUGGUACAUUACCUGGUA | AAAUGCA GUC CUGAGGAG |
| 5319 | UUUCCUCC AGAA GCCCUC ACCAGAGAAACACACGUUGUGGUACAUUACCUGGUA | GAGGGCU GAU GGAGGAAA |
| 5358 | GGUAUAGA AGAA GGGUCU ACCAGAGAAACACACGUUGUGGUACAUUACCUGGUA | AGACCCC GUC UCUAUACC |
| 5392 | UGGGUCCC AGAA GUGUUG ACCAGAGAAACACACGUUGUGGUACAUUACCUGGUA | CAACACA GUU GGGACCCA |
| 5563 | UGAGUCCC AGAA GGAGAA ACCAGAGAAACACACGUUGUGGUACAUUACCUGGUA | UUCUCCA GUU GGGACUCA |
| 5622 | AGUUUCAA AGAA GUUGAA ACCAGAGAAACACACGUUGUGGUACAUUACCUGGUA | UUCAACU GCU UUGAAACU |
| 5738 | UAGCAUCA AGAA GAGCCA ACCAGAGAAACACACGUUGUGGUACAUUACCUGGUA | UGGCUCU GUU UGAUGCUA |
| 5838 | UAGCAUCA AGAA GAGCCA ACCAGAGAAACACACGUUGUGGUACAUUACCUGGUA | UGGCUCU GUU UGAUGCUA |
| 5933 | CCCCAAGA AGAA GCAAUC ACCAGAGAAACACACGUUGUGGUACAUUACCUGGUA | GAUUGCU GCU UCUUGGGG |
| 6022 | CACAUAAG AGAA GAGGCA ACCAGAGAAACACACGUUGUGGUACAUUACCUGGUA | UGCCUCU GUU CUUAUGUG |
| 6120 | UCCACAAA AGAA GCUGCC ACCAGAGAAACACACGUUGUGGUACAUUACCUGGUA | GGCAGCG GCU UUUGUGGA |
| 6163 | GUGGAGAG AGAA GUCCCA ACCAGAGAAACACACGUUGUGGUACAUUACCUGGUA | UGGGACA GUC CUCUCCAC |
| 6270 | AAAUUGCC AGAA GUCACA ACCAGAGAAACACACGUUGUGGUACAUUACCUGGUA | UGUGACA GCU GGCAAUUU |
| 6412 | AAGACAUG AGAA GCUAAG ACCAGAGAAACACACGUUGUGGUACAUUACCUGGUA | CUUAGCU GUU CAUGUCUU |
| 6511 | UUUGAAGG AGAA GAGUAA ACCAGAGAAACACACGUUGUGGUACAUUACCUGGUA | UUACUCA GCU CCUUCAAA |

(continued)

| nt. Position | HP Ribozyme Sequence | Substrate |
|---|---|---|
| 6778 | UCCACCCA AGAA GUUCCA ACCAGAGAAACACACGUUGUGGUACAUUACCUGGUA | UGGAACA GUC UGGGUGGA |
| 6826 | ACUUCUUG AGAA GACAAG ACCAGAGAAACACACGUUGUGGUACAUUACCUGGUA | CUUGUCA GUC CAAGAAGU |
| 7245 | AACAUAAA AGAA GUUGCC ACCAGAGAAACACACGUUGUGGUACAUUACCUGGUA | GGCAACU GCU UUUAUGUU |
| 7258 | UGGAAGGA AGAA GAACAU ACCAGAGAAACACACGUUGUGGUACAUUACCUGGUA | AUGUUCU GUC UCCUUCCA |
| 7433 | CCCAUACA AGAA GCUAAA ACCAGAGAAACACACGUUGUGGUACAUUACCUGGUA | UUUAGCU GAU UGUAUGGG |
| 7512 | UUUUCAUG AGAA GGAAUU ACCAGAGAAACACACGUUGUGGUACAUUACCUGGUA | AAUUCCU GUC CAUGAAAA |
| 7606 | GACAUUCA AGAA GAAAUG ACCAGAGAAACACACGUUGUGGUACAUUACCUGGUA | CAUUUCA GCC UGAAUGUC |
| 7618 | AAUAUAUA AGAA GACAUU ACCAGAGAAACACACGUUGUGGUACAUUACCUGGUA | AAUGUCU GCC UAUAUAUU |
| 7633 | AUACAAAG AGAA GAGAAU ACCAGAGAAACACACGUUGUGGUACAUUACCUGGUA | AUUCUCU GCU CUUUGUAU |

Table IV: Homologous Hammerhead Ribozyme Target Sites Between Human flt-1 and KDR RNA

| nt. Position | flt-1 Target Sequence | nt. Position | KDR Target Sequence |
|---|---|---|---|
| 3388 | CCGGGAU A UUUAUAA | 3151 | CCGGGAU A UUUAUAA |
| 2174 | AAUGUAU A CACAGGG | 3069 | AgUGUAU c CACAGGG |
| 2990 | UGCAAAU A UGGAAAU | 2756 | UGCAAAU u UGGAAAc |
| 2693 | CUCCCUU A UGAUGCC | 2459 | CUgCCUU A UGAUGCC |
| 2981 | GUUGAAU A CUGCAAA | 2747 | GUgGAAU u CUGCAAA |
| 1359 | UAUGGUU A AAAGAUG | 2097 | UgUGGUU u AAAGAUa |
| 3390 | GGGAUAU U UAUAAGA | 3153 | GGGAUAU U UAUAAag |
| 3391 | GGAUAUU U AUAAGAA | 3154 | GGAUAUU U AUAAagA |
| 2925 | ACGUGGU U AACCUGC | 2691 | AUGUGGU c AACCUuC |
| 7140 | UAUUUCU A GUCAUGA | 2340 | UAcUUCU u GUCAUcA |
| 1785 | CAAUAAU A GAAGGAA | 1515 | CucUAAU u GAAGGAA |
| 2731 | GAGACUU A AACUGGG | 768 | uuGACUU c AACUGGG |
| 3974 | GAUGACU A CCAGGGC | 1466 | GAgGACU u CCAGGGa |
| 6590 | UUAAUGU A GAAAGAA | 2603 | aaAAUGU u GAAAGAA |
| 6705 | GCCAUUU A UGACAAA | 3227 | aCaAUUU u UGACAgA |
| 974 | GUCAAAU U ACUUAGA | 147 | uUCAAAU U ACUUgcA |
| 1872 | AUAAAGU U GGGACUG | 1602 | AcAAAGU c GGGAgaG |
| 2333 | ACUUGGU U UAAAAAC | 1088 | AaaUGGU a UAAAAAu |
| 2775 | AAGUGGU U CAAGCAU | 1745 | AcaUGGU a CAAGCuU |
| 3533 | UUCUCCU U AGGUGGG | 3296 | UUuUCCU U AGGUGcu |
| 3534 | UUCUCCUU A GGUGGGU | 3297 | UuUCCUU A GGUGcuU |
| 3625 | GUACUCU A CUCCUGA | 4054 | GagCUCU c CUCCUGu |
| 1814 | AGCACCU U GGUUGUG | 1059 | AGuACCU U GGUUacc |
| 2744 | GGCAAAU C ACUUGGA | 147 | uuCAAAU u ACUUGcA |
| 2783 | CAAGCAU C AGCAUUU | 796 | gAAGCAU C AGCAUaa |
| 3613 | GAGAGCU C CUGAGUA | 2968 | GgaAGCU C CUGAagA |
| 4052 | AAGGCCU C GCUCAAG | 1923 | ucuGCCU u GCUCAAG |
| 5305 | UCUCCAU A UCAAAAC | 456 | ggUCCAU u UCAAAuC |
| 7158 | AUGUAUU U UGUAUAC | 631 | gUcUAUU a UGUAcAu |

(continued)

| nt. Position | flt-1 Target Sequence | nt. Position | KDR Target Sequence |
|---|---|---|---|
| 1836 | CUAGAAU U UCUGGAA | 1007 | aUgGAAU c UCUGGug |
| 2565 | CUCUCUU C UGGCUCC | 2328 | uguUCUU C UGGCUaC |
| 4250 | CUGUACU C CACCCCA | 3388 | uUaUACU a CACCagA |
| 7124 | ACAUGGU U UGGUCCU | 3778 | cagUGGU a UGGUuCU |
| 436 | AUGGUCU U UGCCUGA | 1337 | AcGGUCU a UGCCauu |
| 2234 | GCACCAU A CCUCCUG | 1344 | augCCAU u CCUCCcc |
| 2763 | GGGCUUU U GGAAAAG | 990 | uuGCUUU U GGAAguG |
| 4229 | CCAGACU A CAACUCG | 767 | auuGACU u CAACUgG |
| 5301 | GUUUUCU C CAUAUCA | 3307 | ugcUUCU C CAUAUCc |
| 6015 | AGAAUGU A UGCCUCU | 1917 | AcuAUGU c UGCCUug |
| 6095 | AUUCCCU A GUGAGCC | 1438 | AUaCCCU u GUGAaga |
| 6236 | UGUUGUU C CUCUUCU | 76 | UagUGUU u CUCUUga |
| 5962 | GCUUCCU U UUAUCCA | 3099 | auaUCCU c UUAUCgg |
| 7629 | UAUAUAU U CUCUGCU | 3096 | gAaAUAU c CUCUuaU |

Lowercase letters are used to represent sequence variance between flt-1 and KDR RNA

Table V: 2.5 μmol RNA Synthesis Cycle

| Reagent | Equivalents | Amount | Wait Time* |
|---|---|---|---|
| Phosphoramidites | 6.5 | 163μL | 2.5 |
| S-Ethyl Tetrazole | 23.8 | 238μL | 2.5 |
| Acetic Anhydride | 100 | 233 μL | 5 sec |
| N-Methyl Imidazole | 186 | 233 μL | 5 sec |
| TCA | 83.2 | 1.73 mL | 21 sec |
| Iodine | 8.0 | 1.18 mL | 45 sec |
| Acetonitrile | NA | 6.67 mL | NA |

## Claims

1. A nucleic acid molecule which specifically inhibits the expression of an mRNA encoding flt-1 receptor of human vascular endothelial growth factor, wherein said nucleic acid molecule is an enzymatic nucleic acid molecule targeted to nucleotide position 4229 of flt-1.

2. The nucleic acid molecule of claim 1, wherein said nucleic acid molecule comprises 16 bases complementary to the RNA encoding the flt-1 receptor.

3. The nucleic acid molecule of claim 1 or 2, wherein said nucleic acid molecule is in a hammerhead, hairpin, hepatitis Delta virus, group I introns, VS nucleic acid or RNase P nucleic acid motif.

4. The nucleic acid molecule of claim 3, wherein said nucleic acid molecule is in a hammerhead nucleic acid motif.

5. The nucleic acid molecule of claim 4, wherein said enzymatic nucleic acid molecule comprises binding arms containing sequence complementarity to a substrate nucleotide base sequence CCCAGACUAC AACUCGG (SEQ ID NO: 2130).

6. An *in vitro* method of cleaving RNA of the flt-1 gene comprising the step of contacting said RNA with the enzymatic nucleic acid molecule of claim 4 or 5 under conditions suitable for the cleavage of said RNA.

7. An expression vector comprising nucleic acid sequence encoding the nucleic acid molecule of claim 1.

8. An isolated mammalian cell comprising a nucleic acid molecule of claim 1 or an expression vector of claim 7.

**9.** The mammalian cell of claim 8, wherein said mammalian cell is a human cell.

**10.** Use of the nucleic acid molecule of claim 1 for preparing a medicament for the treatment of a pathological condition selected from the group consisting of tumor angiogenesis, ocular diseases, psoriasis and rheumatoid arthritis, wherein said condition is associated with the level of flt-1 activity.

## Patentansprüche

**1.** Ein Nukleinsäuremolekül, das spezifisch die Expression einer mRNA inhibiert, kodierend für den flt-1-Rezeptor des menschlichen vaskulären endothelialen Wachstumsfaktors, wobei das Nukleinsäuremolekül ein enzymatisches Nukleinsäuremolekül ist, das auf die Nukleotid-Position 4229 des flt-1 gerichtet ist.

**2.** Das Nukleinsäuremolekül gemäß Anspruch 1, wobei das Nukleinsäuremolekül 16 Basen umfasst, die komplementär zur RNA sind, kodierend für den flt-1-Rezeptor.

**3.** Das Nukleinsäuremolekül gemäß Anspruch 1 oder 2, wobei das Nukleinsäuremolekül sich in einem Hammerhead-, Haarnadel-, Hepatitis-Delta-Virus-, Gruppe-I-Intron-, VS-Nukleinsäure- oder RNase-P-Nukleinsäure-Motiv befindet.

**4.** Das Nukleinsäuremolekül gemäß Anspruch 3, wobei das Nukleinsäuremolekül sich in einem Hammerhead-Nukleinsäuremotiv befindet.

**5.** Das Nukleinsäuremolekül gemäß Anspruch 4, wobei das enzymatische Nukleinsäuremolekül Bindungsarme umfasst, die Sequenz enthalten, die komplementär zu einer Substrat-Nukleotid-Basensequenz CCCAGACUAC AACUCGG (SEQ.-ID.Nr.: 2130) ist.

**6.** Ein In-vitro-Verfahren zur Spaltung von RNA des flt-1-Gens, umfassend den Schritt des Inkontaktbringens der RNA mit dem enzymatischen Nukleinsäuremolekül gemäß Anspruch 4 oder 5 unter Bedingungen, die zur Spaltung der RNA geeignet sind.

**7.** Ein Expressionsvektor, umfassend eine Nukleinsäuresequenz, die für das Nukleinsäuremolekül gemäß Anspruch 1 kodiert.

**8.** Eine isolierte Säugerzelle, umfassend ein Nukleinsäuremolekül gemäß Anspruch 1 oder einen Expressionsvektor gemäß Anspruch 7.

**9.** Die Säugerzelle gemäß Anspruch 8, wobei die Säugerzelle eine menschliche Zelle ist.

**10.** Verwendung des Nukleinsäuremoleküls gemäß Anspruch 1 zur Herstellung eines Medikaments zur Behandlung eines Erkrankungszustandes, ausgewählt aus der Gruppe, bestehend aus Tumorangiogenese, Augenerkrankungen, Psoriasis und rheumatoider Arthritis, wobei der Zustand mit dem Grad der flt-1-Aktivität verbunden ist.

## Revendications

**1.** Molécule d'acide nucléique qui inhibe spécifiquement l'expression d'un ARNm codant le récepteur flt-1 du facteur de croissance de l'endothélium vasculaire humain, où ladite molécule d'acide nucléique est une molécule d'acide nucléique enzymatique ciblée sur la position du nucléotide 4229 du flt-1.

**2.** La molécule d'acide nucléique de la revendication 1, où ladite molécule d'acide nucléique comprend 16 bases complémentaires à l'ARN codant le récepteur flt-1.

**3.** La molécule d'acide nucléique de la revendication 1 ou 2, où ladite molécule d'acide nucléique est d'un motif d'acide nucléique en tête de marteau, en épingle à cheveux, du virus de l'hépatite delta, d'introns du groupe I, d'acide nucléique VS ou de RNase P.

**4.** La molécule d'acide nucléique de la revendication 3, où ladite molécule d'acide nucléique est d'un motif d'acide nucléique en tête de marteau.

5. La molécule d'acide nucléique de la revendication 4, où ladite molécule d'acide nucléique enzymatique comprend des bras de liaison contenant une complémentarité de séquence avec une séquence de bases nucléotidiques de substrat CCCAGACUAC AACUCGG (SEQ ID NO: 2130).

6. Procédé *in vitro* de clivage d'ARN du gène flt-1, comprenant l'étape consistant à mettre ledit ARN en contact avec la molécule d'acide nucléique enzymatique de la revendication 4 ou 5 dans des conditions convenant au clivage dudit ARN.

7. Vecteur d'expression comprenant la séquence d'acide nucléique codant la molécule d'acide nucléique de la revendication 1.

8. Cellule mammalienne isolée comprenant une molécule d'acide nucléique de la revendication 1 ou un vecteur d'expression de la revendication 7.

9. La cellule mammalienne de la revendication 8, où la cellule mammalienne est une cellule humaine.

10. Utilisation de la molécule d'acide nucléique de la revendication 1 dans la préparation d'un médicament pour le traitement d'une condition pathologique sélectionnée parmi le groupe consistant en angiogenèse tumorale, maladies oculaires, psoriasis et polyarthrite rhumatoïde, où ladite condition est associée au niveau d'activité de flt-1.

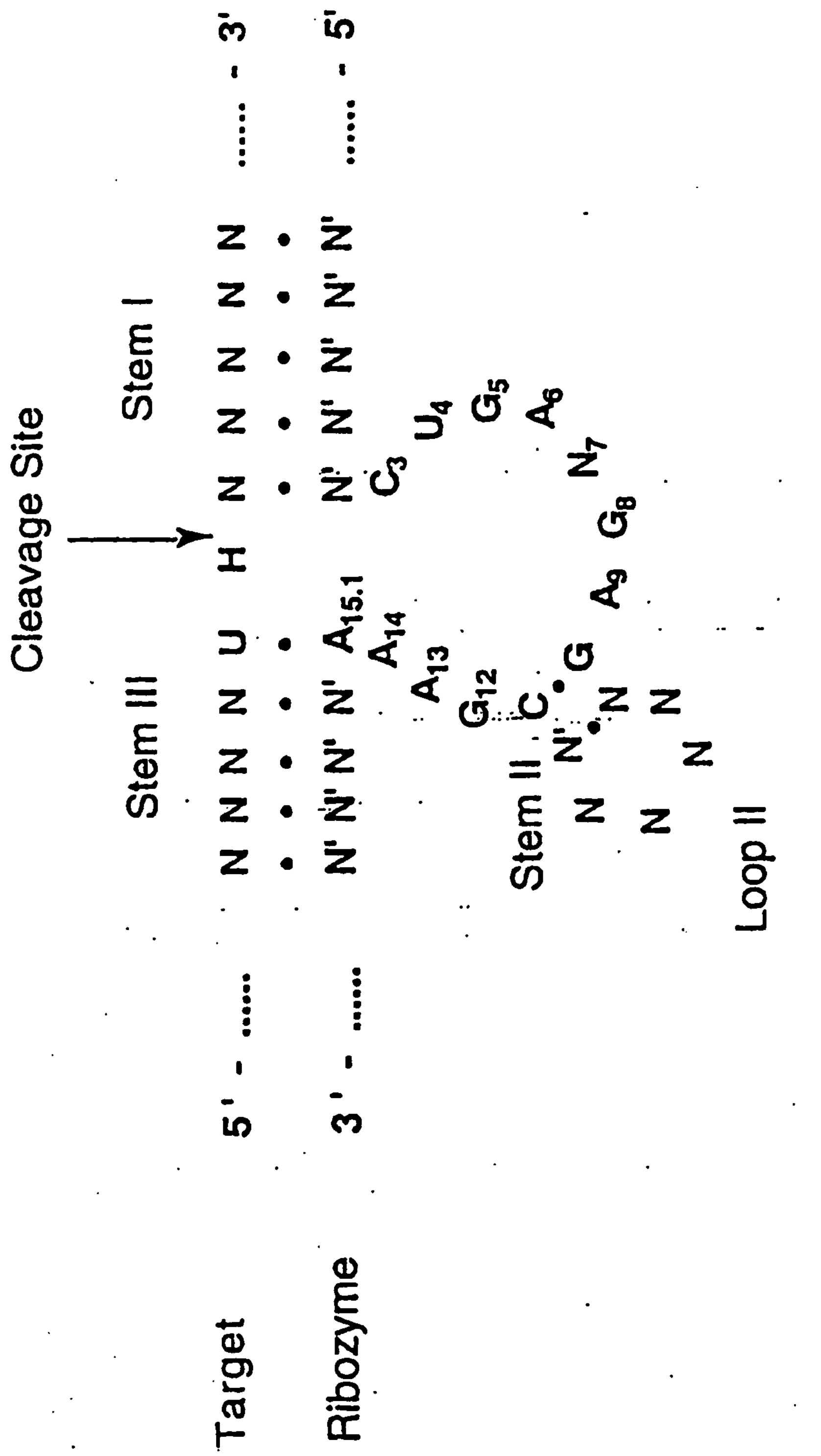

Cleavage Site
Stem I
Stem III
Target
Ribozyme
5' - ...... N N N N U H N N N N N N ...... - 3'
3' - ...... N' N' N' N' A15.1 N' N' N' N' N' N' ...... - 5'
C3 U4 G5 A6 N7 G8 A9
A14 A13 G12
C G
N' N
N N
N N
N
Stem II
Loop II


FIG. 1.

FIG. 2a.

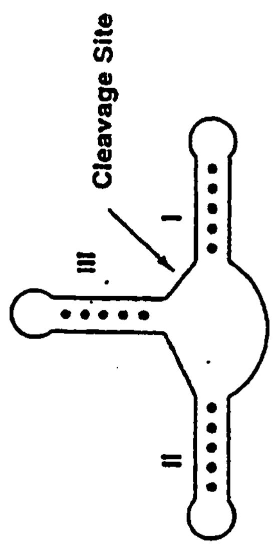


FIG. 2b.

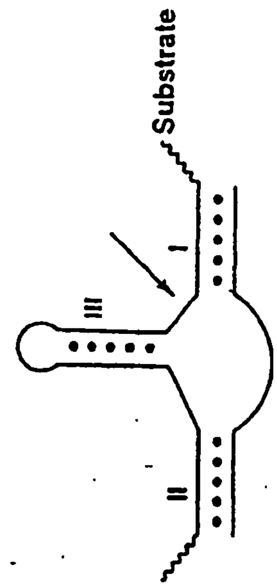


FIG. 2c.

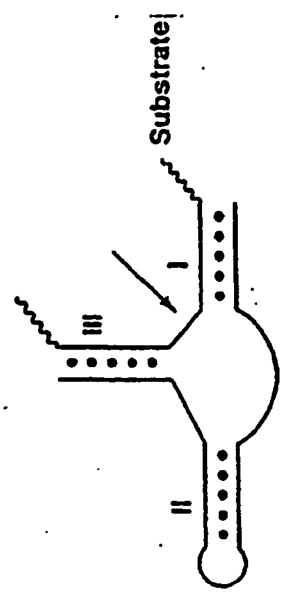


FIG. 2d.

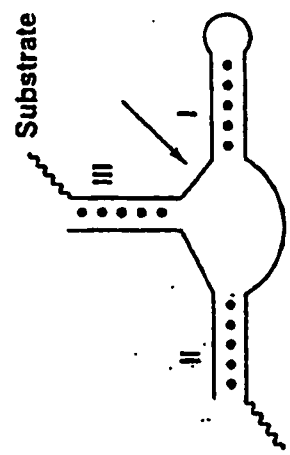

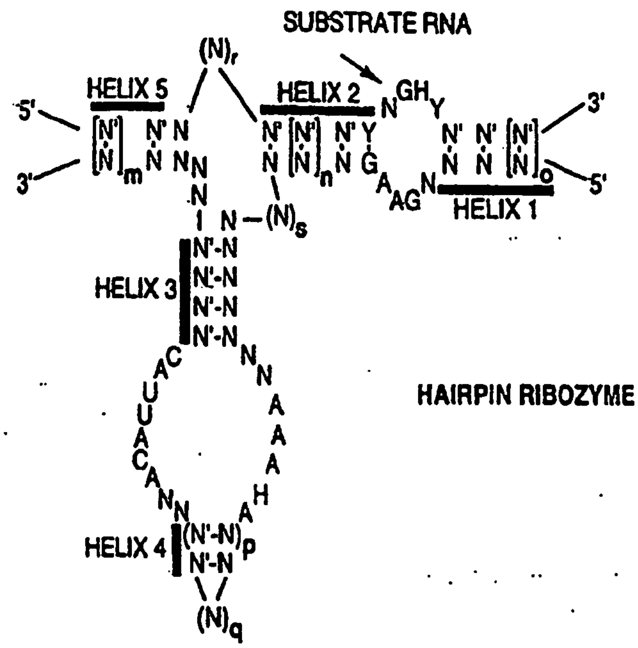
SUBSTRATE RNA
HELIX 5
HELIX 2
HELIX 1
HELIX 3
HELIX 4
5'
3'
(N)r
(N)s
(N)q
HAIRPIN RIBOZYME

FIG. 3.

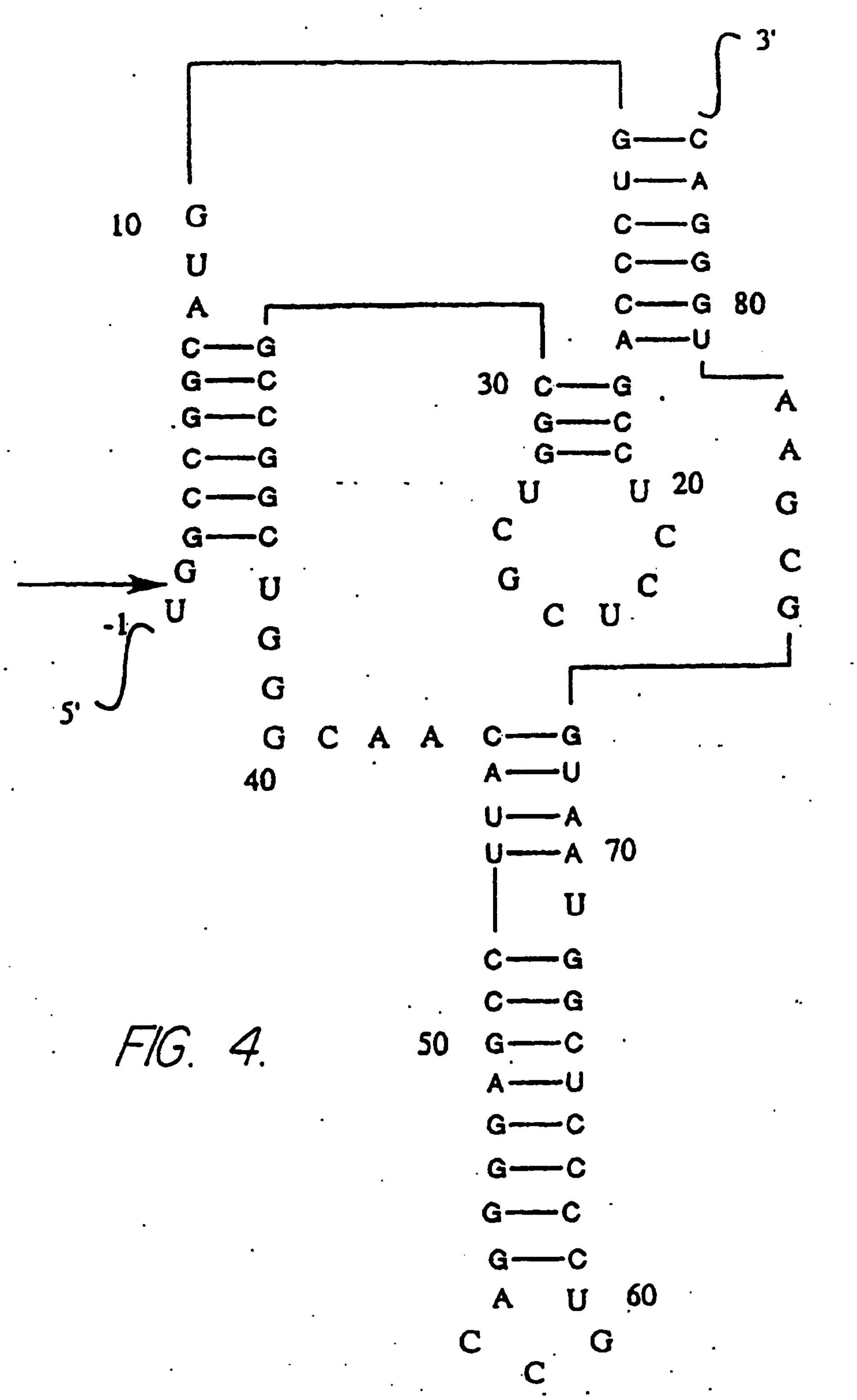


FIG. 4.

NEUROSPORA VS RNA ENZYME

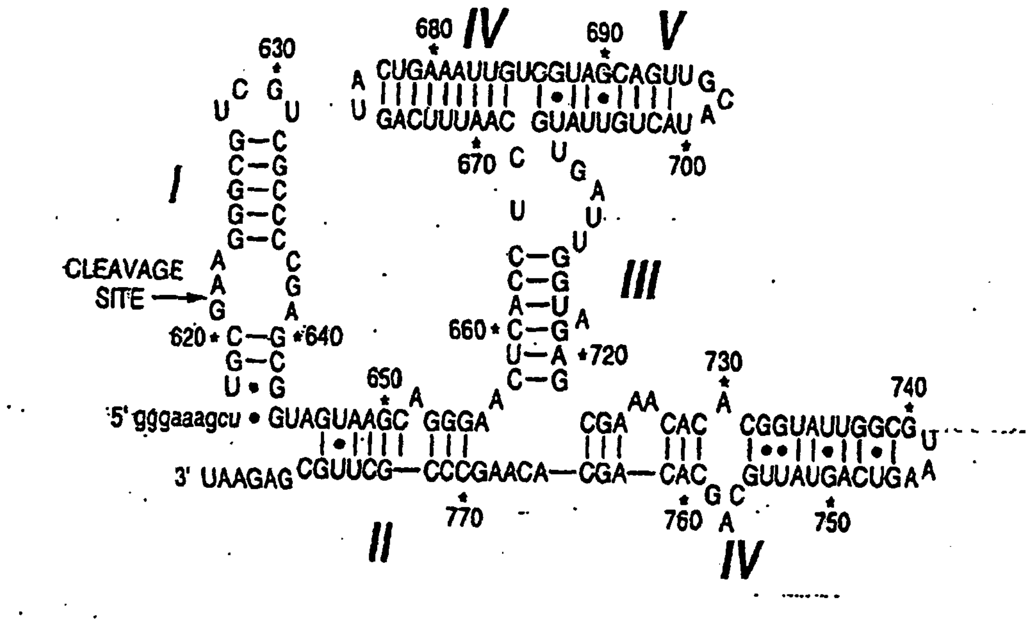


FIG. 5.

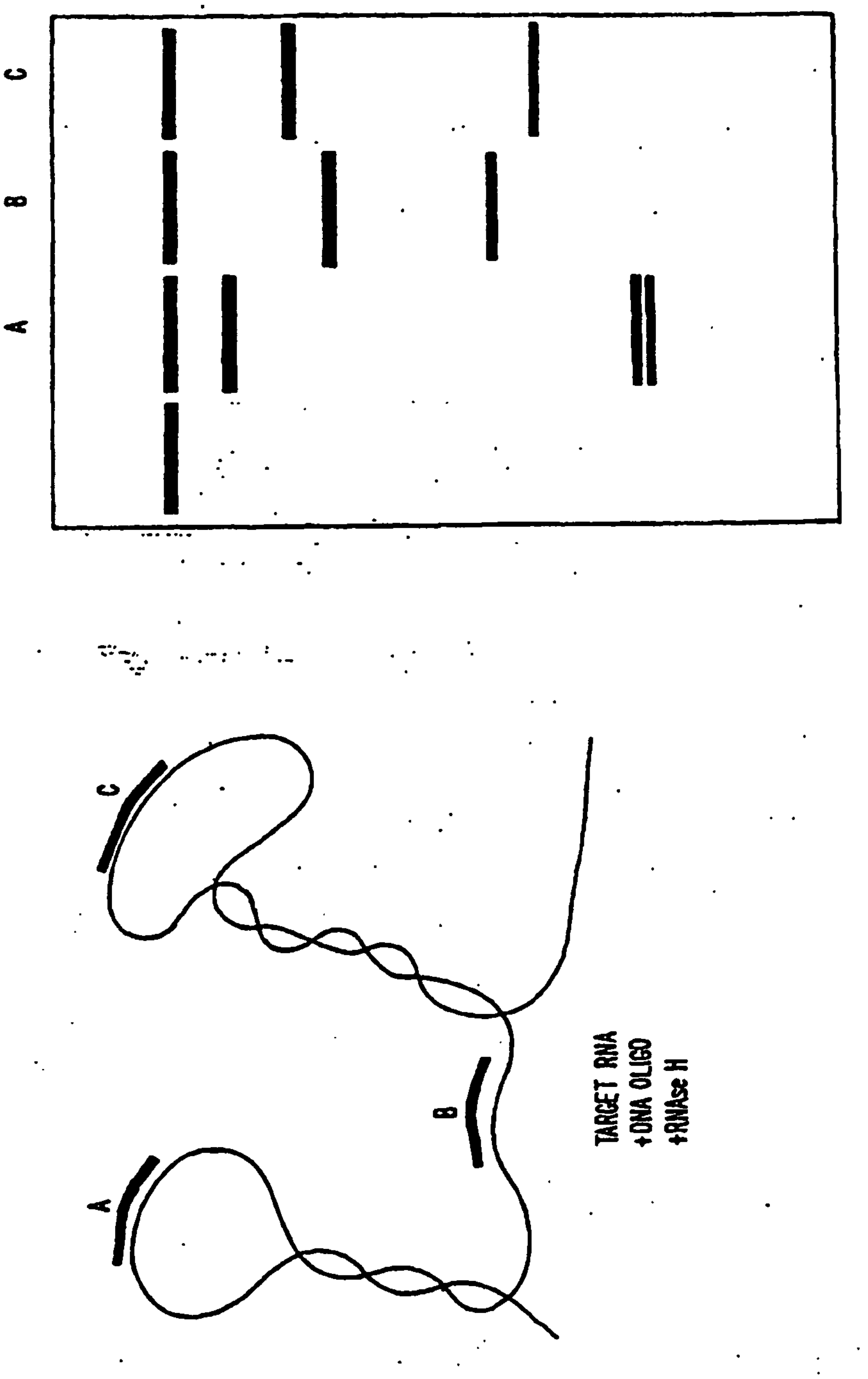


FIG. 6.

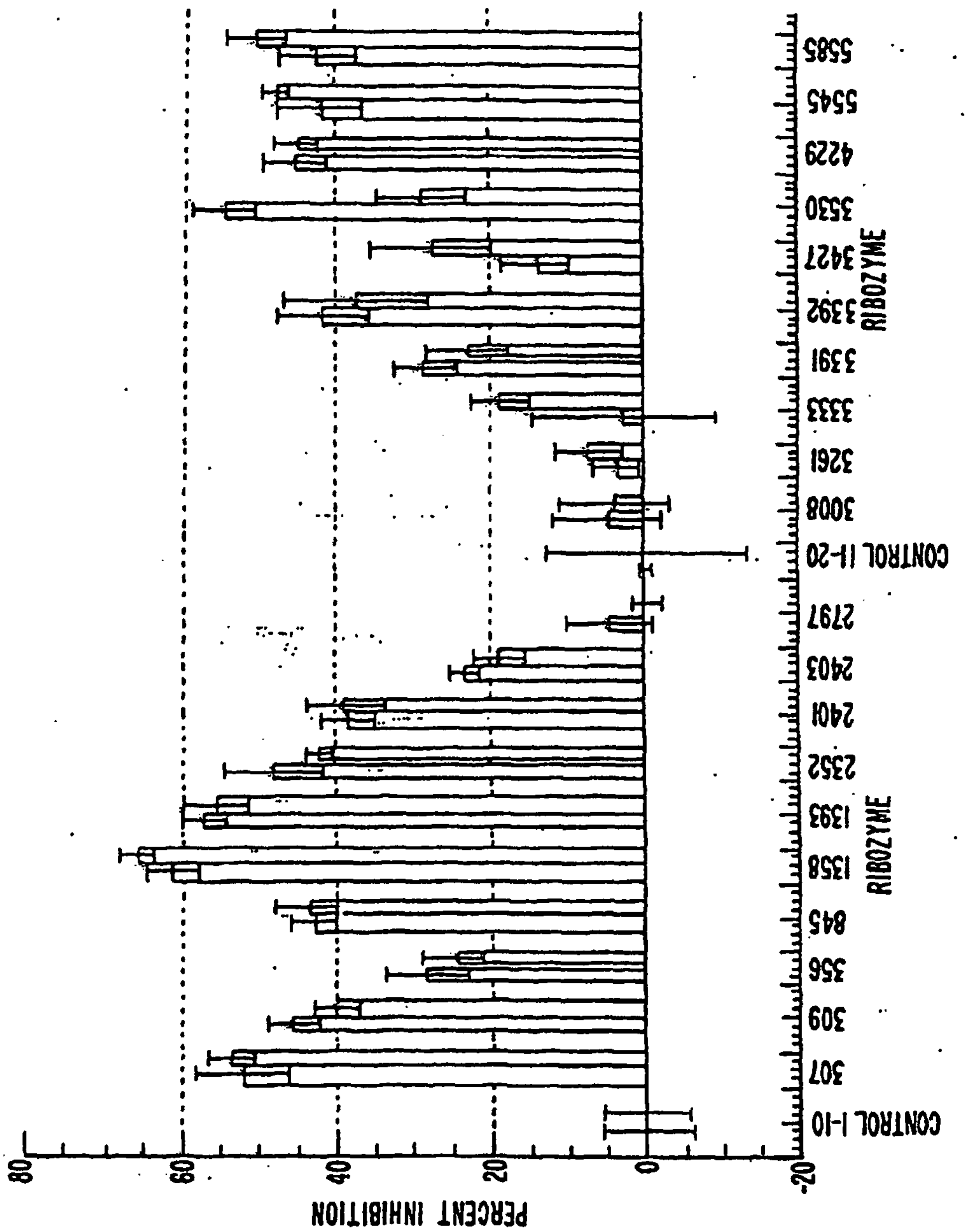
PERCENT INHIBITION
80
60
40
20
0
-20
CONTROL I-10
307
309
356
845
1358
1393
2352
2401
2403
2797
CONTROL II-20
3008
3261
3333
3391
3392
3427
3530
4229
5545
5585
RIBOZYME
RIBOZYME

FIG. 7.

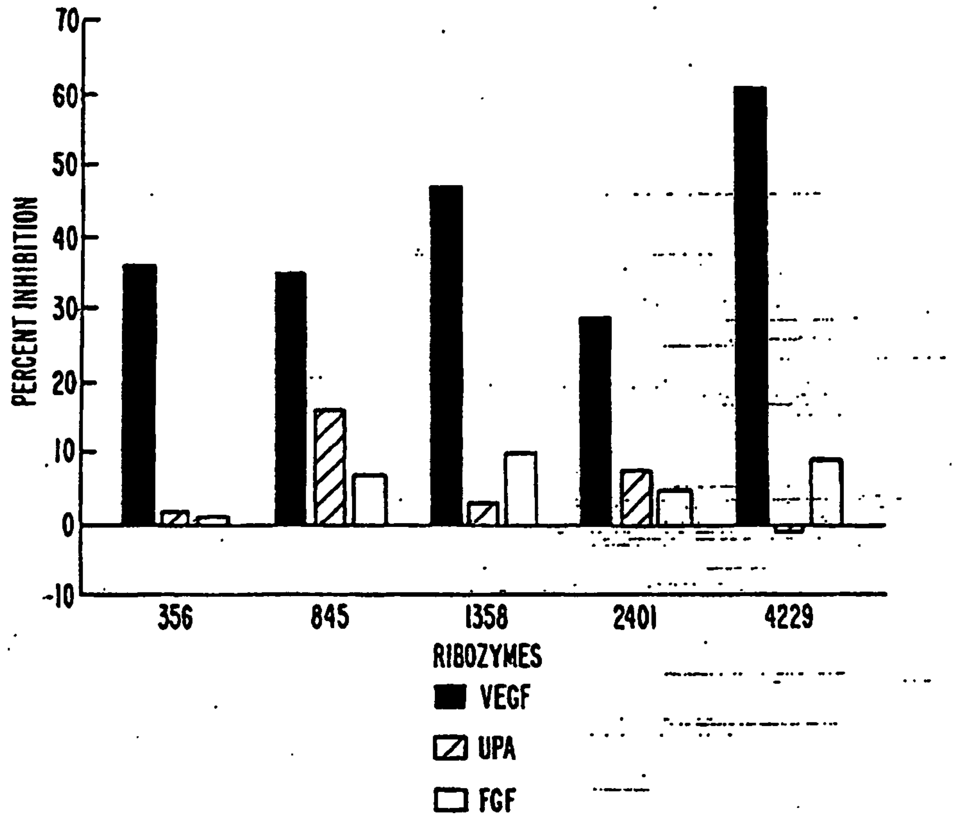
70
60
50
40
30
20
10
0
-10
PERCENT INHIBITION
356
845
1358
2401
4229
RIBOZYMES
VEGF
UPA
FGF

FIG. 8

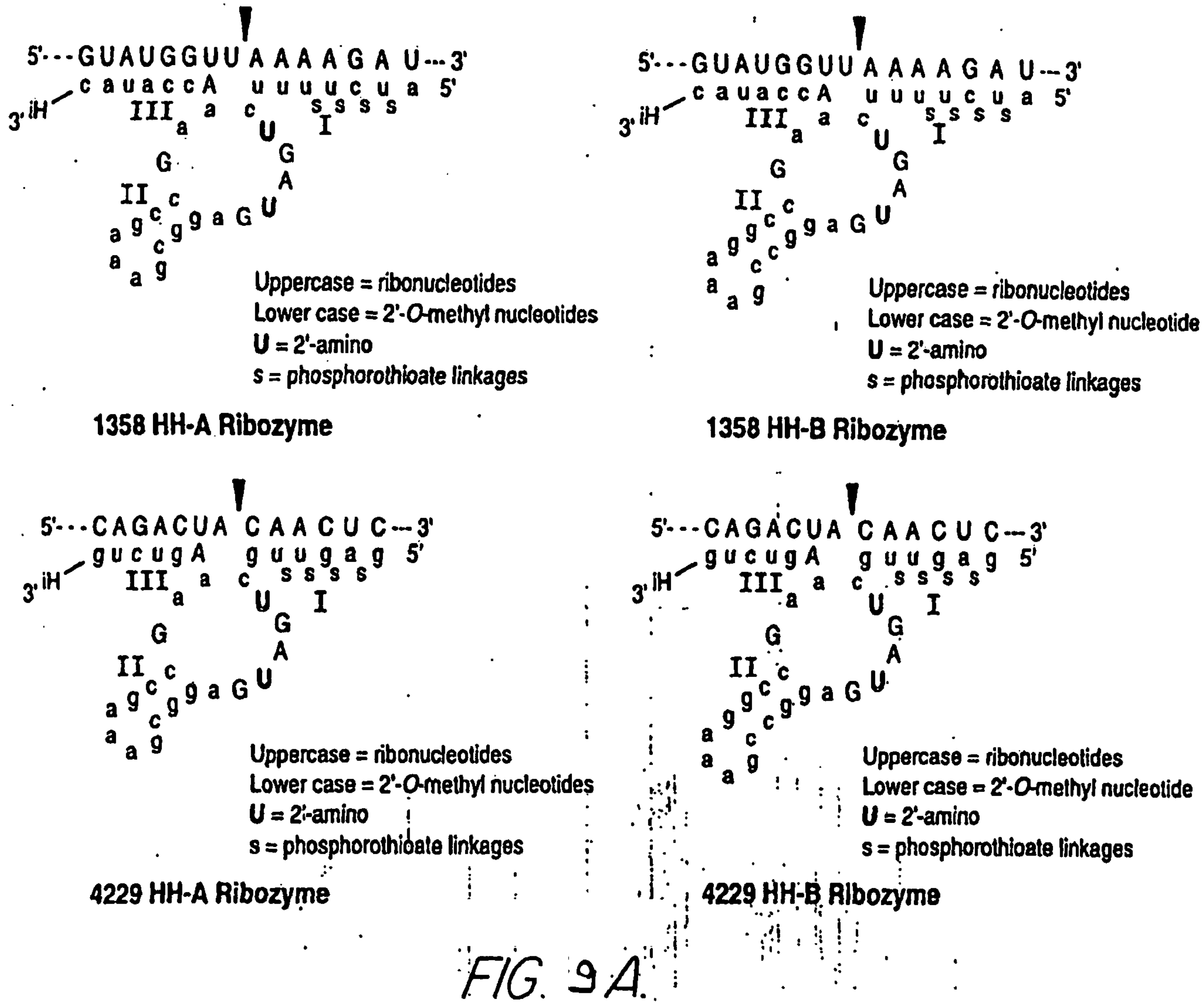
5'---GUAUGGUUAAAAGAU---3'
3' iH caua cc A uuuucua 5'
Uppercase = ribonucleotides
Lower case = 2'-O-methyl nucleotides
U = 2'-amino
s = phosphorothioate linkages
1358 HH-A Ribozyme
5'---GUAUGGUUAAAAGAU---3'
3' iH caua cc A uuuucua 5'
Uppercase = ribonucleotides
Lower case = 2'-O-methyl nucleotide
U = 2'-amino
s = phosphorothioate linkages
1358 HH-B Ribozyme
5'---CAGACUACAACUC---3'
3' iH gucugA guugag 5'
Uppercase = ribonucleotides
Lower case = 2'-O-methyl nucleotides
U = 2'-amino
s = phosphorothioate linkages
4229 HH-A Ribozyme
5'---CAGACUACAACUC---3'
3' iH gucugA guugag 5'
Uppercase = ribonucleotides
Lower case = 2'-O-methyl nucleotide
U = 2'-amino
s = phosphorothioate linkages
4229 HH-B Ribozyme

FIG. 9A.

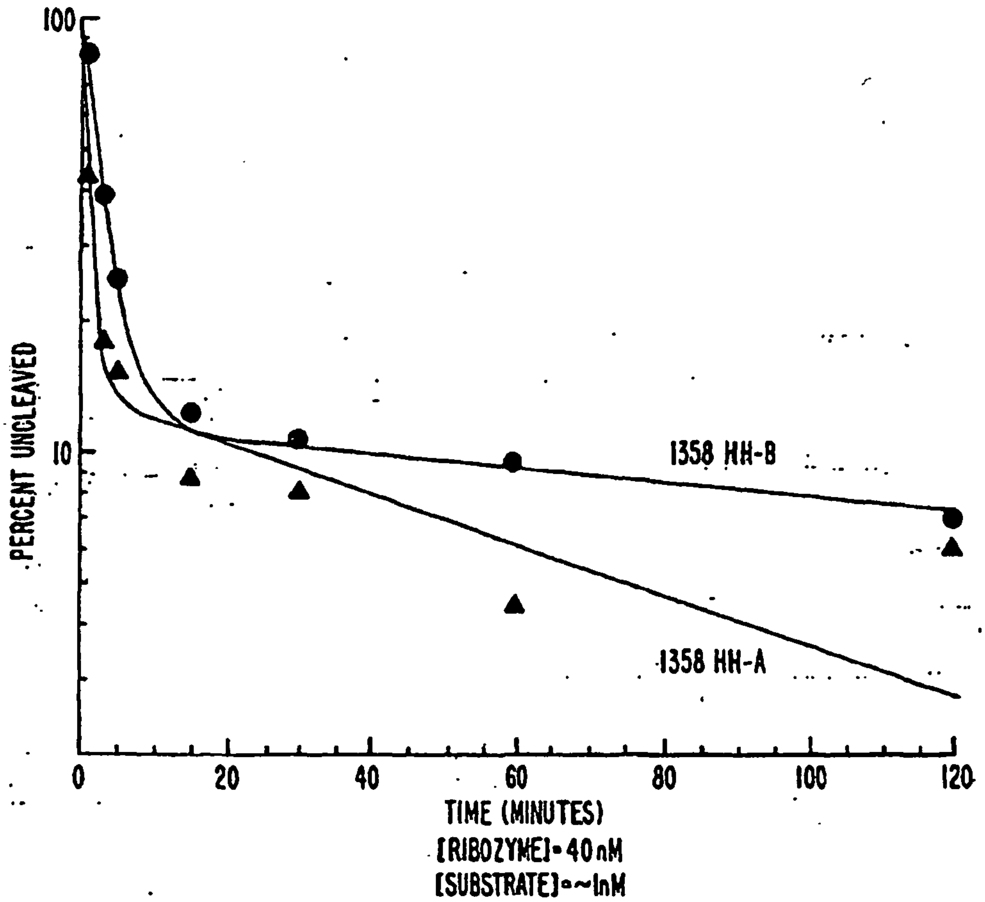
100
10
PERCENT UNCLEAVED
0
20
40
60
80
100
120
TIME (MINUTES)
[RIBOZYME]=40nM
[SUBSTRATE]=~1nM
1358 HH-B
1358 HH-A

FIG. 3B.

FIG. 9

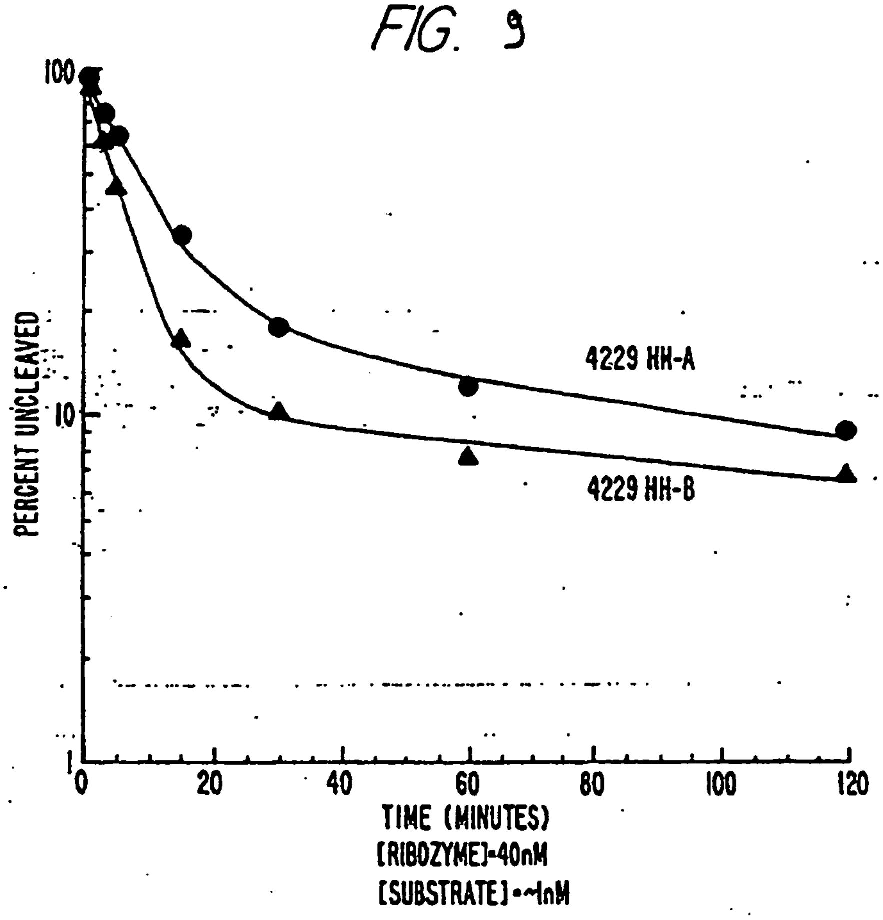
100
10
1
PERCENT UNCLEAVED
0
20
40
60
80
100
120
TIME (MINUTES)
[RIBOZYME]=40nM
[SUBSTRATE]=~1nM
4229 HH-A
4229 HH-B

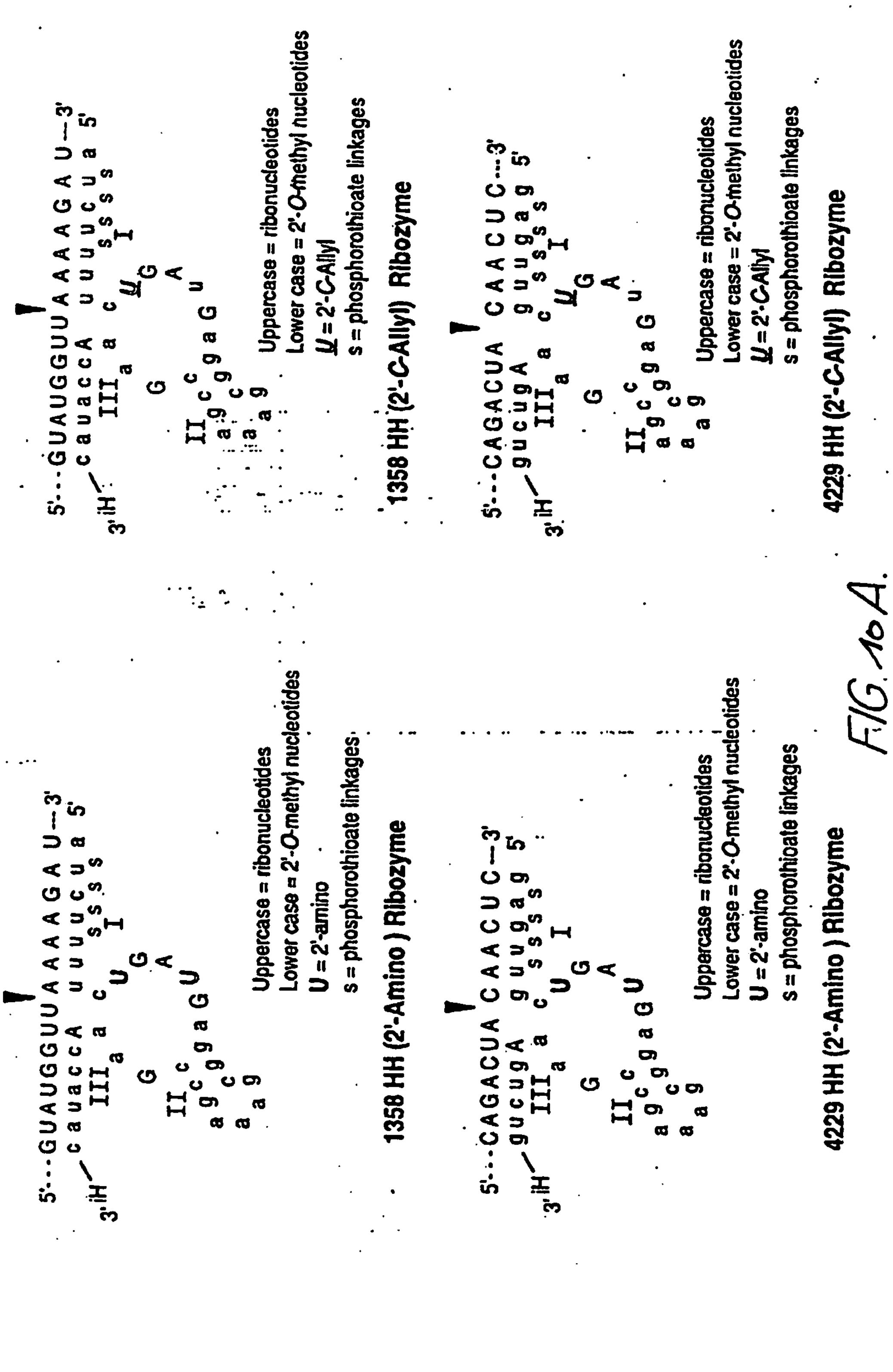
Uppercase = ribonucleotides
Lower case = 2'-O-methyl nucleotides
U = 2'-amino
s = phosphorothioate linkages
1358 HH (2'-Amino) Ribozyme
Uppercase = ribonucleotides
Lower case = 2'-O-methyl nucleotides
U = 2'-C-Allyl
s = phosphorothioate linkages
1358 HH (2'-C-Allyl) Ribozyme
Uppercase = ribonucleotides
Lower case = 2'-O-methyl nucleotides
U = 2'-amino
s = phosphorothioate linkages
4229 HH (2'-Amino) Ribozyme
Uppercase = ribonucleotides
Lower case = 2'-O-methyl nucleotides
U = 2'-C-Allyl
s = phosphorothioate linkages
4229 HH (2'-C-Allyl) Ribozyme

FIG. 10A.

FIG. 10B.

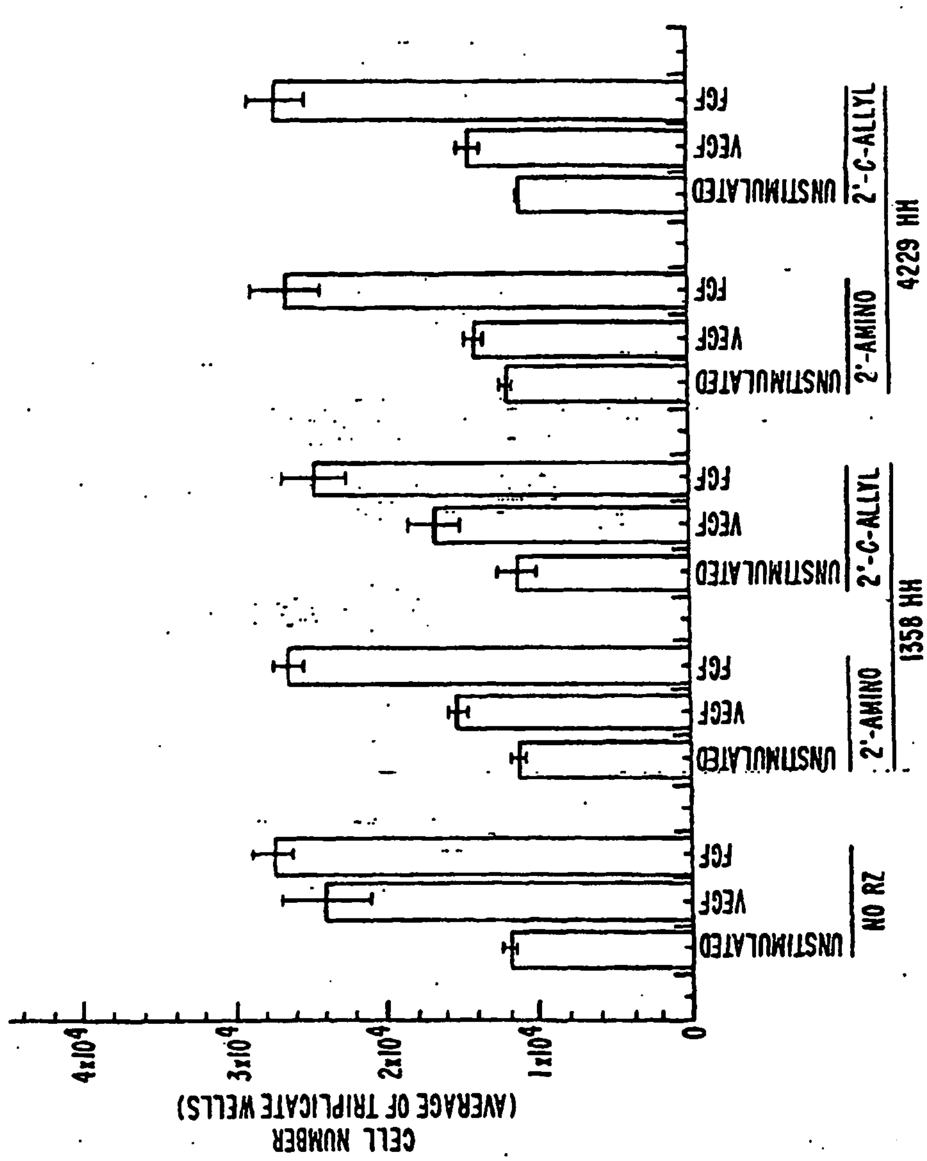
CELL NUMBER
(AVERAGE OF TRIPLICATE WELLS)
0
1x10^4
2x10^4
3x10^4
4x10^4
UNSTIMULATED
VEGF
FGF
NO RZ
2'-AMINO
2'-C-ALLYL
1358 HH
4229 HH

FIG. 11

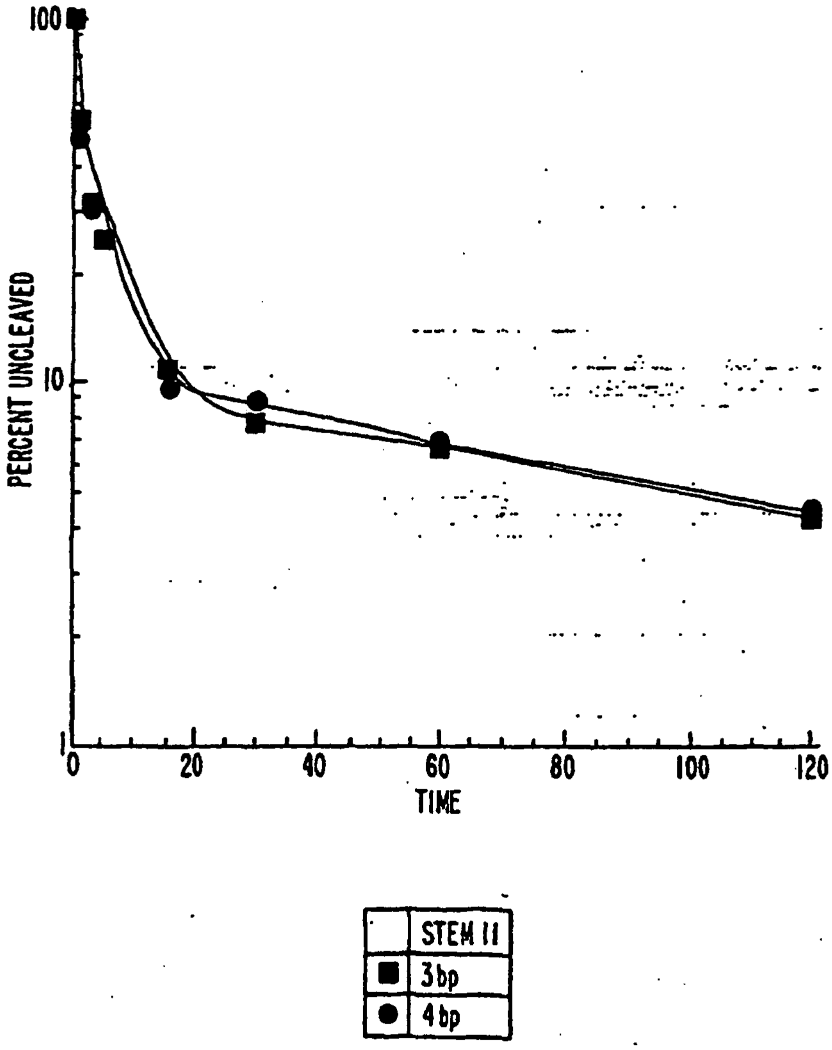
100
10
1
PERCENT UNCLEAVED
0
20
40
60
80
100
120
TIME
STEM II
3bp
4bp

FIG. 12

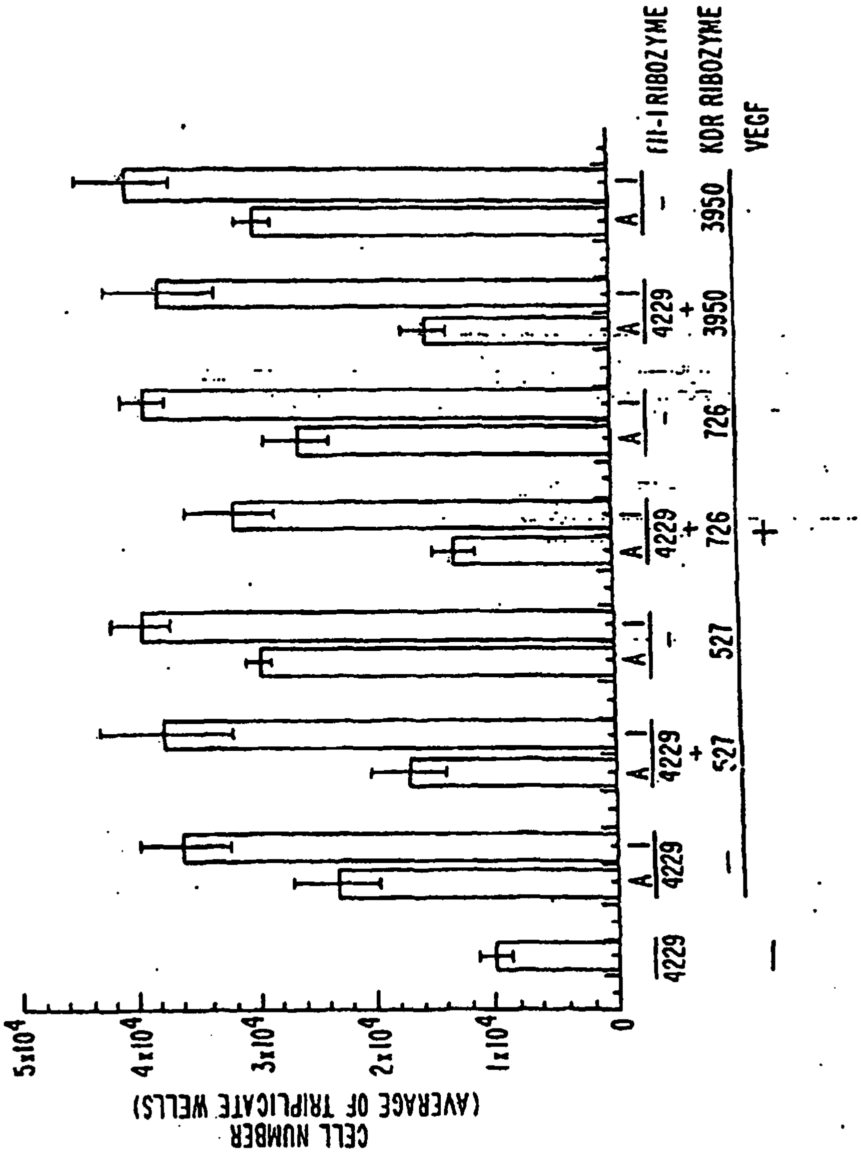
CELL NUMBER
(AVERAGE OF TRIPLICATE WELLS)
0
1x10^4
2x10^4
3x10^4
4x10^4
5x10^4
FII-I RIBOZYME
KDR RIBOZYME
VEGF
4229
A I
4229
527
726
3950
+
−

FIG. 13

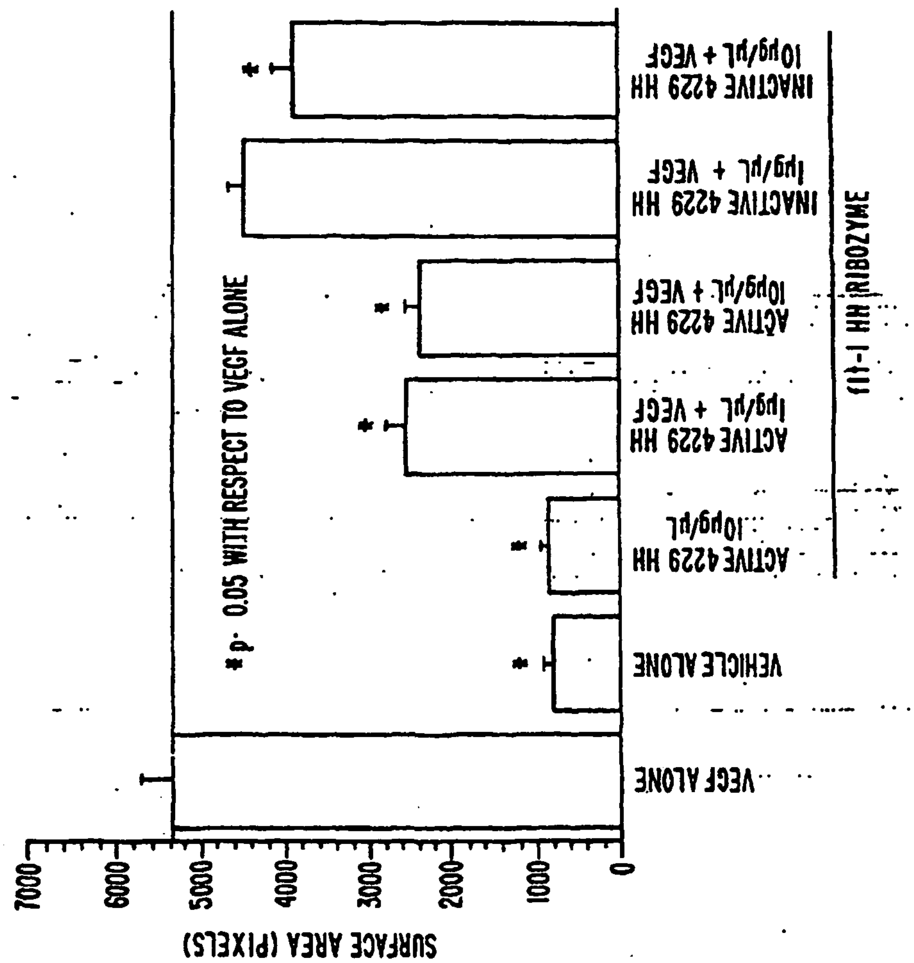
SURFACE AREA (PIXELS)
0
1000
2000
3000
4000
5000
6000
7000
VEGF ALONE
VEHICLE ALONE
ACTIVE 4229 HH 10µg/µL
ACTIVE 4229 HH 1µg/µL + VEGF
ACTIVE 4229 HH 10µg/µL + VEGF
INACTIVE 4229 HH 1µg/µL + VEGF
INACTIVE 4229 HH 10µg/µL + VEGF
flt-1 HH RIBOZYME
* p · 0.05 WITH RESPECT TO VEGF ALONE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9411499 A, Ullrich
- WO 9421679 A, Kendall and Thomas
- WO 9504142 A, Robinson
- WO 9521868 A, Rockwell and Goldstein
- EP 0360257 A, Hampel
- US 4987071 A, Cech
- WO 9323569 A, Draper
- WO 9402595 A, Sullivan
- WO 9513380 A, Draper
- WO 9523225 A
- WO 9207065 A, Eckstein
- WO 9315187 A
- WO 9103162 A, Rossi
- US 5334711 A
- WO 9323057 A, Thompson
- WO 9504818 A, Draper


### Non-patent literature cited in the description


- **Ferrara.** *Trends Cardiovas. Med.,* 1993, vol. 3, 244
- **Neufeld et al.** *Prog. Growth Factor Res.,* 1994, vol. 5, 89
- **Plouet et al.** *EMBO J.,* 1989, vol. 8, 3801
- **Vaisman et al.** *J. Biol. Chem.,* 1990, vol. 265, 19461
- **Shibuya et al.** *Oncogene,* 1990, vol. 5, 519
- **Terman et al.** *Oncogene,* 1991, vol. 6, 1677
- **Mathews et al.** *Proc. Natl. Acad. Sci., USA,* 1991, vol. 88, 9026
- **Gitay-Goren et al.** *J. Biol. Chem.,* 1992, vol. 267, 6093
- **Berkman et al.** *J. Clini. Invest.,* 1993, vol. 91, 153
- **Jim Kim et al.** *Nature,* 1993, vol. 362, 841
- **Millauer et al.** *Nature,* 1994, vol. 367, 576
- **Aiello et al.** *New Engl. J. Med.,* 1994, vol. 331, 1480
- **Miller et al.** *Am. J. Pathol.,* 1994, vol. 145, 574
- **Detmar et al.** *J. Exp. Med.,* 1994, vol. 180, 1141
- **Fava et al.** *J. Exp. Med.,* 1994, vol. 180, 341
- **Koch et al.** *J. Immunol.,* 1994, vol. 152, 4149
- **Takashita et al.** *J. Clin. Invest.,* 1995, vol. 93, 662
- **Millauer et al.** *Cell,* 1993, vol. 72, 835
- **Shalaby et al.** *J. Clin. Invest.,* 1993, vol. 91, 2235
- **Shalaby et al.** *Nature,* 1995, vol. 376, 62
- **Fung et al.** *Nature,* 1995, vol. 376, 66
- **Kim et al.** *Nature,* 1993, vol. 362, 841
- **Jellinek et al.** *Biochemistry,* 1994, vol. 33, 10450
- **Rossi et al.** *AIDS Research and Human Retroviruses,* 1992, vol. 8, 183
- **Hampel ; Tritz.** *Biochemistry,* 1989, vol. 28, 4929
- **Hampel et al.** *Nucleic Acids Res.,* 1990, vol. 18, 299
- **Perrotta ; Been.** *Biochemistry,* 1992, vol. 31, 16
- **Guerrier-Takada et al.** *Cell,* 1983, vol. 35, 849
- **Saville ; Collins.** *Cell,* 1990, vol. 61, 685-696
- **Saville ; Collins.** *Proc. Natl. Acad. Sci. USA,* 1991, vol. 88, 8826-8830
- **Collins ; Olive.** *Biochemistry,* 1993, vol. 32, 2795-2799
- **Izant ; Weintraub.** *Science,* 1985, vol. 229, 345
- **McGarry ; Lindquist.** *Proc. Natl. Acad. Sci. USA,* 1986, vol. 83, 399
- **Sullenger-Scanlon et al.** *Proc. Natl. Acad. Sci. USA,* 1991, vol. 88, 10591-5
- **Kashani-Sabet et al.** *Antisense Res. Dev.,* 1992, vol. 2, 3-15
- **Dropulic et al.** *J. Virol,* 1992, vol. 66, 1432-41
- **Weerasinghe et al.** *J. Virol,* 1991, vol. 65, 5531-4
- **Ojwang et al.** *Proc. Natl. Acad. Sci. USA,* 1992, vol. 89, 10802-6
- **Chen et al.** *Nucleic Acids Res.,* 1992, vol. 20, 4581-9
- **Sarver et al.** *Science,* 1990, vol. 247, 1222-1225
- **Thompson et al.** *Nucleic Acids Res.,* 1995, vol. 23, 2259
- **Ohkawa et al.** *Nucleic Acids Symp. Ser.,* 1992, vol. 27, 15-6

- **Taira et al.** *Nucleic Acids Res,* 1991, vol. 19, 5125-30
- **Ventura et al.** *Nucleic Acids Res,* 1993, vol. 21, 3249-55
- **Chowrira et al.** *J. Biol. Chem.,* 1994, vol. 269, 25856
- **Uhlenbeck.** *Nature,* 1987, vol. 327, 596-600
- **Haseloff ; Gerlach.** *Nature,* 1988, vol. 334, 585-591
- **Jeffries ; Symons.** *Nucl. Acids. Res.,* 1989, vol. 17, 1371-1371
- **Jaeger et al.** *Proc. Natl. Acad. Sci. USA,* 1989, vol. 86, 7706
- **Usman et al.** *J. Am. Chem. Soc.,* 1987, vol. 109, 7845
- **Scaringe et al.** *Nucleic Acids Res.,* 1990, vol. 18, 5433
- **Wincott et al.** *Nucleic Acids Res.,* 1995, vol. 23, 2677-2684
- **Hertel, K. J. et al.** *Nucleic Acids Res.,* 1992, vol. 20, 3252
- **Wincott et al.** *Nucleic Acids Res,* 1995, vol. 23, 2677-2684
- **Chowrira ; Burke.** *Nucleic Acids Res.,* 1992, vol. 20, 2835-2840
- **Milligan ; Uhlenbeck.** *Methods Enzymol.,* 1989, vol. 180, 51
- **Usman ; Cedergren.** *TIBS,* 1992, vol. 17, 34
- **Usman et al.** *Nucleic Acids Symp. Ser.,* 1994, vol. 31, 163
- **Perrault et al.** *Nature,* 1990, vol. 344, 565
- **Pieken et al.** *Science,* 1991, vol. 253, 314
- **Usman ; Cedergren.** *Trends in Biochem. Sci.,* 1992, vol. 17, 334
- **Beigelman et al.** *J. Biol Chem,* 1995
- **Elroy-Stein ; Moss.** *Proc. Natl. Acad. Sci. U S A,* 1990, vol. 87, 6743-7
- **Gao ; Huang.** *Nucleic Acids Res.,* 1993, vol. 21, 2867-72
- **Lieber et al.** *Methods Enzymol.,* 1993, vol. 217, 47-66
- **Zhou et al.** *Mol. Cell. Biol.,* 1990, vol. 10, 4529-37
- **Ojwang et al.** *Proc. Natl. Acad. Sci. U S A,* 1992, vol. 89, 10802-6
- **Yu et al.** *Proc. Natl. Acad. Sci. U S A,* 1993, vol. 90, 6340-4
- **L'Huillier et al.** *EMBO J,* 1992, vol. 11, 4411-8
- **Lisziewicz et al.** *Proc. Natl. Acad. Sci. U. S. A.,* 1993, vol. 90, 8000-4
- **Zabner et al.** *Cell,* 1993, vol. 75, 207
- **Carter.** *Curr. Opi. Biotech.,* 1992, vol. 3, 533
- **Folkman.** *J. Natl. Cancer Inst.,* 1990, vol. 82, 4
- **Folkman ; Shing.** *J. Biol. Chem.,* 1992, vol. 267, 10931
- **Beigelman et al.** *J Biol. Chem.,* 1995, vol. 270, 25702
- **Pandey et al.** *Science,* 1995, vol. 268, 567-569
- **Pierce et al.** *Proc. Natl. Acad. Sci. USA.,* 1995, vol. 92, 905-909
- **Shweiki et al.** *J. Clin. Invest.,* 1992, vol. 91, 2235-2243
- **Plate et al.** *Nature,* 1992, vol. 359, 845
- **Passaniti et al.** *Lab. Invest.,* 1992, vol. 67, 519-528
- **Burger et al.** *Cornea,* 1985, vol. 4, 35-41
- **Lepri et al.** *J. Ocular Pharmacol.,* 1994, vol. 10, 273-280
- **Ormerod et al.** *Am. J. Pathol.,* 1990, vol. 137, 1243-1252
- **Grant et al.** *Diabetologia,* 1993, vol. 36, 282-291
- **Zieche et al.** *Lab. Invest.,* 1992, vol. 67, 711-715
- **Shweiki et al.** *Clin. Invest.,* 1993, vol. 91, 2235-2243
- **O'Reilly et al.** *Cell,* 1994, vol. 79, 315-328
- **Senger et al.** *Cancer and Metas. Rev.,* 1993, vol. 12, 303-324
- **Takahasi et al.** *Cancer Res.,* 1994, vol. 54, 4233-4237
- **Folkman.** *Adv. Cancer. Res.,* 1985, vol. 43, 175
- **Hertel et al.** *Nucleic Acids Res.,* 1992, vol. 20, 3252